# EUROPEAN PATENT APPLICATION

(11) **EP 2 722 329 A1**
(43) Date of publication of application: **23.04.2014**
(21) Application number: 12189107.1
(22) Date of filing: 18.10.2012
(51) Int. Cl.: C07D 401/14, C07D 405/14, C07D 401/04, C07D 401/06, C07D 403/06, C07D 405/04, A61K 31/404, A61K 31/454, C07D 403/04, A61K 31/497, A61P 25/00

(54) **Indol-amide compounds as beta-amyloid inhbitors**

(71) Applicant: F. Hoffmann-La Roche AG, 4070 Basel (CH)
(72) Inventor: The designation of the inventor has not yet been filed
(74) Representative: Salud, Carlos E.

(57) **Abstract**

The present invention provides compounds of formula (I) 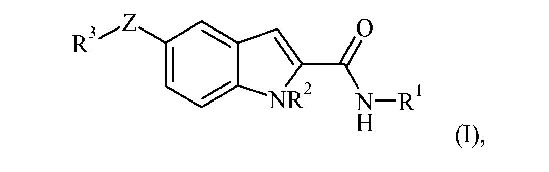 wherein Z, R¹, R², and R³ are as described herein, as well as pharmaceutically acceptable salts and esters thereof. Further the present invention is concerned with the manufacture of the compounds of formula (I), pharmaceutical compositions comprising them and their use as medicaments for the treatment of Alzheimer's disease.

## Description

The present invention provides compounds which are modulators of β-amyloid, their manufacture, pharmaceutical compositions comprising them and their use as medicaments.

In particular, the present invention relates to compounds of formula (I)

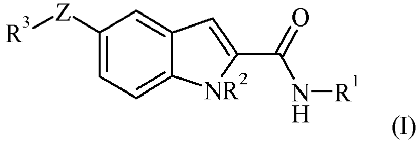

Wherein R¹, R², R³ and Z are as described herein, and pharmaceutically acceptable salts and esters thereof.

It has been found that the compounds of formula (I) as described herein are modulators for amyloid beta and thus, they may be useful for the treatment or prevention of a disease associated with the deposition of β-amyloid in the brain, in particular Alzheimer's disease, and other diseases such as cerebral amyloid angiopathy, hereditary cerebral hemorrhage with amyloidosis Dutch-type (HCHWA-D), multi-infarct dementia, dementia pugilistica and Down syndrome.

Alzheimer's disease (AD) is the most common cause of dementia in later life. Pathologically, AD is characterized by the deposition of amyloid in extracellular plaques and intracellular neurofibrillary tangles in the brain. The amyloid plaques are mainly composed of amyloid peptides (Aβ peptides) which originate from the β-amyloid Precursor Protein (APP) by a series of proteolytic cleavage steps. Several forms of APP have been identified of which the most abundant are proteins of 695, 751 and 770 amino acids length. They all arise from a single gene through differential splicing. The Aβ peptides are derived from the same domain of the APP.

Aβ peptides are produced from APP through the sequential action of two proteolytic enzymes termed β- and γ-secretase. β-Secretase cleaves first in the extracellular domain of APP just outside of the trans-membrane domain (TM) to produce a C-terminal fragment of APP containing the TM- and cytoplasmatic domain (CTFβ). CTFβ is the substrate for γ-secretase which cleaves at several adjacent positions within the TM to produce the Aβ peptides and the cytoplasmic fragment. Various proteolytic cleavages mediated by γ-secretase result in Aβ peptides of different chain length, e.g. Aβ38, Aβ40 and Aβ42. The latter one is regarded to be the more pathogenic amyloid peptide because of its strong tendency to form neurotoxic aggregates.

The β-secretase is a typical aspartyl protease. The γ-secretase is a proteolytic activity consisting of several proteins, its exact composition is incompletely understood. However, the presenilins are essential components of this activity and may represent a new group of atypical aspartyl proteases which cleave within the TM of their substrates and which are themselves polytopic membrane proteins. Other essential components of γ-secretase may be nicastrin and the products of the aph1 and pen-2 genes. Proven substrates for γ-secretase are the APP and the proteins of the Notch receptor family, however, γ-secretase has loose substrate specificity and may cleave further membrane proteins unrelated to APP and Notch.

The γ-secretase activity is absolutely required for the production of Aβ peptides. This has been shown both by genetic means, i.e., ablation of the presenilin genes and by low-molecular-weight inhibitory compounds. Since according to the amyloid hypothesis for AD the production and deposition of Aβ is the ultimate cause for the disease, it is thought that selective and potent inhibitors of γ-secretase will be useful for the prevention and treatment of AD.

An alternative mode of treatment is the modulation of the γ-secretase activity which results in a selective reduction of the Aβ42 production. This will result in an increase of shorter Aβ isoforms, such as Aβ38, Aβ37 or others, which have reduced capability for aggregation and plaque formation, and are less neurotoxic. Compounds which show this effect on modulating γ-secretase activity include certain non-steroidal anti-inflammatory drugs (NSAIDs) and related analogues (Weggen et al. Nature, 414 (2001) 212-16).

Thus, the compounds of this invention will be useful for the treatment or prevention of a disease associated with the deposition of β-amyloid in the brain, in particular Alzheimer's disease, and other diseases such as cerebral amyloid angiopathy, hereditary cerebral hemorrhage with amyloidosis Dutch-type (HCHWA-D), multi-infarct dementia, dementia pugilistica and Down syndrome.

The current knowledge on γ-secretase modulation is described for example in the following publications:
○Morihara et al, J. Neurochem., 83 (2002) 1009-12
○Jantzen et al, J.Neuroscience, 22 (2002) 226-54
○Takahashi et al, J. Biol. Chem., 278 (2003) 18644-70
○Beher et al, J. Biol. Chem. 279 (2004) 43419-26
○Lleo et al, Nature Med. 10 (2004) 1065-6
○Kukar et al, Nature Med. 11 (2005) 545-50
○Perretto et al, J. Med. Chem. 48 (2005) 5705-20
○Clarke et al, J. Biol. Chem. 281 (2006) 31279-89
o Stock et al, Bioorg. Med. Chem. Lett. 16 (2006) 2219-2223
○Narlawar et al, J. Med. Chem. 49 (2006) 7588-91.

### Detailed description of the invention

Unless otherwise defined, all technical and scientific terms used herein have the same meaning as commonly understood by one of ordinary skill in the art to which this invention belongs. Although methods and materials similar or equivalent to those described herein can be used in the practice or testing of the invention, suitable methods and materials are described below.

The nomenclature used in this Application is based on IUPAC systematic nomenclature, unless indicated otherwise.

Any open valency appearing on a carbon, oxygen, sulfur or nitrogen atom in the structures herein indicates the presence of a hydrogen, unless indicated otherwise.

The definitions described herein apply irrespective of whether the terms in question appear alone or in combination. It is contemplated that the definitions described herein can be appended to form chemically-relevant combinations, such as e.g. "heterocycloalkylaryl", "haloalkylheteroaryl", "arylalkylheterocycloalkyl", or "alkoxyalkyl". The last member of the combination is the radical which is binding to the rest of the molecule. The other members of the combination are attached to the binding radical in reversed order in respect of the literal sequence, e.g. the combination arylalkylheterocycloalkyl refers to a heterocycloalkyl-radical which is substituted by an alkyl which is substituted by an aryl.

When indicating the number of substituents, the term "one or more" refers to the range from one substituent to the highest possible number of substitution, i.e. replacement of one hydrogen up to replacement of all hydrogens by substituents.

The term "optional" or "optionally" denotes that a subsequently described event or circumstance can but need not occur, and that the description includes instances where the event or circumstance occurs and instances in which it does not.

The term "substituted" denotes that a specified group bears one or more substituents. Where any group can carry multiple substituents and a variety of possible substituents is provided, the substituents are independently selected and need not to be the same. The term "unsubstituted" means that the specified group bears no substituents. The term "optionally substituted" means that the specified group is unsubstituted or substituted by one or more substituents, independently chosen from the group of possible substituents. When indicating the number of substituents, the term "one or more" means from one substituent to the highest possible number of substitution, i.e. replacement of one hydrogen up to replacement of all hydrogens by substituents.

The term "compound(s) of this invention" and "compound(s) of the present invention" refers to compounds as disclosed herein and stereoisomers, tautomers, solvates, and salts (e.g., pharmaceutically acceptable salts) thereof.
When the compounds of the invention are solids, it is understood by those skilled in the art that these compounds, and their solvates and salts, may exist in different solid forms, particularly different crystal forms, all of which are intended to be within the scope of the present invention and specified formulas.

The term "pharmaceutically acceptable esters" denotes derivatives of the compounds of present invention, in which a carboxy group has been converted to an ester, wherein carboxy group means -C(O)O-. Methyl-, ethyl-, methoxymethyl-, methylthiomethyl-, and pivaloyloxymethylesters are examples of such suitable esters. The term "pharmaceutically acceptable esters" furthermore embraces derivatives of the compounds of present invention in which hydroxy groups have been converted to the corresponding esters with inorganic or organic acids such as nitric acid, sulfuric acid, phosphoric acid, citric acid, formic acid, maleic acid, acetic acid, succinic acid, tartaric acid, methanesulfonic acid, or p-toluenesulfonic acid, and which are non toxic to living organisms.

The term "pharmaceutically acceptable salts" denotes salts which are not biologically or otherwise undesirable. Pharmaceutically acceptable salts include both acid and base addition salts.
The term "pharmaceutically acceptable acid addition salt" denotes those pharmaceutically acceptable salts formed with inorganic acids such as hydrochloric acid, hydrobromic acid, sulfuric acid, nitric acid, carbonic acid, phosphoric acid, and organic acids selected from aliphatic, cycloaliphatic, aromatic, araliphatic, heterocyclic, carboxylic, and sulfonic classes of organic acids such as formic acid, acetic acid, propionic acid, glycolic acid, gluconic acid, lactic acid, pyruvic acid, oxalic acid, malic acid, maleic acid, maloneic acid, succinic acid, fumaric acid, tartaric acid, citric acid, aspartic acid, ascorbic acid, glutamic acid, anthranilic acid, benzoic acid, cinnamic acid, mandelic acid, embonic acid, phenylacetic acid, methanesulfonic acid, ethanesulfonic acid, p-toluenesulfonic acid, and salicyclic acid.
The term "pharmaceutically acceptable base addition salt" denotes those pharmaceutically acceptable salts formed with an organic or inorganic base. Examples of acceptable inorganic bases include sodium, potassium, ammonium, calcium, magnesium, iron, zinc, copper, manganese, and aluminum salts. Salts derived from pharmaceutically acceptable organic nontoxic bases includes salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-diethylaminoethanol, trimethamine, dicyclohexylamine, lysine, arginine, histidine, caffeine, procaine, hydrabamine, choline, betaine, ethylenediamine, glucosamine, methylglucamine, theobromine, purines, piperizine, piperidine, N-ethylpiperidine, and polyamine resins.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., "Stereochemistry of Organic Compounds", John Wiley & Sons, Inc., New York, 1994. In describing an optically active compound, the prefixes D and L, or R and S, are used to denote the absolute configuration of the molecule about its chiral center(s). The substituents attached to the chiral center under consideration are ranked in accordance with the Sequence Rule of Cahn, Ingold and Prelog. (Cahn et al. Angew. Chem. Inter. Edit. 1966, 5, 385; errata 511). The prefixes D and L or (+) and (-) are employed to designate the sign of rotation of plane-polarized light by the compound, with (-) or L designating that the compound is levorotatory. A compound prefixed with (+) or D is dextrorotatory.

The term "halo", "halogen", and "halide" are used interchangeably herein and denote fluoro, chloro, bromo, or iodo. Particular examples of halogen are fluoro and chloro.

The term "alkyl" denotes a monovalent linear or branched saturated hydrocarbon group of 1 to 12 carbon atoms. In particular embodiments, alkyl has 1 to 7 carbon atoms, and in more particular embodiments 1 to 4 carbon atoms. Examples of alkyl include methyl, ethyl, propyl, isopropyl, n-butyl, iso-butyl, sec-butyl, or tert-butyl. Particular examples of alkyl are methyl, ethyl, n-propyl, iso-propyl and tert-butyl.

The term "alkenyl" denotes a monovalent linear or branched hydrocarbon group of 2 to 7 carbon atoms with at least one double bond. In particular embodiments, alkenyl has 2 to 4 carbon atoms with at least one double bond. Examples of alkenyl include ethenyl, propenyl, prop-2-enyl, and n-butenyl. Particular examples of alkenyl are propenyl and butenyl.

The term "alkynyl" denotes a monovalent linear or branched saturated hydrocarbon group of 2 to 7 carbon atoms comprising one, two or three triple bonds. In particular embodiments alkynyl has from 2 to 4 carbon atoms comprising one or two triple bonds. Examples of alkynyl include ethynyl, propynyl, prop-2-ynyl, isopropynyl, and n-butynyl.

The term "alkoxy" denotes a group of the formula -O-R', wherein R' is an alkyl group. Examples of alkoxy moieties include methoxy, ethoxy, isopropoxy, and tert-butoxy. Particular example of alkoxy is methoxy.

The term "haloalkyl" denotes an alkyl group wherein at least one of the hydrogen atoms of the alkyl group has been replaced by same or different halogen atoms, particularly fluoro atoms. Examples of haloalkyl include monofluoro-, difluoro- or trifluoro-methyl, -ethyl or -propyl, for example 3,3,3-trifluoropropyl, 2-fluoroethyl, 2,2,2-trifluoroethyl, fluoromethyl, or trifluoromethyl. The term "perhaloalkyl" denotes an alkyl group where all hydrogen atoms of the alkyl group have been replaced by the same or different halogen atoms. Particular example of haloalkyl is trifluoromethyl.

The term "haloalkoxy" denotes an alkoxy group wherein at least one of the hydrogen atoms of the alkoxy group has been replaced by same or different halogen atoms, particularly fluoro atoms. Examples of haloalkoxyl include monofluoro-, difluoro- or trifluoro-methoxy, - ethoxy or -propoxy, for example 3,3,3-trifluoropropoxy, 2-fluoroethoxy, 2,2,2-trifluoroethoxy, fluoromethoxy, or trifluoromethoxy. The term "perhaloalkoxy" denotes an alkoxy group where all hydrogen atoms of the alkoxy group have been replaced by the same or different halogen atoms.

The term "cyanoalkyl" denotes an alkyl group wherein at least one of the hydrogen atoms of the alkyl group has been replaced by a cyano group. Examples of cyanoalkyl include cyanomethyl, cyanoethyl, cyanopropyl, cyano-isopropyl, cyano-isobutyl, cyano-sec-butyl, cyano-tert-butyl, cyanopentyl or cyanohexyl. Particular example of cyanoalkyl is cyanomethyl.

The term "hydroxyalkyl" denotes an alkyl group wherein at least one of the hydrogen atoms of the alkyl group has been replaced by a hydroxy group. Examples of hydroxyalky include hydroxymethyl, 2-hydroxyethyl, 2-hydroxypropyl, 3-hydroxypropyl, 1-(hydroxymethyl)-2-methylpropyl, 2-hydroxybutyl, 3-hydroxybutyl, 4-hydroxybutyl, 2,3-dihydroxypropyl, 2-hydroxy-1-hydroxymethylethyl, 2,3-dihydroxybutyl, 3,4-dihydroxybutyl or 2-(hydroxymethyl)-3-hydroxypropyl. Particular example of hydroxyalkyl is hydroxyethyl.

The term "cycloalkyl" denotes a monovalent saturated monocyclic or bicyclic hydrocarbon group of 3 to 10 ring carbon atoms. In particular embodiments cycloalkyl denotes a monovalent saturated monocyclic hydrocarbon group of 3 to 8 ring carbon atoms. Bicyclic means consisting of two saturated carbocycles having one or more carbon atoms in common. Particular cycloalkyl groups are monocyclic. Examples for monocyclic cycloalkyl are cyclopropyl, cyclobutanyl, cyclopentyl, cyclohexyl or cycloheptyl. Examples for bicyclic cycloalkyl are bicyclo[2.2.1]heptanyl, or bicyclo[2.2.2]octanyl. Particular example of cycloalkyl is cyclopropyl.

The term "heterocycloalkyl" denotes a monovalent saturated or partly unsaturated mono-or bicyclic ring system of 3 to 9 ring atoms, comprising 1, 2, or 3 ring heteroatoms selected from N, O and S, the remaining ring atoms being carbon. In particular embodiments, heterocycloalkyl is a monovalent saturated monocyclic ring system of 4 to 7 ring atoms, comprising 1, 2, or 3 ring heteroatoms selected from N, O and S, the remaining ring atoms being carbon. Examples for monocyclic saturated heterocycloalkyl are aziridinyl, oxiranyl, azetidinyl, oxetanyl, pyrrolidinyl, tetrahydrofuranyl, tetrahydro-thienyl, pyrazolidinyl, imidazolidinyl, oxazolidinyl, isoxazolidinyl, thiazolidinyl, piperidinyl, tetrahydropyranyl, tetrahydrothiopyranyl, piperazinyl, morpholinyl, thiomorpholinyl, 1,1-dioxo-thiomorpholin-4-yl, azepanyl, diazepanyl, homopiperazinyl, or oxazepanyl. Examples for bicyclic saturated heterocycloalkyl are 8-aza-bicyclo[3.2.1]octyl, quinuclidinyl, 8-oxa-3-aza-bicyclo[3.2.1]octyl, 9-aza-bicyclo[3.3.1]nonyl, 3-oxa-9-aza-bicyclo[3.3.1]nonyl, or 3-thia-9-aza-bicyclo[3.3.1]nonyl. Examples for partly unsaturated heterocycloalkyl are dihydrofuryl, imidazolinyl, dihydro-oxazolyl, tetrahydro-pyridinyl, or dihydropyranyl. Particular examples of heterocycloalkyl are pyrrolidinyl, piperidinyl, tetrahydrofuranyl, tetrahydropyridinyl, tetrahydropyranyl, and dihydropyranyl. Most particular examples of heterocycloalkyl are pyrrolidinyl, and piperidinyl.

The term "aromatic" denotes the conventional idea of aromaticity as defined in the literature, in particular in IUPAC - Compendium of Chemical Terminology, 2nd, A. D. McNaught & A. Wilkinson (Eds). Blackwell Scientific Publications, Oxford (1997).

The term "aryl" denotes a monovalent aromatic carbocyclic mono- or bicyclic ring system comprising 6 to 10 carbon ring atoms. Examples of aryl moieties include phenyl and naphthyl. Particular example of aryl is phenyl.

The term "heteroaryl" denotes a monovalent aromatic heterocyclic mono- or bicyclic ring system of 5 to 12 ring atoms, comprising 1, 2, 3 or 4 heteroatoms selected from N, O and S, the remaining ring atoms being carbon. Examples of heteroaryl moieties include pyrrolyl, furanyl, thienyl, imidazolyl, oxazolyl, thiazolyl, triazolyl, oxadiazolyl, thiadiazolyl, tetrazolyl, pyridinyl, pyrazinyl, pyrazolyl, pyridazinyl, pyrimidinyl, triazinyl, azepinyl, diazepinyl, isoxazolyl, benzofuranyl, isothiazolyl, benzothienyl, indolyl, isoindolyl, isobenzofuranyl, benzimidazolyl, benzoxazolyl, benzoisoxazolyl, benzothiazolyl, benzoisothiazolyl, benzooxadiazolyl, benzothiadiazolyl, benzotriazolyl, purinyl, quinolinyl, isoquinolinyl, quinazolinyl, or quinoxalinyl. Particular examples of heteroaryl are imidazolyl and pyridazinyl.

The term "thiophenyl" is synonymous with "thiofuranyl" and "thienyl" and denotes a thiophene substituent.

The term "alkylene" denotes a linear saturated divalent hydrocarbon group of 1 to 7 carbon atoms or a divalent branched saturated divalent hydrocarbon group of 3 to 7 carbon atoms. Examples of alkylene groups include methylene, ethylene, propylene, 2-methylpropylene, butylene, 2-ethylbutylene, pentylene, hexylene. Particular example of alkylene is ethylene, most particularly :CH(CH₃).

The term "alkenylene" denotes a linear divalent hydrocarbon chain of 2 to 7 carbon atoms or a branched divalent hydrocarbon chain of 3 to 7 carbon atoms with at least one double bond. Exemplary alkenylene include ethenylene, 2,2-dimethylethenylene, propenylene, 2-methylpropenylene, butenylene, and pentenylene. Particular example of alkenylene is ethenylene, most particularly :C(=CH₂).

The term "oxo" denotes a divalent oxygen atom =O.

The term "acyl" or "alkylcarbonyl" denotes a group of the formula -C(O)-R' in which R' is alkyl.

The term "protecting group" denotes the group which selectively blocks a reactive site in a multifunctional compound such that a chemical reaction can be carried out selectively at another unprotected reactive site in the meaning conventionally associated with it in synthetic chemistry. Protecting groups can be removed at the appropriat point. Exemplary protecting groups are amino-protecting groups, carboxy-protecting groups or hydroxy-protecting groups.

The term "amino-protecting group" denotes groups intended to protect an amino group and includes benzyl, benzyloxycarbonyl (carbobenzyloxy, CBZ), Fmoc (9-Fluorenylmethyloxycarbonyl), p-methoxybenzyloxycarbonyl, p-nitrobenzyloxycarbonyl, tert-butoxycarbonyl (BOC), and trifluoroacetyl. Further examples of these groups are found in T. W. Greene and P. G. M. Wuts, "Protective Groups in Organic Synthesis", 2nd ed., John Wiley & Sons, Inc., New York, NY, 1991, chapter 7; E. Haslam, "Protective Groups in Organic Chemistry", J. G. W. McOmie, Ed., Plenum Press, New York, NY, 1973, Chapter 5, and T.W. Greene, "Protective Groups in Organic Synthesis", John Wiley and Sons, New York, NY, 1981. The term "protected amino group" refers to an amino group substituted by an amino-protecting groups.

The term "deprotection" or "deprotecting" denotes the process by which a protective group is removed after the selective reaction is completed. Deprotecting reagents include acids, bases or hydrogen, in particular potassium or sodium carbonates, lithium hydroxide in alcoholic solutions, zinc in methanol, acetic acid, trifluoroacetic acid, palladium catalysts, or boron tribromide.

The term "leaving group" denotes the group with the meaning conventionally associated with it in synthetic organic chemistry, i.e., an atom or group displaceable under substitution reaction conditions. Examples of leaving groups include halogen, alkane- or arylenesulfonyloxy, such as methanesulfonyloxy, ethanesulfonyloxy, thiomethyl, benzenesulfonyloxy, tosyloxy, and thienyloxy, dihalophosphinoyloxy, optionally substituted benzyloxy, isopropyloxy, and acyloxy.

The term "halogenating agent" denotes a reagent that incorporates a halogen atom into a molecule in substitution of a hydrogen atom. Particular halogenating agent is a "chlorinating agent" which denotes a reagent that incorporates a chlorine atom into a molecule in substitution of a hydrogen atom. An example of a chlorinating agent is N-chlorosuccinimide (NCS).

The term "pharmaceutical composition" (or "composition") denotes a mixture or solution comprising a therapeutically effective amount of an active pharmaceutical ingredient together with pharmaceutically acceptable excipients to be administered to a mammal, e.g., a human in need thereof.

The term "pharmaceutically acceptable" denotes an attribute of a material which is useful in preparing a pharmaceutical composition that is generally safe, non-toxic, and neither biologically nor otherwise undesirable and is acceptable for veterinary as well as human pharmaceutical use.

The terms "pharmaceutically acceptable excipient" and "therapeutically inert excipient" can be used interchangeably and denote any pharmaceutically acceptable ingredient in a pharmaceutical composition having no therapeutic activity and being non-toxic to the subject administered, such as disintegrators, binders, fillers, solvents, buffers, tonicity agents, stabilizers, antioxidants, surfactants, carriers, diluents or lubricants used in formulating pharmaceutical products.

The term "modulator" denotes a molecule that interacts with a target receptor or enzyme thereby affecting its activity. The interactions include e.g. agonistic, antagonistic, or inverse agonistic activity.

The term "inhibitor" denotes a compound which competes with, reduces or prevents the binding of a particular ligand to a particular receptor or enzyme and/or which reduces or prevents the activity of a particular protein, e.g. of a receptor or an enzyme.

The term "subject" denotes a vertebrate. In certain embodiments, the vertebrate is a mammal. Mammals include humans, non-human primates such as chimpanzees and other apes and monkey species, farm animals such as cattle, horses, sheep, goats, and swine, domestic animals such as rabbits, dogs, and cats, laboratory animals including rodents, such as rats, mice, and guinea pigs. In certain embodiments, a mammal is a human. The term subject does not denote a particular age or sex.

The term "half maximal inhibitory concentration" (IC₅₀) denotes the concentration of a particular compound or molecule required for obtaining 50% inhibition of a biological process in vitro. IC₅₀ values can be converted logarithmically to pIC₅₀ values (-log IC₅₀), in which higher values indicate exponentially greater potency. The IC₅₀ value is not an absolute value but depends on experimental conditions e.g. concentrations employed. The IC₅₀ value can be converted to an absolute inhibition constant (Ki) using the Cheng-Prusoff equation (Biochem. Pharmacol. (1973) 22:3099).

The term "half maximal effective concentration" (EC₅₀) denotes the plasma concentration of a particular compound or molecule required for obtaining 50% of the maximum of a particular effect in vivo or in vitro.

The term "therapeutically effective amount" denotes an amount of a compound or molecule of the present invention that, when administered to a subject, (i) treats or prevents the particular disease, condition or disorder, (ii) attenuates, ameliorates or eliminates one or more symptoms of the particular disease, condition, or disorder, or (iii) prevents or delays the onset of one or more symptoms of the particular disease, condition or disorder described herein. The therapeutically effective amount will vary depending on the compound, the disease state being treated, the severity of the disease treated, the age and relative health of the subject, the route and form of administration, the judgement of the attending medical or veterinary practitioner, and other factors.

The term "treating" or "treatment" of a disease state includes inhibiting the disease state, i.e., arresting the development of the disease state or its clinical symptoms, or relieving the disease state, i.e., causing temporary or permanent regression of the disease state or its clinical symptoms.

The term "preventing" or "prevention" of a disease state denotes causing the clinical symptoms of the disease state not to develop in a subject that can be exposed to or predisposed to the disease state, but does not yet experience or display symptoms of the disease state.

In detail, the present invention relates to compounds of formula (I) 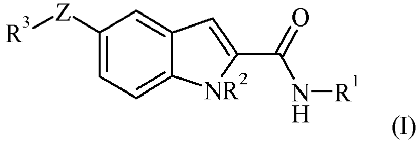 wherein
- Z: is a bond, alkylene, or alkenylene, wherein alkylene and alkenylene are optionally substituted with one, two or three substituents selected independently from the list of halo, cyano, hydroxy, and alkoxy;
- R¹: is aryl or heteroaryl, wherein each is optionally substituted with one, two or three substituents selected independently from the list of halo, cyano, alkyl, haloalkyl, hydroxyalkyl, alkoxy and haloalkoxy;
- R²: is hydrogen, alkyl, haloalkyl, cyanoalkyl, or hydroxyalkyl;
- R³: is saturated or partially unsaturated heterocycloalkyl which is substituted with one, two, three or four R⁴;
- R⁴: is hydrogen, alkyl, alkenyl, alkynyl, cycloalkyl, heterocycloalkyl, aryl, heteroaryl, hydroxy, alkoxy, oxo, -C(O)-R⁵, or -S(O)₂-R⁶; wherein alkyl, alkenyl, alkynyl, cycloalkyl, heterocycloalkyl, aryl, heteroaryl, and alkoxy are optionally substituted by one, two or three R⁷;
- R⁵: is hydrogen, alkyl, haloalkyl, cyanoalkyl, hydroxyalkyl, alkoxyalkyl, cycloalkyl, aryl, hydroxy, or alkoxy;
- R⁶: is hydrogen, alkyl, haloalkyl, cyanoalkyl, hydroxyalkyl, alkoxyalkyl, cycloalkyl, aryl, haloaryl, heteroaryl or alkylheteroaryl;
- R⁷: is halo, haloalkyl, cyano, hydroxy, alkoxy, cycloalkyl, heterocycloalkyl, aryl or -C(O)O-alkyl;
and pharmaceutically acceptable salts and esters thereof.

Particular embodiments of present invention are compounds of formula (I) and pharmaceutically acceptable salts thereof and pharmaceutically acceptable esters thereof.

Further, it is to be understood that every embodiment relating to a specific residue Z, R¹, R², R³, R⁴, R⁵, R⁶ or R⁷ as disclosed herein may be combined with any other embodiment relating to another residue Z, R¹, R², R³, R⁴, R⁵, R⁶ or R⁷ as disclosed herein.

A particular embodiment of the present invention relates to compounds of formula (I), wherein Z is a bond, alkylene, or alkenylene.

A particular embodiment of the present invention relates to compounds of formula (I), wherein Z is a bond, -CH(CH₃)-, or -C(=CH₂)-.

A particular embodiment of the present invention relates to compounds of formula (I), wherein Z is a bond.

A particular embodiment of the present invention relates to compounds of formula (I), wherein R¹ is aryl or heteroaryl, wherein each is substituted with one or two substituents selected independently from the list of halo, cyano, alkyl, haloalkyl, and alkoxy.

A particular embodiment of the present invention relates to compounds of formula (I), wherein R¹ is aryl substituted with one or two halo.

A particular embodiment of the present invention relates to compounds of formula (I), wherein R¹ is phenyl or pyridinyl, wherein each is substituted with one or two substituents selected independently from the list of halo, cyano, alkyl, haloalkyl, and alkoxy.

A particular embodiment of the present invention relates to compounds of formula (I), wherein R¹ is phenyl substituted with one fluoro, chloro, methyl, *tert*-butyl, trifluoromethyl or methoxy; or R¹ is phenyl substituted with two substituents selected independently from the list of fluoro, chloro, cyano and methyl; or R¹ is pyridinyl substituted with one fluoro.

A particular embodiment of the present invention relates to compounds of formula (I), wherein R¹ is phenyl substituted with one or two fluoro.

A particular embodiment of the present invention relates to compounds of formula (I), wherein R¹ is 3-fluorophenyl or 3,5-difluorophenyl.

A particular embodiment of the present invention relates to compounds of formula (I), wherein R¹ is 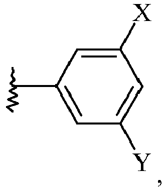 wherein X is fluoro and Y is hydrogen, fluoro, chloro, cyano or methyl, most particularly wherein X is fluoro and Y is hydrogen or fluoro.

A particular embodiment of the present invention relates to compounds of formula (I), wherein R¹ is not dichloro-pyridinyl.

A particular embodiment of the present invention relates to compounds of formula (I), wherein R¹ is not 3,5-dichloro-pyridine-4-yl.

A particular embodiment of the present invention relates to compounds of formula (I), wherein R² is hydrogen or cyanoalkyl.

A particular embodiment of the present invention relates to compounds of formula (I), wherein R² is hydrogen or cyanomethyl.

A particular embodiment of the present invention relates to compounds of formula (I), wherein R² is hydrogen.

A particular embodiment of the present invention relates to compounds of formula (I), wherein R³ is a saturated or partly unsaturated mono- or bicyclic ring system of 5 to 7 ring atoms, comprising 1 or 2 ring heteroatoms selected from N and O the remaining ring atoms being carbon, which is substituted by one, two, three or four R⁴.

A particular embodiment of the present invention relates to compounds of formula (I), wherein R³ is a saturated monocyclic ring system of 5 or 6 ring atoms, comprising one nitrogen ring heteroatom the remaining ring atoms being carbon, which is substituted by one R⁴.

A particular embodiment of the present invention relates to compounds of formula (I), wherein R³ is pyrrolidinyl, piperidinyl, tetrahydropyridinyl, tetrahydropyranyl, or dihydropyranyl, wherein each is substituted by one, two, three or four R⁴.

A particular embodiment of the present invention relates to compounds of formula (I), wherein R³ is pyrrolidinyl or piperidinyl, wherein each is substituted by one, two, three or four R⁴.

A particular embodiment of the present invention relates to compounds of formula (I), wherein R³ is pyrrolidinyl substituted by one R⁴ or piperidinyl substituted by one R⁴.

A particular embodiment of the present invention relates to compounds of formula (I), wherein R³ is

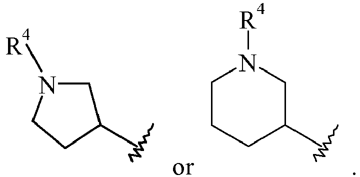

A particular embodiment of the present invention relates to compounds of formula (I), wherein R³ is not bound to Z via a nitrogen atom.

A particular embodiment of the present invention relates to compounds of formula (I), wherein R³ is not piperazinyl.

A particular embodiment of the present invention relates to compounds of formula (I), wherein R³ is not [1,3]dithianyl.

A particular embodiment of the present invention relates to compounds of formula (I), wherein R³ is not 2-methyl-[1,3]dithian-2-yl.

A particular embodiment of the present invention relates to compounds of formula (I), wherein R³ is not imidazolidinyl.

A particular embodiment of the present invention relates to compounds of formula (I), wherein R³ is not 2,5-dioxo-1-imidazolidinyl.

A particular embodiment of the present invention relates to compounds of formula (I), wherein R⁴ is hydrogen, alkyl, alkenyl, cycloalkyl, heteroaryl, oxo, -C(O)-R⁵, or -S(O)₂-R⁶; wherein alkyl, alkenyl, cycloalkyl, and heteroaryl are optionally substituted by one, two or three R⁷.

A particular embodiment of the present invention relates to compounds of formula (I), wherein R⁴ is hydrogen, alkyl, -C(O)-R⁵, or -S(O)₂-R⁶; wherein alkyl is optionally substituted by one R⁷.

A particular embodiment of the present invention relates to compounds of formula (I), wherein R⁴ is hydrogen, methyl, ethyl, n-propyl, isopropyl, propenyl, butenyl, cyclopropyl, pyridazinyl, oxo, -C(O)-R⁵, or -S(O)₂-R⁶; wherein methyl, ethyl and pyridazinyl are optionally substituted by one R⁷.

A particular embodiment of the present invention relates to compounds of formula (I), wherein R⁴ is hydrogen, methyl, cyanomethyl, ethyl, hydroxyethyl, isopropyl, -C(O)-methyl,-C(O)-isopropyl, -C(O)-cyclopropyl, -C(O)-phenyl, -S(O)₂-methyl, -S(O)₂-isopropyl, -S(O)₂-cyclopropyl, or -S(O)₂-phenyl.

A particular embodiment of the present invention relates to compounds of formula (I), wherein R⁵ is alkyl, alkoxyalkyl, cycloalkyl, aryl, or alkoxy.

A particular embodiment of the present invention relates to compounds of formula (I), wherein R⁵ is alkyl, cycloalkyl, or aryl.

A particular embodiment of the present invention relates to compounds of formula (I), wherein R⁵ is methyl, ethyl, isopropyl, methoxymethyl, cyclopropyl, phenyl, methoxy, isopropoxy.

A particular embodiment of the present invention relates to compounds of formula (I), wherein R⁵ is methyl, isopropyl, cyclopropyl, or phenyl.

A particular embodiment of the present invention relates to compounds of formula (I), wherein R⁶ is alkyl, cycloalkyl, aryl, haloaryl, heteroaryl, or alkylheteroaryl.

A particular embodiment of the present invention relates to compounds of formula (I), wherein R⁶ is alkyl, cycloalkyl, or aryl.

A particular embodiment of the present invention relates to compounds of formula (I), wherein R⁶ is methyl, isopropyl, cyclopropyl, phenyl, fluoro-phenyl, or methyl-imidazolyl.

A particular embodiment of the present invention relates to compounds of formula (I), wherein R⁶ is methyl, isopropyl, cyclopropyl, or phenyl.

A particular embodiment of the present invention relates to compounds of formula (I), wherein R⁷ is haloalkyl, cyano, hydroxy, alkoxy, cycloalkyl, heterocycloalkyl, aryl or -C(O)O-alkyl.

A particular embodiment of the present invention relates to compounds of formula (I), wherein R⁷ is haloalkyl, cyano, or hydroxy.

A particular embodiment of the present invention relates to compounds of formula (I), wherein R⁷ is trifluoromethyl, cyano, hydroxy, methoxy, cyclopropyl, tetrahydrofuranyl, phenyl or -C(O)O-methyl.

A particular embodiment of the present invention relates to compounds of formula (I), wherein R⁷ is trifluoromethyl, cyano, or hydroxy.

Particular compounds of formula (I) of present invention are those selected from the group consisting of:
5-((1-Benzylpyrrolidin-3-yl)-N-(3,5-difluorophenyl)-1H-indole-2-carboxamide;
5-((1-Methyl-pyrrolidin-3-yl)-1H-indole-2-carboxylic acid (3,5-difluoro-phenyl)-amide;
5-((1-Benzylpyrrolidin-3-yl)-N-(3-fluorophenyl)-1H-indole-2-carboxamide;
N-(3-Fluorophenyl)-5-(1-methylpyrrolidin-3-yl)-1H-indole-2-carboxamide;
5-((1-Benzylpyrrolidin-3-yl)-N-(3-cyano-5-fluorophenyl)-1H-indole-2-carboxamide;
N-(3-Cyano-5-fluorophenyl)-5-(1-methylpyrrolidin-3-yl)-1H-indole-2-carboxamide;
5-Pyrrolidin-3-yl-1H-indole-2-carboxylic acid (3,5-difluoro-phenyl)-amide;
N-(3-Fluorophenyl)-5-(pyrrolidin-3-yl)-1H-indole-2-carboxamide;
N-(3-cyano-5-fluorophenyl)-5-(pyrrolidin-3-yl)-1H-indole-2-carboxamide;
5-[1-(2,2,2-Trifluoro-ethyl)-pyrrolidin-3-yl]-1H-indole-2-carboxylic acid (3,5-difluoro-phenyl)-amide;
5-((1-Isopropyl-pyrrolidin-3-yl)-1H-indole-2-carboxylic acid (3,5-difluoro-phenyl)-amide;
5-[1-(Propane-2-sulfonyl)-pyrrolidin-3-yl]-1H-indole-2-carboxylic acid (3,5-difluoro-phenyl)-amide;
5-((1-Isobutyryl-pyrrolidin-3-yl)-1H-indole-2-carboxylic acid (3,5-difluoro-phenyl)-amide;
N-(3-Fluorophenyl)-5-(1-isopropylpyrrolidin-3-yl)-1H-indole-2-carboxamide;
N-(3-Fluorophenyl)-5-(1-(2,2,2-trifluoroethyl)pyrrolidin-3-yl)-1H-indole-2-carboxamide;
5-((1-(Cyanomethyl)pyrrolidin-3-yl)-N-(3-fluorophenyl)-1H-indole-2-carboxamide;
5-((1-Benzyl-2,5-dioxopyrrolidin-3-yl)-N-(3-fluorophenyl)-1H-indole-2-carboxamide;
5-((3-Isopropyl-5-oxo-pyrrolidin-3-yl)-1H-indole-2-carboxylic acid (3,4-difluoro-phenyl)-amide;
5-((3-Ethyl-2,6-dioxo-piperidin-3-yl)-1H-indole-2-carboxylic acid (3,5-difluoro-phenyl)-amide;
5-((1-Isopropyl-piperidin-4-yl)-1H-indole-2-carboxylic acid (3-fluoro-phenyl)-amide;
5-((1-Isopropyl-piperidin-3-yl)-1H-indole-2-carboxylic acid (3,5-difluoro-phenyl)-amide;
5-((1-Isopropyl-piperidin-4-yl)-1H-indole-2-carboxylic acid (3-trifluoromethyl-phenyl)-amide;
5-((1-Isopropyl-piperidin-4-yl)-1H-indole-2-carboxylic acid (3-chloro-phenyl)-amide;
5-((1-Isopropyl-piperidin-4-yl)-1H-indole-2-carboxylic acid (2-fluoro-phenyl)-amide;
5-((1-Isopropyl-piperidin-4-yl)-1H-indole-2-carboxylic acid (3-methoxy-phenyl)-amide;
5-((1-Isopropyl-piperidin-4-yl)-1H-indole-2-carboxylic acid (4-methoxy-phenyl)-amide;
5-((1-Isopropyl-piperidin-4-yl)-1H-indole-2-carboxylic acid (4-tert-butyl-phenyl)-amide;
5-((1-Isopropyl-piperidin-4-yl)-1H-indole-2-carboxylic acid (3,4-difluoro-phenyl)-amide;
5-((1-Isopropyl-piperidin-4-yl)-1H-indole-2-carboxylic acid (2,4-difluoro-phenyl)-amide;
N-(3-Chloro-5-fluorophenyl)-5-(1-isopropylpiperidin-3-yl)-1H-indole-2-carboxamide;
5-((1-Isopropyl-piperidin-3-yl)-1H-indole-2-carboxylic acid (3-cyano-5-fluoro-phenyl)-amide;
5-((1-Isopropyl-piperidin-4-yl)-1H-indole-2-carboxylic acid (6-fluoro-pyridin-3-yl)-amide;
5-((1-Isopropyl-piperidin-3-yl)-1H-indole-2-carboxylic acid m-tolylamide;
N-(3-Fluoro-5-methylphenyl)-5-(1-isopropylpiperidin-3-yl)-1H-indole-2-carboxamide;
5-((1-Benzoyl-piperidin-4-yl)-1H-indole-2-carboxylic acid (4-fluoro-phenyl)-amide;
5-((1-Methyl-piperidin-4-yl)-1H-indole-2-carboxylic acid (4-fluoro-phenyl)-amide;
5-[1-(2-Methoxy-ethyl)-piperidin-4-yl]-1H-indole-2-carboxylic acid (4-fluoro-phenyl)-amide;
5-((1-Benzyl-piperidin-4-yl)-1H-indole-2-carboxylic acid (4-fluoro-phenyl)-amide;
5-[1-Allyl-piperidin-4-yl]-1H-indole-2-carboxylic acid (4-fluoro-phenyl)-amide;
5-[1-Propyl-piperidin-4-yl]-1H-indole-2-carboxylic acid (4-fluoro-phenyl)-amide;
4-[2-(4-Fluoro-phenylcarbamoyl)-1H-indol-5-yl]-piperidine-1-carboxylic acid methyl ester;
5-[1-Ethyl-piperidin-4-yl]-1H-indole-2-carboxylic acid (4-fluoro-phenyl)-amide;
{4-[2-(4-Fluoro-phenylcarbamoyl)-1H-indol-5-yl]-piperidin-1-yl}-acetic acid methyl ester;
5-((1-Propionyl-piperidin-4-yl)-1H-indole-2-carboxylic acid (4-fluoro-phenyl)-amide;
5-((1-Phenethyl-piperidin-4-yl)-1H-indole-2-carboxylic acid (4-fluoro-phenyl)-amide;
5-((1-Isopropyl-piperidin-3-yl)-1H-indole-2-carboxylic acid (3-fluoro-phenyl)-amide;
5-((1-Isopropyl-piperidin-3-yl)-1H-indole-2-carboxylic acid (4-fluoro-phenyl)-amide;
5-((1-Isopropyl-piperidin-4-yl)-1H-indole-2-carboxylic acid (4-fluoro-phenyl)-amide;
5-((1-Methanesulfonyl-piperidin-3-yl)-1H-indole-2-carboxylic acid (3,5-difluoro-phenyl)-amide;
5-[1-(Propane-2-sulfonyl)-piperidin-3-yl]-1H-indole-2-carboxylic acid (3,5-difluoro-phenyl)-amide;
5-((1-Isobutyryl-piperidin-3-yl)-1H-indole-2-carboxylic acid (3,5-difluoro-phenyl)-amide;
5-((1-Acetyl-piperidin-3-yl)-1H-indole-2-carboxylic acid (3,5-difluoro-phenyl)-amide;
5-((1-Benzenesulfonyl-piperidin-3-yl)-1H-indole-2-carboxylic acid (3,5-difluoro-phenyl)-amide;
5-((1-Cyclopropanecarbonyl-piperidin-3-yl)-1H-indole-2-carboxylic acid (3,5-difluoro-phenyl)-amide;
5-((1-Benzoyl-piperidin-3-yl)-1H-indole-2-carboxylic acid (3,5-difluoro-phenyl)-amide;
5-((1-Cyclopropanesulfonyl-piperidin-3-yl)-1H-indole-2-carboxylic acid (3,5-difluoro-phenyl)-amide;
5-[1-(2-Methoxy-acetyl)-piperidin-3-yl]-1H-indole-2-carboxylic acid (3,5-difluoro-phenyl)-amide;
5-[1-(1-Methyl-1H-imidazole-4-sulfonyl)-piperidin-3-yl]-1H-indole-2-carboxylic acid (3,5-difluoro-phenyl)-amide;
5-((1-Methyl-piperidin-3-yl)-1H-indole-2-carboxylic acid (3,5-difluoro-phenyl)-amide;
5-[1-(2-Methoxy-ethyl)-piperidin-3-yl]-1H-indole-2-carboxylic acid (3,5-difluoro-phenyl)-amide;
{3-[2-(3,5-Difluoro-phenylcarbamoyl)-1H-indol-5-yl]-piperidin-1-yl}-acetic acid methyl ester;
5-Piperidin-3-yl-1H-indole-2-carboxylic acid (3,5-difluoro-phenyl)-amide;
5-((1-Isopropyl-piperidin-4-ylamino)-1H-indole-2-carboxylic acid (3,4-difluoro-phenyl)-amide;
N-(3-Fluorophenyl)-5-(1-(2-methoxyethyl)piperidin-3-yl)-1H-indole-2-carboxamide;
N-(3-Fluorophenyl)-5-(1-(2-hydroxyethyl)piperidin-3-yl)-1H-indole-2-carboxamide;
N-(3-Fluorophenyl)-5-(1-((tetrahydrofuran-2-yl)methyl)piperidin-3-yl)-1H-indole-2-carboxamide;
N-(3-Fluorophenyl)-5-(piperidin-3-yl)-1H-indole-2-carboxamide;
5-((1-Ethylpiperidin-3-yl)-N-(3-fluorophenyl)-1H-indole-2-carboxamide;
5-((1-(Cyclopropylmethyl)piperidin-3-yl)-N-(3-fluorophenyl)-1H-indole-2-carboxamide;
5-((1-Cyclopropylpiperidin-3-yl)-N-(3-fluorophenyl)-1H-indole-2-carboxamide;
N-(3-Fluorophenyl)-5-(1-(2,2,2-trifluoroethyl)piperidin-3-yl)-1H-indole-2-carboxamide;
5-((1-Benzyl-pyrrolidin-3-yl)-1H-indole-2-carboxylic acid (5-fluoro-pyridin-3-yl)-amide;
N-(5-Fluoropyridin-3-yl)-5-(pyrrolidin-3-yl)-1H-indole-2-carboxamide;
N-(5-Fluoropyridin-3-yl)-5-(1-isopropylpyrrolidin-3-yl)-1H-indole-2-carboxamide;
Isopropyl 3-(2-(5-fluoropyridin-3-ylcarbamoyl)-1H-indol-5-yl)pyrrolidine-1-carboxylate;
5-((1-(But-3-enyl)pyrrolidin-3-yl)-N-(5-fluoropyridin-3-yl)-1H-indole-2-carboxamide;
5-((1-(Cyclopropylmethyl)pyrrolidin-3-yl)-N-(5-fluoropyridin-3-yl)-1H-indole-2-carboxamide;
5-((1-Cyanomethyl-pyrrolidin-3-yl)-1H-indole-2-carboxylic acid (5-fluoro-pyridin-3-yl)-amide;
1-Cyanomethyl-5-(1-cyanomethyl-pyrrolidin-3-yl)-1H-indole-2-carboxylic acid (5-fluoro-pyridin-3-yl)-amide;
N-(5-Fluoropyridin-3-yl)-5-(1-(2-methoxyacetyl)pyrrolidin-3-yl)-1H-indole-2-carboxamide;
5-((1-(6-Cyanopyridazin-3-yl)pyrrolidin-3-yl)-N-(5-fluoropyridin-3-yl)-1H-indole-2-carboxamide;
5-((1-(4-Fluorophenylsulfonyl)pyrrolidin-3-yl)-N-(5-fluoropyridin-3-yl)-1H-indole-2-carboxamide;
5-((5,6-Dihydro-4H-pyran-3-yl)-1H-indole-2-carboxylic acid (3,4-difluoro-phenyl)-amide;
5-((Tetrahydro-pyran-3-yl)-1H-indole-2-carboxylic acid (3,4-difluoro-phenyl)-amide;
5-((1-Isopropyl-1,2,3,6-tetrahydro-pyridin-4-yl)-1H-indole-2-carboxylic acid (3,5-difluorophenyl)-amide;
5-((1-(1-Benzylpyrrolidin-3-yl)vinyl)-N-(3,5-difluorophenyl)-1H-indole-2-carboxamide;
N-(3,5-Difluorophenyl)-5-(1-(pyrrolidin-3-yl)ethyl)-1H-indole-2-carboxamide;
N-(3,5-Difluorophenyl)-5-(1-(1-isopropylpyrrolidin-3-yl)ethyl)-1H-indole-2-carboxamide;
5-((1-(1-(Cyanomethyl)pyrrolidin-3-yl)ethyl)-N-(3,5-difluorophenyl)-1H-indole-2-carboxamide;
5-((1-(1-Acetylpyrrolidin-3-yl)ethyl)-N-(3,5-difluorophenyl)-1H-indole-2-carboxamide;
N-(3,5-Difluorophenyl)-5-(1-(1-(2,2,2-trifluoroethyl)pyrrolidin-3-yl)ethyl)-1H-indole-2-carboxamide;
5-(((S)-1-Methyl-pyrrolidin-3-yl)-1H-indole-2-carboxylic acid (3,5-difluoro-phenyl)-amide;
5-(((R)-1-Methyl-pyrrolidin-3-yl)-1H-indole-2-carboxylic acid (3,5-difluoro-phenyl)-amide;
5-[((S)-1-(2,2,2-Trifluoro-ethyl)-pyrrolidin-3-yl]-1H-indole-2-carboxylic acid (3,5-difluorophenyl)-amide;
5-(((R)-1-Isobutyryl-pyrrolidin-3-yl)-1H-indole-2-carboxylic acid (3,5-difluoro-phenyl)-amide;
5-(((S)-1-Isobutyryl-pyrrolidin-3-yl)-1H-indole-2-carboxylic acid (3,5-difluoro-phenyl)-amide;
5-(((S)-1-Isobutyryl-pyrrolidin-3-yl)-1H-indole-2-carboxylic acid (3,5-difluoro-phenyl)-amide;
(R)-5-(1-(Cyanomethyl)pyrrolidin-3-yl)-N-(3-fluorophenyl)-1H-indole-2-carboxamide;
(S)-5-(1-(Cyanomethyl)pyrrolidin-3-yl)-N-(3-fluorophenyl)-1H-indole-2-carboxamide;
5-(((R)-1-Isopropyl-piperidin-3-yl)-1H-indole-2-carboxylic acid (3-fluoro-phenyl)-amide;
5-(((S)-1-Isopropyl-piperidin-3-yl)-1H-indole-2-carboxylic acid (3-fluoro-phenyl)-amide;
5-(((R)-1-Acetyl-piperidin-3-yl)-1H-indole-2-carboxylic acid (3,5-difluoro-phenyl)-amide;
5-(((S)-1-Acetyl-piperidin-3-yl)-1H-indole-2-carboxylic acid (3,5-difluoro-phenyl)-amide;
(R)-N-(3-Fluorophenyl)-5-(1-(2-hydroxyethyl)piperidin-3-yl)-1H-indole-2-carboxamide;
(S)-N-(3-Fluorophenyl)-5-(1-(2-hydroxyethyl)piperidin-3-yl)-1H-indole-2-carboxamide;
(R)-5-(1-Cyclopropylpiperidin-3-yl)-N-(3-fluorophenyl)-1H-indole-2-carboxamide;
(S)-5-(1-Cyclopropylpiperidin-3-yl)-N-(3-fluorophenyl)-1H-indole-2-carboxamide;
N-(3,5-Difluorophenyl)-5-((R)-1-((S)-1-(2,2,2-trifluoroethyl)pyrrolidin-3-yl)ethyl)-1H-indole-2-carboxamide;
N-(3,5-Difluorophenyl)-5-((R)-1-((R)-1-(2,2,2-trifluoroethyl)pyrrolidin-3-yl)ethyl)-1H-indole-2-carboxamide;
5-(((S)-1-((S)-1-Acetylpyrrolidin-3-yl)ethyl)-N-(3,5-difluorophenyl)-1H-indole-2-carboxamide;
and pharmaceutically acceptable salts and esters thereof.

Particular compounds of formula (I) of present invention are those selected from the group consisting of:
5-((1-Methyl-pyrrolidin-3-yl)-1H-indole-2-carboxylic acid (3,5-difluoro-phenyl)-amide;
N-(3-Fluorophenyl)-5-(1-methylpyrrolidin-3-yl)-1H-indole-2-carboxamide;
5-Pyrrolidin-3-yl-1H-indole-2-carboxylic acid (3,5-difluoro-phenyl)-amide;
5-((1-Isopropyl-pyrrolidin-3-yl)-1H-indole-2-carboxylic acid (3,5-difluoro-phenyl)-amide;
5-((1-(Cyanomethyl)pyrrolidin-3-yl)-N-(3-fluorophenyl)-1H-indole-2-carboxamide;
5-((1-Isopropyl-piperidin-3-yl)-1H-indole-2-carboxylic acid (3,5-difluoro-phenyl)-amide;
5-((1-Methanesulfonyl-piperidin-3-yl)-1H-indole-2-carboxylic acid (3,5-difluoro-phenyl)-amide;
5-[1-(Propane-2-sulfonyl)-piperidin-3-yl]-1H-indole-2-carboxylic acid (3,5-difluoro-phenyl)-amide;
5-((1-Isobutyryl-piperidin-3-yl)-1H-indole-2-carboxylic acid (3,5-difluoro-phenyl)-amide;
5-((1-Acetyl-piperidin-3-yl)-1H-indole-2-carboxylic acid (3,5-difluoro-phenyl)-amide;
5-((1-Benzenesulfonyl-piperidin-3-yl)-1H-indole-2-carboxylic acid (3,5-difluoro-phenyl)-amide;
5-((1-Cyclopropanecarbonyl-piperidin-3-yl)-1H-indole-2-carboxylic acid (3,5-difluoro-phenyl)-amide;
5-((1-Benzoyl-piperidin-3-yl)-1H-indole-2-carboxylic acid (3,5-difluoro-phenyl)-amide;
5-((1-Cyclopropanesulfonyl-piperidin-3-yl)-1H-indole-2-carboxylic acid (3,5-difluoro-phenyl)-amide;
5-((1-Methyl-piperidin-3-yl)-1H-indole-2-carboxylic acid (3,5-difluoro-phenyl)-amide;
5-Piperidin-3-yl-1H-indole-2-carboxylic acid (3,5-difluoro-phenyl)-amide;
5-(((S)-1-Methyl-pyrrolidin-3-yl)-1H-indole-2-carboxylic acid (3,5-difluoro-phenyl)-amide;
5-(((R)-1-Methyl-pyrrolidin-3-yl)-1H-indole-2-carboxylic acid (3,5-difluoro-phenyl)-amide;
(S)-5-(1-(Cyanomethyl)pyrrolidin-3-yl)-N-(3-fluorophenyl)-1H-indole-2-carboxamide;
5-(((R)-1-Isopropyl-piperidin-3-yl)-1H-indole-2-carboxylic acid (3-fluoro-phenyl)-amide;
5-(((R)-1-Acetyl-piperidin-3-yl)-1H-indole-2-carboxylic acid (3,5-difluoro-phenyl)-amide;
(R)-N-(3-Fluorophenyl)-5-(1-(2-hydroxyethyl)piperidin-3-yl)-1H-indole-2-carboxamide;
and pharmaceutically acceptable salts and esters thereof.

A particular embodiment of the present invention relates to compounds of formula (Ia) 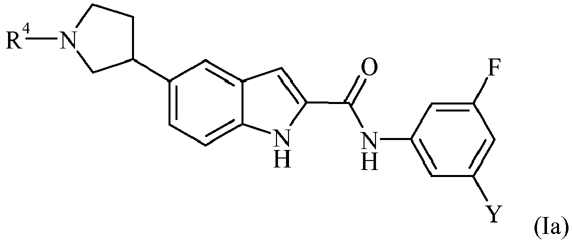 wherein R⁴ is as described herein and Y is hydrogen or fluoro.

Particular compounds of formula (Ia) of present invention are those selected from the group consisting of:
5-((1-Benzylpyrrolidin-3-yl)-N-(3-fluorophenyl)-1H-indole-2-carboxamide;
N-(3-Fluorophenyl)-5-(1-methylpyrrolidin-3-yl)-1H-indole-2-carboxamide;
N-(3-Fluorophenyl)-5-(pyrrolidin-3-yl)-1H-indole-2-carboxamide;
N-(3-Fluorophenyl)-5-(1-isopropylpyrrolidin-3-yl)-1H-indole-2-carboxamide;
N-(3-Fluorophenyl)-5-(1-(2,2,2-trifluoroethyl)pyrrolidin-3-yl)-1H-indole-2-carboxamide;
5-((1-(Cyanomethyl)pyrrolidin-3-yl)-N-(3-fluorophenyl)-1H-indole-2-carboxamide;
(R)-5-(1-(Cyanomethyl)pyrrolidin-3-yl)-N-(3-fluorophenyl)-1H-indole-2-carboxamide;
(S)-5-(1-(Cyanomethyl)pyrrolidin-3-yl)-N-(3-fluorophenyl)-1H-indole-2-carboxamide;
5-((1-Benzylpyrrolidin-3-yl)-N-(3,5-difluorophenyl)-1H-indole-2-carboxamide;
5-((1-Methyl-pyrrolidin-3-yl)-1H-indole-2-carboxylic acid (3,5-difluoro-phenyl)-amide;
5-Pyrrolidin-3-yl-1H-indole-2-carboxylic acid (3,5-difluoro-phenyl)-amide;
5-[1-(2,2,2-Trifluoro-ethyl)-pyrrolidin-3-yl]-1H-indole-2-carboxylic acid (3,5-difluoro-phenyl)-amide;
5-((1-Isopropyl-pyrrolidin-3-yl)-1H-indole-2-carboxylic acid (3,5-difluoro-phenyl)-amide;
5-(((S)-1-Methyl-pyrrolidin-3-yl)-1H-indole-2-carboxylic acid (3,5-difluoro-phenyl)-amide;
5-(((R)-1-Methyl-pyrrolidin-3-yl)-1H-indole-2-carboxylic acid (3,5-difluoro-phenyl)-amide;
5-[((S)-1-(2,2,2-Trifluoro-ethyl)-pyrrolidin-3-yl]-1H-indole-2-carboxylic acid (3,5-difluorophenyl)-amide;
and pharmaceutically acceptable salts and esters thereof.

A particular embodiment of the present invention relates to compounds of formula (Ib) 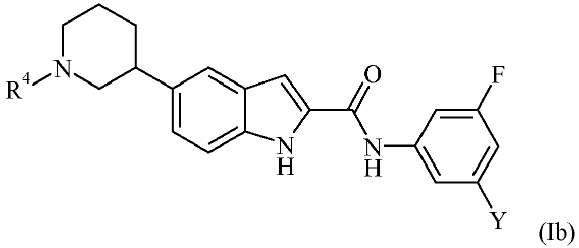 wherein R⁴ is as described herein and Y is hydrogen or fluoro.

Particular compounds of formula (Ib) of present invention are those selected from the group consisting of:
5-((1-Isopropyl-piperidin-3-yl)-1H-indole-2-carboxylic acid (3-fluoro-phenyl)-amide;
5-((1-Isopropyl-piperidin-3-yl)-1H-indole-2-carboxylic acid (4-fluoro-phenyl)-amide;
N-(3-Fluorophenyl)-5-(piperidin-3-yl)-1H-indole-2-carboxamide;
N-(3-Fluorophenyl)-5-(1-(2,2,2-trifluoroethyl)piperidin-3-yl)-1H-indole-2-carboxamide;
5-(((R)-1-Isopropyl-piperidin-3-yl)-1H-indole-2-carboxylic acid (3-fluoro-phenyl)-amide;
5-(((S)-1-Isopropyl-piperidin-3-yl)-1H-indole-2-carboxylic acid (3-fluoro-phenyl)-amide;
5-((1-Isopropyl-piperidin-3-yl)-1H-indole-2-carboxylic acid (3,5-difluoro-phenyl)-amide;
5-((1-Methanesulfonyl-piperidin-3-yl)-1H-indole-2-carboxylic acid (3,5-difluoro-phenyl)-amide;
5-[1-(Propane-2-sulfonyl)-piperidin-3-yl]-1H-indole-2-carboxylic acid (3,5-difluoro-phenyl)-amide;
5-((1-Isobutyryl-piperidin-3-yl)-1H-indole-2-carboxylic acid (3,5-difluoro-phenyl)-amide;
5-((1-Acetyl-piperidin-3-yl)-1H-indole-2-carboxylic acid (3,5-difluoro-phenyl)-amide;
5-((1-Benzenesulfonyl-piperidin-3-yl)-1H-indole-2-carboxylic acid (3,5-difluoro-phenyl)-amide;
5-((1-Cyclopropanecarbonyl-piperidin-3-yl)-1H-indole-2-carboxylic acid (3,5-difluoro-phenyl)-amide;
5-((1-Benzoyl-piperidin-3-yl)-1H-indole-2-carboxylic acid (3,5-difluoro-phenyl)-amide;
5-((1-Cyclopropanesulfonyl-piperidin-3-yl)-1H-indole-2-carboxylic acid (3,5-difluoro-phenyl)-amide;
5-[1-(2-Methoxy-acetyl)-piperidin-3-yl]-1H-indole-2-carboxylic acid (3,5-difluoro-phenyl)-amide;
5-[1-(1-Methyl-1H-imidazole-4-sulfonyl)-piperidin-3-yl]-1H-indole-2-carboxylic acid (3,5-difluoro-phenyl)-amide;
5-((1-Methyl-piperidin-3-yl)-1H-indole-2-carboxylic acid (3,5-difluoro-phenyl)-amide;
5-[1-(2-Methoxy-ethyl)-piperidin-3-yl]-1H-indole-2-carboxylic acid (3,5-difluoro-phenyl)-amide;
{3-[2-(3,5-Difluoro-phenylcarbamoyl)-1H-indol-5-yl]-piperidin-1-yl}-acetic acid methyl ester;
5-Piperidin-3-yl-1H-indole-2-carboxylic acid (3,5-difluoro-phenyl)-amide;
5-((R)-1-Acetyl-piperidin-3-yl)-1H-indole-2-carboxylic acid (3,5-difluoro-phenyl)-amide;
5-(((S)-1-Acetyl-piperidin-3-yl)-1H-indole-2-carboxylic acid (3,5-difluoro-phenyl)-amide
and pharmaceutically acceptable salts and esters thereof.

The invention further relates to a process for the manufacture of compounds of formula (I) as defined above comprising:
a) the reaction of a compound of formula (II) 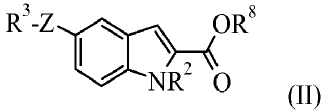 with a compound of formula R¹NH₂; or
b) the reaction of a compound of formula (III) 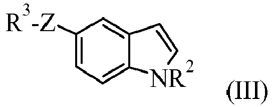 with a compound of formula R¹NCO; or
c) the reaction of a compound of formula (IV) 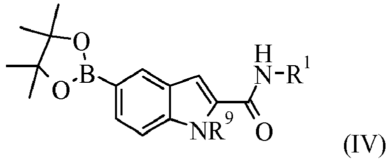 with 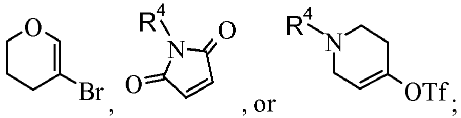 wherein Z, R¹, R², R³ and R⁴ are as defined above, R⁸ is hydrogen or alkyl, R⁹ is either R² as defined above or a protecting group selected from 4-methoxybenzyl bromide and Tf is trifluoromethanesulfonyl.

Particularly, compounds of formula (I) can be prepared following standard methods in accordance with any of Schemes 1 to 7.

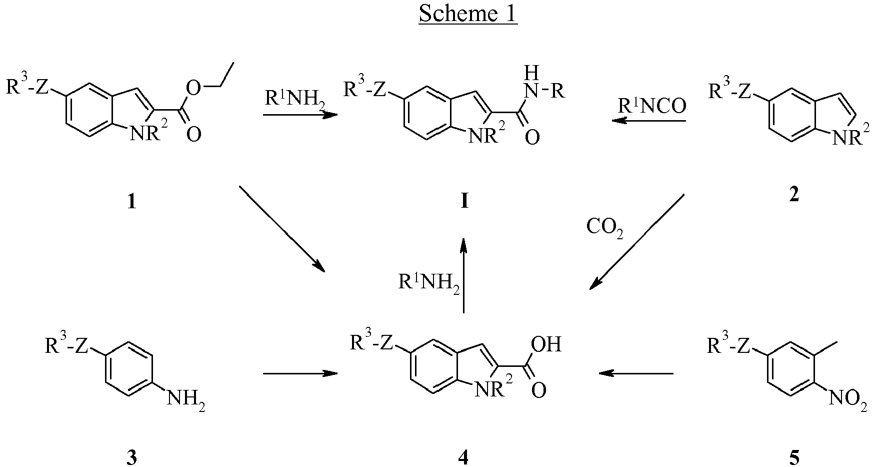

The present compounds of formula (I) and their pharmaceutically acceptable salts can be prepared by condensation of the 2-indol carboxylic acid ester of general formula **1** with an aniline derivative in an inert solvent like dioxane in the presence of trimethylaluminium at room temperature or at elevated temperatures (see Scheme 1). Alternatively compounds of formula (I) can be prepared from indole **2** by deprotonation with a base like n-butyl lithium at low temperature under inert gas in an inert solvent like THF and intermediate protection with carbondioxide. Second deprotonation of the 2-position of the indole with *tert*-butyl lithium and reaction with an isocyanate derivative yields compounds of formula (I). Compounds of formula (I) can be prepared from the 2-indol carboxylic acid **4** by conversion of the acid to the acid chloride (e.g. with thionyl chloride or oxalyl chloride in THF in the presence of DMF) or through activation with a coupling reagent (like HBTU, HATU, EDC etc.) in an inert solvent at ambient or higher reaction temperatures. The indole 2-carboxylic acid derivative **4** can be prepared from either the indole 2 carboxylic acid ester ester **1** through saponification, or by deprotonation of the indole derivative **2** with n-butyllithium, quenching with carbondioxide and deprotonation with *tert*-butyllithium and reaction with carbondioxide, or from the aniline derivative **3** by iodination (e.g. with iodine or other iodine source like NIS in an inert solvent or acid (like TFA) in the presence of a catalyst (like silver sulfate)) and subsequent palladium catalyzed reaction of the intermediate 2-iodo-aniline derivative with puryvic acid in the presence of DABCO and a base or from the 2-nitro-toluene derivative **5** through base catalyzed condensation with diethyl oxalate, reduction of the nitro group and saponification of the ester.

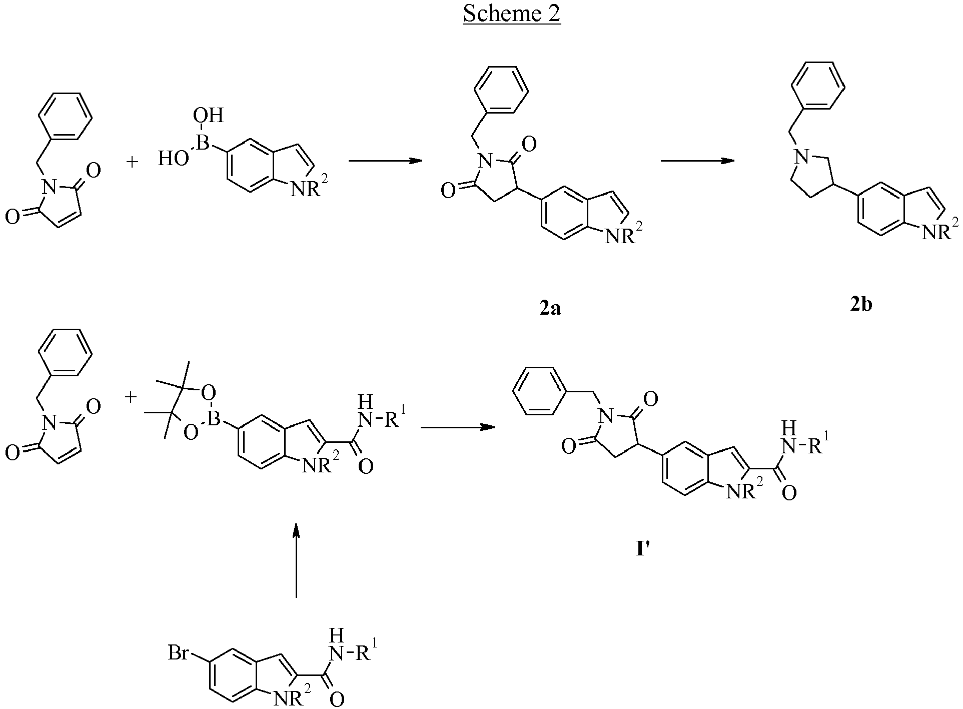

Introduction of a pyrrolidine ring in the 5-position of the indole can be accomplished by a rhodium catalyzed addition of 5-indol boronic acid or 5-indol boronic acid ester with an α,β-unsaturated amide (e.g. benzylmaleimide) at ambient to higher temperature in an inert solvent like dioxane to provide the indole derivative **2a** or the final compound (**I**') (see Scheme 2). The boronic acid ester derivative can be prepared from the 5-bromo-indole derivative through palladium catalyzed boronation with bis(pinacolato)diboron. Reduction of the imide **2a** to the pyrrolidine derivative **2b** can be achieved with a reducing agent like lithium aluminium hydride in an inert solvent like THF at ambient to higher temperatures.

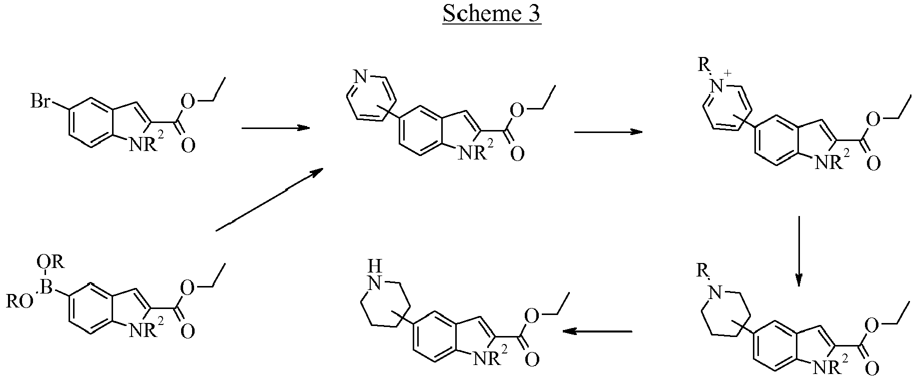

Synthesis of 5-piperidylindoles can be achieved as described in Scheme 3. 5-Bromo-indole 2-carboxylic acid ester can be converted into the pyridine derivatives by the palladium catalyzed coupling with 3- or 4-stannyl pyridine derivatives or 3- or 4-boronic acid pyridine derivatives. Alternatively the reaction of the 5-boron indole ester with 3- or 4-iodopyridine under palladium catalysis leads to the same product. N-alkylation of the pyridine with an alkyl halogenide or alkyl sulfonate in an inert solvent like DMF in the presence of a base like potassium carbonate or N,N-diisopropylethylamine at ambient temperature leads to the pyridinium salts. These can be hydrogenated with hydrogen in the presence of PtO₂ or with sodium borohydride and subsequent hydrogenation with hydrogen in the presence of palladium hydroxide. If the alkyl substituent is a benzyl derivative than this group can be cleaved off by hydrogenation with hydrogen gas in a solvent like ethanol in the presence of an acid and palladium on carbon or through dealkylation with a chloro formiate ester. The resulting amine can be further derivatized through acylation with sulfonyl chlorides, acid chlorides, carboxylic acids or carboxylic acid esters, through alkylation with alkyl halogenides or alkyl sulfates or through reductive amination with aldehydes, ketones or ketales.

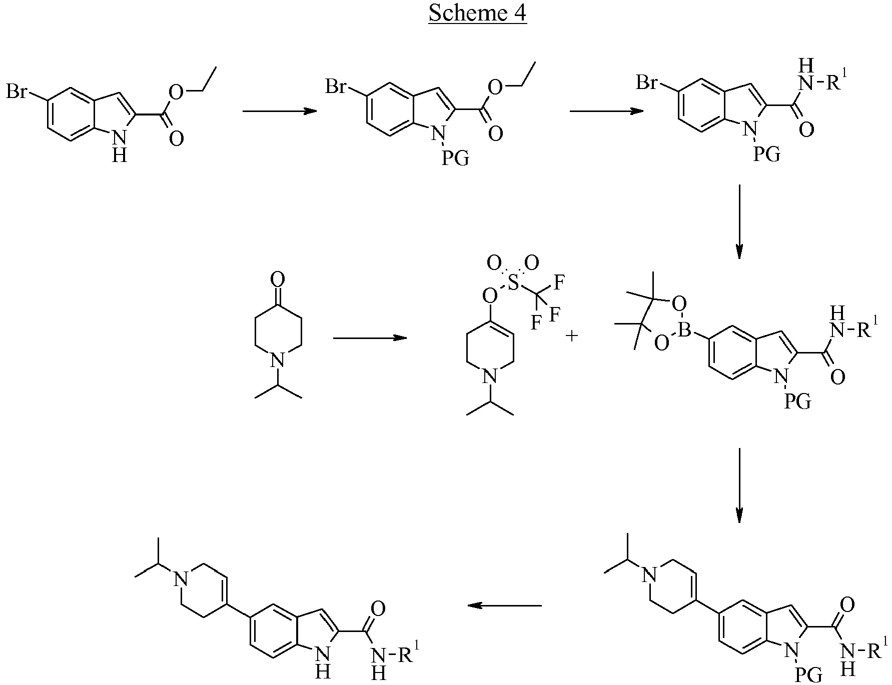

The 1-position of the indole can be alkylated with a protecting group PG if needed for example with 4-methoxybenzyl bromide in an inert solvent like DMF in the presence of a base like potassium carbonate. The boronic acid ester derivative can be coupled with an enol-triflate, which can be prepared from the corresponding ketone with LDA and phenyltrifluoromethanesulfonimide, under palladium catalysis to the 5-piperidinyl derivative. Deprotection for example with TFA in the presence of anisole provides the free indole derivative (Scheme 4.

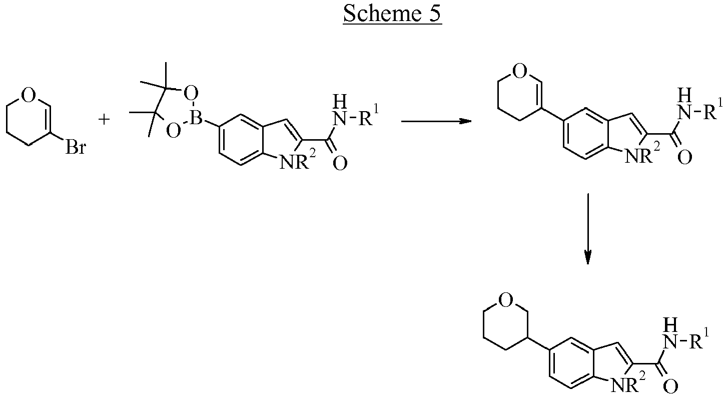

Coupling of a 5-boronic acid indole derivative with a vinyl halogenide can be accomplished without protection under palladium catalysis. Subsequent reduction of the double bond yields the 5-alkyl derivative (Scheme 5).

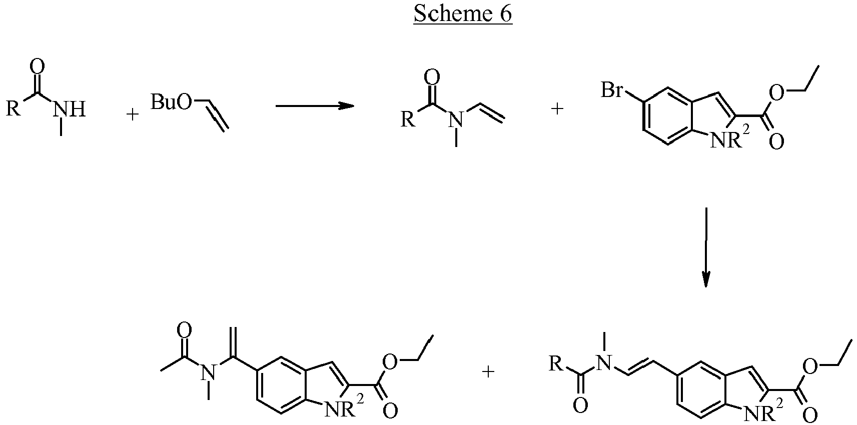

Palladium catalyzed Heck reaction of 5-bromo-indole 2-carboxylic acid ester with vinyl amides yields the styrene derivatives. Depending on the nature of the vinyl amide one or the other or a mixture of the regioisomers are formed (Scheme 6). The vinyl amides can be prepared from the amide and butylvinyl ether in the presence of palladium (II) catalyst like bis-trifluoroaceto-(4,7-diphenyl-1,10-phenanthroline)palladium(II) (in analogy to Org. Lett., Vol 6, No. 11, 2004).

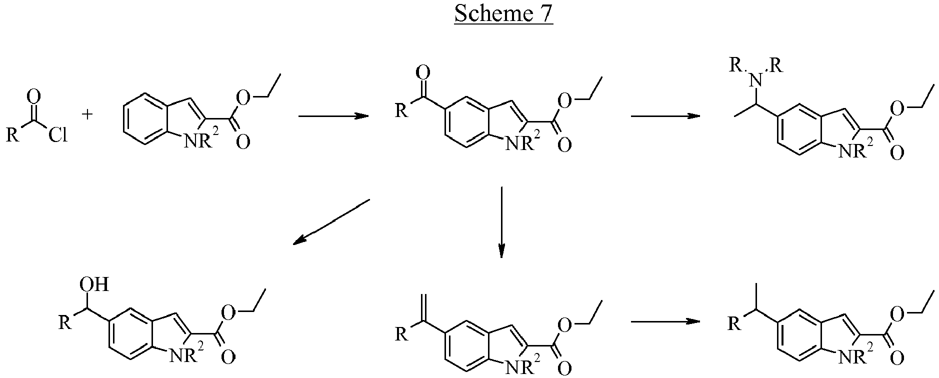

Friedel Craft acylation of indol 2-carboxylic esters with an acid chloride in the presence of aluminiumtrichloride in an inert solvent like dichloroethane yields the 5-acyl-indole 2-carboxylic acid ester derivatives. These can be transformed into the amines by reductive amination, into the styrol derivatives via Wittig reaction or to the alcoholes via reduction of the ketone. Hydrogenation of the styrene derivative leads to the 5-alkyl-indol 2-carboxylic acid ester (Scheme 7).

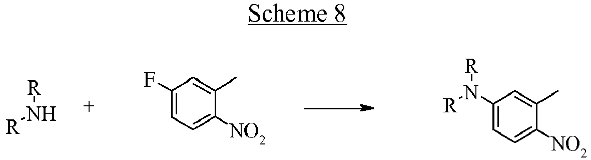

In the 4-position derivatized nitrotoluene derivatives can be easily prepared e.g. by the nucleophilic substitution of 4-fluoro nitrotoluene with an amine in the presence of a base like potassium carbonate in an inert solvent like DMSO at elevated temperatures (scheme 8).

Insofar as their preparation is not described in the examples, the compounds of formula (I) as well as all intermediate products can be prepared according to analogous methods or according to the methods set forth above. Starting materials are commercially available, known in the art or can be prepared by methods known in the art or in analogy thereto.

The present invention also relates to compounds of formula (I) as defined above, when prepared by a process as described above.

Another embodiment provides pharmaceutical compositions or medicaments comprising the compounds of the invention and a therapeutically inert carrier, diluent or pharmaceutically acceptable excipient, as well as methods of using the compounds of the invention to prepare such compositions and medicaments.

Compositions are formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners.

The compounds of the invention may be administered by any suitable means, including oral, topical (including buccal and sublingual), rectal, vaginal, transdermal, parenteral, subcutaneous, intraperitoneal, intrapulmonary, intradermal, intrathecal and epidural and intranasal, and, if desired for local treatment, intralesional administration. Parenteral infusions include intramuscular, intravenous, intraarterial, intraperitoneal, or subcutaneous administration.

The compounds of the present invention may be administered in any convenient administrative form, e.g., tablets, powders, capsules, solutions, dispersions, suspensions, syrups, sprays, suppositories, gels, emulsions, patches, etc. Such compositions may comprise components conventional in pharmaceutical preparations, e.g., diluents, carriers, pH modifiers, preservatives, solubilizers, stabilizers, wetting agents, emulsifiers, sweeteners, colorants, flavorants, salts for varying the osmotic pressure, buffers, masking agents, antioxidants, and further active agents. They can also comprise still other therapeutically valuable substances.

A typical formulation is prepared by mixing a compound of the present invention and a carrier or excipient. Suitable carriers and excipients are well known to those skilled in the art and are described in detail in, e.g., Ansel H.C. et al., Ansel's Pharmaceutical Dosage Forms and Drug Delivery Systems (2004) Lippincott, Williams & Wilkins, Philadelphia*;* Gennaro A.R. et al., Remington: The Science and Practice of Pharmacy (2000) Lippincott, Williams & Wilkins, Philadelphia*; and* Rowe R.C, Handbook of Pharmaceutical Excipients (2005) Pharmaceutical Press, Chicago*.* The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents, diluents and other known additives to provide an elegant presentation of the drug (i.e., a compound of the present invention or pharmaceutical composition thereof) or aid in the manufacturing of the pharmaceutical product (i.e., medicament).

The dosage at which compounds of the invention can be administered can vary within wide limits and will, of course, be fitted to the individual requirements in each particular case. In general, in the case of oral administration a daily dosage of about 0.01 to 1000 mg per person of a compound of general formula (I) should be appropriate, although the above upper limit can also be exceeded when necessary.

An example of a suitable oral dosage form is a tablet comprising about 100 mg to 500 mg of the compound of the invention compounded with about 30 to 90 mg anhydrous lactose, about 5 to 40 mg sodium croscarmellose, about 5 to 30 mg polyvinylpyrrolidone (PVP) K30, and about 1 to 10 mg magnesium stearate. The powdered ingredients are first mixed together and then mixed with a solution of the PVP. The resulting composition can be dried, granulated, mixed with the magnesium stearate and compressed to tablet form using conventional equipment.

An example of an aerosol formulation can be prepared by dissolving the compound, for example 10 to 100 mg, of the invention in a suitable buffer solution, e.g. a phosphate buffer, adding a tonicifier, e.g. a salt such as sodium chloride, if desired. The solution may be filtered, e.g., using a 0.2 µm filter, to remove impurities and contaminants.

As described above, the compounds of formula (I) and their pharmaceutically acceptable salts and esters possess valuable pharmacological properties and have been found to be modulators for amyloid beta. The compounds of formula (I) and their pharmaceutically acceptable salts and esters are useful in the control or prevention of diseases based on the inhibition of Aβ42 secretion. The compounds of formula (I) and their pharmaceutically acceptable salts and esters are useful for the treatment or prevention of diseases associated with the secretion and deposition of β-amyloid in the brain. The compounds of the present invention can therefore be used, either alone or in combination with other drugs, for the treatment or prevention of diseases associated with the deposition of β-amyloid in the brain. These diseases include, but are not limited to Alzheimer's disease, cerebral amyloid angiopathy, hereditary cerebral hemorrhage with amyloidosis Dutch-type (HCHWA-D), multi-infarct dementia, dementia pugilistica or Down syndrome.

A particular embodiment of the present invention relates to pharmaceutical compositions comprising compounds of formula (I) or their pharmaceutically acceptable salts and esters as defined above and one or more pharmaceutically acceptable excipients.

A particular embodiment of the present invention relates to compounds of formula (I) or their pharmaceutically acceptable salts and esters as defined above for use as therapeutically active substances, especially for use as therapeutically active substances for the treatment or prevention of diseases which are related to amyloid beta modulation, particularly for the treatment or prevention of Alzheimer's disease, cerebral amyloid angiopathy, hereditary cerebral hemorrhage with amyloidosis Dutch-type (HCHWA-D), multi-infarct dementia, dementia pugilistica or Down syndrome.

A particular embodiment of the present invention relates to a method for the treatment or prevention of diseases which are related to amyloid beta modulation, particularly for the treatment or prevention of Alzheimer's disease, cerebral amyloid angiopathy, hereditary cerebral hemorrhage with amyloidosis Dutch-type (HCHWA-D), multi-infarct dementia, dementia pugilistica or Down syndrome, which method comprises administering compounds of formula (I) or their pharmaceutically acceptable salts and esters as defined above to a human being or animal.

A particular embodiment of the present invention relates to the use of compounds of formula (I) or their pharmaceutically acceptable salts and esters as defined above for the treatment or prevention of diseases which are related to amyloid beta modulation, particularly for the treatment or prevention of Alzheimer's disease, cerebral amyloid angiopathy, hereditary cerebral hemorrhage with amyloidosis Dutch-type (HCHWA-D), multi-infarct dementia, dementia pugilistica or Down syndrome.

A particular embodiment of the present invention relates to the use of compounds of formula (I) or their pharmaceutically acceptable salts and esters as defined above for the preparation of medicaments for the treatment or prevention of diseases which are related to amyloid beta modulation, particularly for the treatment or prevention of Alzheimer's disease, cerebral amyloid angiopathy, hereditary cerebral hemorrhage with amyloidosis Dutch-type (HCHWA-D), multi-infarct dementia, dementia pugilistica or Down syndrome. Such medicaments comprise compounds of formula (I) or their pharmaceutically acceptable salts and esters as defined above.

The invention will be more fully understood by reference to the following examples. They should however not be construed as limiting the scope of the invention.

### Example 1

### 5-(1-Benzylpyrrolidin-3-yl)-N-(3,5-difluorophenyl)-1H-indole-2-carboxamide

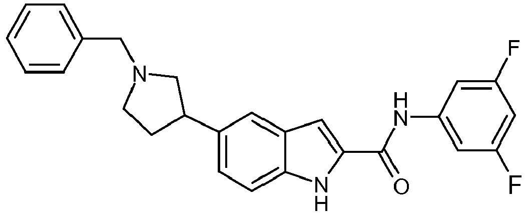

### a) 1-Benzyl-3-(1H-indol-5-yl)-pyrrolidine-2,5-dione

To a solution ofN-benzylmaleimide (1.16 g, 6.21 mmol) in dioxane (5 mL) was added under an atmosphere of nitrogen triethylamine (866 µL, 6.21 mmol) and hydroxy(1,5-cyclo-octadiene)rhodium(I)dimer (85 mg, 0.19 mmol). A solution of 5-indolylboronic acid (1.00 g, 6.21 mmol) in dioxane (12 mL) and water (1.4 mL) was added drop wise during 40 minutes at 45-50°C. The solution was stirred for 30 minutes at 50°C. After cooling to room temperature the solution was diluted with ethyl acetate (20 mL) and washed with a 1 M aqueous solution of Na₂CO₃ (20 mL), water (20 mL) and brine (20 mL). The aqueous layers were extracted with ethyl acetate (20 mL). The combined organic layers were dried over sodium sulfate, filtered and the solvent was removed under reduced pressure. The residue was purified by column chromatography on silica gel using a mixture of heptane / ethyl acetate (66:34 to 30:70) as eluent to yield the title compound as an off-white solid (1.77 g, 94%).
MS ISP (m/e): 305.1 [(M+H)⁺].

### b) 5-(1-Benzyl-pyrrolidin-3-yl)-1H-indole

To a solution of 1-benzyl-3-(1H-indol-5-yl)-pyrrolidine-2,5-dione (1.75 g, 5.75 mmol) in tetrahydrofuran (17 mL) was added drop wise during 5 minutes a 2 M solution of lithium aluminium hydride (11.5 mL, 23.0 mmol). After the addition of some lithium aluminium hydride a gum precipitated. After the addition of the whole amount of lithium aluminium hydride a solution occurred. The reaction was stirred for 1 hour at reflux. After cooling water (873 µL) was added carefully followed by the addition of a 15% aqueous solution of sodium hydroxide (873 µL) and water (2.6 mL). The reaction was stirred for 18 hours at room temperature, filtered over Hyflo and washed with tetrahydrofuran to yield the title compound as a light brown solid (1.563 g, 98 %).
MS ISP (m/e): 277.2 (100) [(M+H)⁺].

### c) 5-(1-Benzylpyrrolidin-3-yl)-N-(3,5-difluorophenyl)-1H-indole-2-carboxamide

To a solution of 5-(1-benzyl-pyrrolidin-3-yl)-1H-indole (1.54 g, 5.59 mmol) in tetrahydrofuran (15 mL) was added under an atmosphere of argon at -70°C a 1.6 M solution of n-butyllithium in hexane (3.6 mL, 5.8 mmol) during 5 minutes. The solution was stirred for 30 minutes at -70°C. The reaction was evacuated and the flask was flashed with carbon dioxide. This procedure was repeated five times. The dry ice bath was removed and the solution was stirred for 1 hour. The solvent was distilled off and the residue was dissolved in THF (15 mL). After cooling to -70°C a 1 .7 M solution of *tert*-butyllithium in pentane (3.4 mL, 5.8 mmol) was added drop wise during 5 minutes. After stirring for 1 hour at -70°C a solution of 3,5-difluorophenyl isocyanate (728 µL, 6.15 mmol) in tetrahydrofuran (1.5 mL) was added drop wise during 5 minutes. The solution was stirred for 4.5 hours at -70°C under an atmosphere of argon. After the addition of water (2 mL) the dry ice bath was replaced by a water bath. The reaction was diluted with ethyl acetate (10 mL) and washed with a 1 M aqueous solution of sodium hydroxide (15 mL) and brine (15 mL). The aqueous layers were extracted with ethyl acetate (15 mL). The combined organic layers were dried over sodium sulfate, filtered and the solvent was evaporated under reduced pressure. The residue was purified by column chromatography on silica gel using a mixture of heptane / ethyl acetate (66:34 to 30:70) as eluent to yield the title compound as an off-white solid (1.54 g, 71%).
MS ISP (m/e): 432.3 (100) [(M+H)⁺].

The following examples (Table 1) were prepared in analogy to example 1 step c, replacing 5-(1-benzyl-pyrrolidin-3-yl)-1H-indole and 3,5-difluorophenyl isocyanate with the corresponding indol or isocyanate.

**Table 1**

| **Ex**. | **Indol / Isocyanate** | **Name and Structure** | **MS (m/e)** |
|---|---|---|---|
| 2 | 5-(1-methyl-pyrrolidin-3-yl)-1H-indole (1188399-12-3; WO 2009115427) / 3,5-difluorophenyl isocyanate | **5-(1-Methyl-pyrrolidin-3-yl)-1H-indole-2-carboxylic acid (3,5-difluoro-phenyl)-amide** | ISP 356.1 [(M+H)⁺] |
| | | 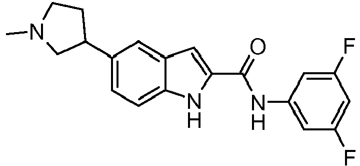 | |
| 3 | 5-(1-benzylpyrrolidin-3-yl)-1H-indole/ 1-fluoro-3-isocyanatobenzene | **5-(1-Benzylpyrrolidin-3-yl)-N-(3-fluorophenyl)-1H-indole-2-carboxamide** | ISP 414.4 [(M+H)⁺] |
| | | 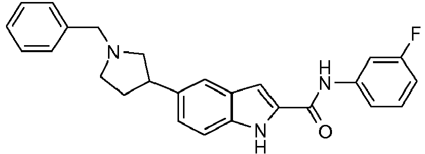 | |

### Example 4

### N-(3-Fluorophenyl)-5-(1-methylpyrrolidin-3-yl)-1H-indole-2-carboxamide

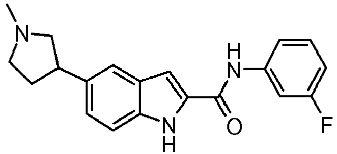

### a) 5-(1-Methyl-pyrrolidin-3-yl)-1H-indole-2-carboxylic acid

To a solution of 5-(1-methylpyrrolidin-3-yl)-1H-indole (3.64 g, 18.2 mmol) in tetrahydrofuran (36 mL) was added under an atmosphere of argon at -70°C a 1.6 M solution of n-butyllithium in hexane (11.6 mL, 18.5 mmol) during 25 minutes. The solution was stirred for 30 minutes at - 70°C. The reaction was evacuated and the flask was backfilled with carbon dioxide. This procedure was repeated five times. The dry ice bath was removed and the solution was allowed to rise to 0°C. The solution was stirred for 15minutes in an ice bath. The flask was carefully evacuated and backfilled with argon for five times to remove all carbon dioxide. After cooling down to -70°C a 1.7 M solution of *tert*-butyllithium (11.0 mL, 18.7 mmol) in pentane was added drop wise during 25 minutes. After stirring for 1 hour at -70°C the flask was evacuated and backfilled with carbon dioxide for five times. The temperature was raised to -62°C. The solution was stirred for 2 hours at -70°C under an atmosphere of carbon dioxide.

After the carefully addition of water (10 mL) the dry ice bath raised to 0°C during 18 hours. The reaction was set to pH 5.0 by the drop wise addition of a 1 M aqueous solution of hydrochloric acid (50 mL). After the addition of water (100 mL) the tetrahydrofuran was distilled off. After the addition of *tert*-butylmethylether (10 mL) the solution was stirred for 15 minutes. The reaction was diluted with ethyl acetate (250 mL) and washed with water (200 mL) and brine (150 mL). The aqueous layers were extracted with ethyl acetate (250 mL). The aqueous layers were again washed with ethyl acetate (250 mL) and the water was distilled off to yield the title compound as a white solid (4.06 g, 92 %).
MS ISP (m/e): 245.2 [(M+H)⁺].

### b) N-(3-Fluorophenyl)-5-(1-methylpyrrolidin-3-yl)-1H-indole-2-carboxamide

To a solution of 5-(1-methylpyrrolidin-3-yl)-1H-indole-2-carboxylic acid (200 mg, 819 µmol) in N-methyl-2-pyrrolidinone (2 mL) was added at 5°C 3-fluoroaniline (91 mg) and N,N-diisopropylethylamine (317 mg, 429 µL). A solution of 1-propanephosphonic acid cyclic anhydride 50% in ethyl acetate (1.3 g, 1.21 mL) was added drop wise during 15 minutes under an atmosphere of nitrogen. The ice bath was replaced with an oil bath and the reaction was stirred for 1 hour at room temperature and for 20 hours at 110°C and for 48 hours at room temperature. The reaction was diluted with ethyl acetate (50 mL) and washed with a 1 M aqueous solution of Na₂CO₃ (50 mL), water (50 mL) and brine (30 mL). The aqueous layers were extracted with ethyl acetate (50 mL). The combined organic layers were dried over sodium sulfate, filtered and concentrated. The residue was purified by column chromatography on silica gel using a mixture of heptane / (ethyl acetate/5% triethylamine) / methanol (100:0:0 to 0:90:10) as eluent to yield the title compound as a brown foam (52 mg, 19 %).
MS ISP (m/e): 338.2 (100) [(M+H)⁺].

The following examples (Table 2) were prepared in analogy to example 4 step b), replacing 5-(1-methylpyrrolidin-3-yl)-1H-indole-2-carboxylic acid and 3-fluoroaniline with the corresponding carboxylic acid and aniline.

**Table 2**

| **Ex**. | **Carboxylic acid** / **Aniline** | **Name and Structure** | **MS (m/e)** |
|---|---|---|---|
| 5 | 5-(1-benzylpyrrolidin-3-yl)-1H-indole-2-carboxylic acid / 5-amino-3-fluorobenzonitrile | **5-(1-Benzylpyrrolidin-3-yl)-N-(3-cyano-5-fluorophenyl)-1H-indole-2-carboxamide** | ISP 439.2 [(M+H)⁺] |
| | | 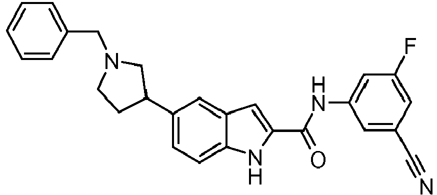 | |
| 6 | 5-(1-methylpyrrolidin-3-yl)-1H-indole-2-carboxylic acid / 3-amino-5-fluorobenzonitrile | **N-(3-Cyano-5-fluorophenyl)-5-(1-methylpyrrolidin-3-yl)-1H-indole-2-carboxamide** | ISP 363.2 [(M+H)+] |
| | | 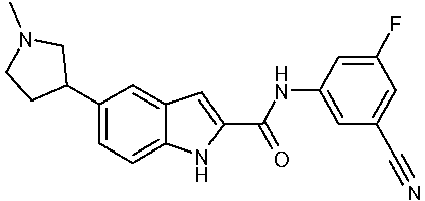 | |

### Example 7

### 5-Pyrrolidin-3-yl-1H-indole-2-carboxylic acid (3,5-difluoro-phenyl)-amide

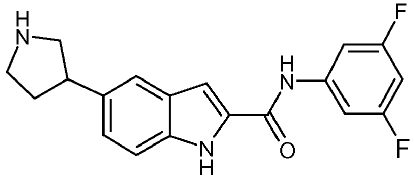

A suspension of 5-(1-benzylpyrrolidin-3-yl)-N-(3,5-difluorophenyl)-1H-indole-2-carboxamide (270 mg, 0.626 mmol) in methanol (4 mL) and tetrahydrofuran (2 mL) was refluxed until a clear solution occurred. After cooling to room temperature palladium on charcoal 10% (27 mg) was added under an atmosphere of argon. The reaction was stirred for 18 hours under an atmosphere of hydrogen. The solvent was distilled off. The residue was suspended in acetic acid (1 mL) and the reaction was stirred for 3 hours under an atmosphere of hydrogen. The reaction was filtered over Hyflo and washed with ethyl acetate (20 mL). The concentrated filtrate was diluted in ethyl actetate (15 mL) and washed with aqueous solution of Na₂CO₃ (15 mL) and brine (15 mL). The aqueous layers were extracted with ethyl acetate (15 mL). The combined organic layers were dried over sodium sulfate, filtered and the solvent was evaporated under reduced pressure. The residue was purified by column chromatography on silica gel using a mixture of ethyl acetate / (ethyl acetate/5% triethylamine) / methanol (100:0:0 to 0:80:20) as eluent to yield the title compound as an off-white solid (0.183 g, 86 %).
MS ISP (m/e): 342.1 (100) [(M+H)⁺].

### Example 8

### N-(3-Fluorophenyl)-5-(pyrrolidin-3-yl)-1H-indole-2-carboxamide

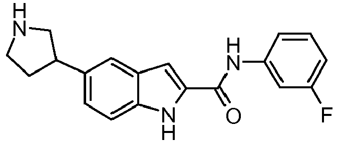

To a suspension of 5-(1-benzylpyrrolidin-3-yl)-N-(3-fluorophenyl)-1H-indole-2-carboxamide (250 mg, 605 µmol) in methanol (5 mL) was added under an atmosphere of argon ammonium formate (191 mg, 3.02 mmol) and palladium on charcoal (25.5 mg, 23.9 µmol). The reaction was stirred at 90°C for 1 hour under an atmosphere of argon. The reaction was filtered over Hyflo and the filter was washed with methanol. The concentrated filtrat was purified by column chromatography on silica gel using a mixture of heptane / (ethyl acetate/5% triethylamine) / methanol (50:50:0 to 0:85:15) as eluent to yield the title compound as a colorless oil (0.196 g, 28 %).
MS ISP (m/e): 324.2 (100) [(M+H)⁺].

### Example 9

### N-(3-Cyano-5-fluorophenyl)-5-(pyrrolidin-3-yl)-1H-indole-2-carboxamide

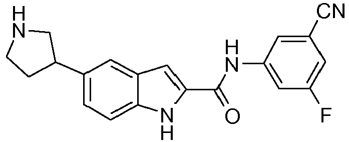

To a suspension of 5-(1-benzylpyrrolidin-3-yl)-N-(3-cyano-5-fluorophenyl)-1H-indole-2-carboxamide (107 mg, 244 µmol) and N,N-diisopropylethylamine (158 mg, 213 µL) in dichloromethane (2 mL) and tetrahydrofuran (2 mL) was added at 0°C during 2 minutes 1-chloroethyl chloroformate (140 mg, 106 µL). After stirring for 1 hour at 0°C the ice bath was removed and the solution was stirred for 18 hours at room temperature. The solution was concentrated in vacuo, the residue dissolved in methanol (5 mL) and stirred for 1 hour at 70°C. The concentrated reaction was diluted with ethyl acetate (15 mL) and washed with a 1 M aqueous solution of Na₂CO₃ (15 mL), water (10 mL) and brine (15 mL). The aqueous layers were extracted with ethyl acetate (15 mL). The combined organic layers were dried over sodium sulfate, filtered and concentrated. The residue was purified by column chromatography basic silica gel using heptane : ethyl acetate :(ethyl acetate / 5 % triethyl amien) : methanol (50:50:0:0 to 0:0:80:20) as eluent and the isolated product was purified by preparative HPLC yielding a white solid (8 mg, 9%).
MS ISP (m/e): 349.3 (100) [(M+H)⁺].

### Example 10

### 5-[1-(2,2,2-Trifluoro-ethyl)-pyrrolidin-3-yl]-1H-indole-2-carboxylic acid (3,5-difluoro-phenyl)-amide

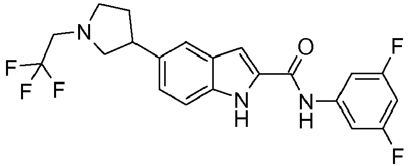

To a turbid solution of N-(3,5-difluorophenyl)-5-(pyrrolidin-3-yl)-1H-indole-2-carboxamide (208 mg, 0.512 mmol) in tetrahydrofuran (2.5 ml) was added N,N-diisopropyl ethyl amine (417 µL, 2.44 mmol) and 2,2,2-trifluoroethyl trifluoromethanesulfonate (170 mg, 0.731 mmol). The reaction was stirred for 3 days at room temperature. The reaction was diluted with ethyl acetate (15 mL) and washed with a 1 M aqueous solution of sodium carbonate (10 mL), water (10 mL) and brine (10 mL). The aqueou layers were extracted with ethyl acetate (15 mL). The combined organic layers were dried over sodium sulfate, filtered and the solvent was evaporated under reduced pressure. The residue was purified by column chromatography on silica gel using a mixture of dichloromethane / methanol (100:0 to 90:10) as eluent to yield the title compound as a light brown solid (0.172 g, 79 %).
MS ISP (m/e): 424.1 (100) [(M+H)⁺].

The following examples (Table 3) were prepared in analogy to example 10, replacing 2,2,2-trifluoroethyl trifluoromethanesulfonate with the corresponding alkylating or acylating reagent.

**Table 3**

| **Ex**. | **Starting material-Alkylating/ Acylating reagent** | **Name and Structure** | **MS (m/e)** |
|---|---|---|---|
| 11 | Example 7 / 2-bromopropane | **5-(1-Isopropyl-pyrrolidin-3-yl)-1H-indole-2-carboxylic acid (3,5-difluoro-phenyl)-amide** | ISP 384.1 [(M+H)⁺] |
| | | 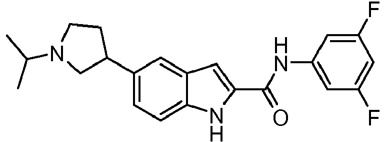 | |
| 12 | Example 7 / isopropylsulfonyl chloride | **5-[1-(Propane-2-sulfonyl)-pyrrolidin-3-yl]-1H-indole-2-carboxylic acid (3,5-difluorophenyl)-amide** | ISN 446.14 [(M-H)⁻] |
| | | 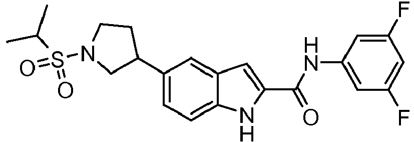 | |
| 13 | Example 7 / isobutyryl chloride | **5-(1-Isobutyryl-pyrrolidin-3-yl)-1H-indole-2-carboxylic acid (3,5-difluoro-phenyl)-amide** | ISP 412.2 [(M+H)⁺] |
| | | 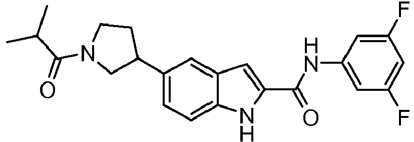 | |
| 14 | Example 8 / 2-bromopropane in NMP | **N-(3-Fluorophenyl)-5-(1-isopropylpyrrolidin-3-yl)-1H-indole-2-carboxamide** | EI 365.18 [M⁺] |
| | | 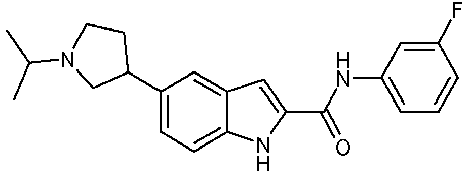 | |
| 15 | Example 8 / 2,2,2-trifluoroethyl trifluoromethanesulfonate | **N-(3-Fluorophenyl)-5-(1-(2,2,2-trifluoroethyl)pyrrolidin-3-yl)-1H-indole-2-carboxamide** | EI 405.1 [M⁺] |
| | | 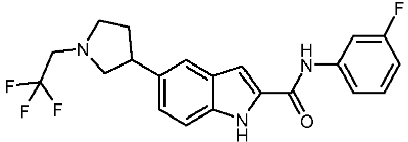 | |
| 16 | Example 8 / 2-bromoacetonitrile | **5-(1-(Cyanomethyl)pyrrolidin-3-yl)-N-(3-fluorophenyl)-1H-indole-2-carboxamide** | ISP 363.1 [(M+H)⁺] |
| | | 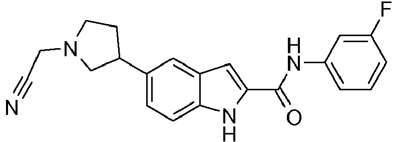 | |

### Example 17

### 5-(1-Benzyl-2,5-dioxopyrrolidin-3-yl)-N-(3-fluorophenyl)-1H-indole-2-carboxamide

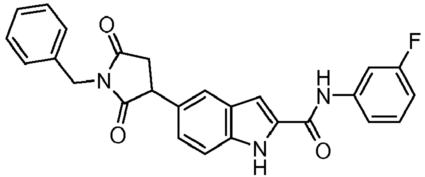

### a) 5-Bromo-1H-indole-2-carboxylic acid (3-fluoro-phenyl)-amid

The title compound was prepared in the same manner as described in example 4 step b) from 5-bromo-1H-indole-2-carboxylic acid and 3-fluoroaniline as a light brown solid (68 %). HPLC-MS (purity; retention time) = 99 %; 1.73 min.
MS ISN (m/e): 332.2 (100) [(M-H)⁻].

### b) 5-(4,4,5,5-Tetramethyl-[1,3,2]dioxaborolan-2-yl)-1H-indole-2-carboxylic acid (3-fluoro-phenyl)-amid

A suspension of 5-bromo-N-(3-fluorophenyl)-1H-indole-2-carboxamide (3.1 g, 9.3 mmol), bis(pinacolato)diboron (3.54 g, 14.0 mmol), potassium acetate (2.74 g, 27.9 mmol) in dioxane (40 mL) was evacuated and backfilled with argon for five times. After the addition of bis(triphenylphosphine)palladium(II) chloride (653 mg, 930 µmol) the suspension was stirred for 1 hour at reflux. The reaction went into solution and was diluted with ethyl acetate (50 mL) and washed with a 1 M aqueous solution of Na₂CO₃ (50 mL), water (50 mL) and brine (50 mL). The aqueous layers were extracted with ethyl acetate (50 mL). The combined organic layers were dried over magnesium sulfate. The concentrated filtered solution was purified by flash chromatography using heptane / ethyl acetate (90:10 to 50:50) as eluent to yield the title compound as an off-white solid (3.49 g, 99 %).
MS ISN (m/e): 379.3 (100) [(M-H)⁻].

### c) 5-(1-Benzyl-2,5-dioxopyrrolidin-3-yl)-N-(3-fluorophenyl)-1H-indole-2-carboxamide

The title compound was prepared in the same manner as described in example 1 step a) from 1-benzyl-1H-pyrrole-2,5-dione and N-(3-fluorophenyl)-5-(4,4,5,5-tetramethyl-1,3,2-dioxaborolan-2-yl)-1H-indole-2-carboxamide as a light brown solid (14 %).
MS ISN (m/e): 440.3 (100) [(M-H)⁻].

### Example 18

### 5-(3-Isopropyl-5-oxo-pyrrolidin-3-yl)-1H-indole-2-carboxylic acid (3,4-difluoro-phenyl)-amide

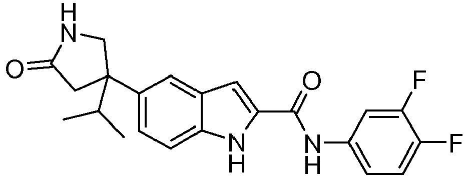

### a) 4-(4-Amino-3-iodo-phenyl)-4-isopropyl-pyrrolidin-2-on

4-(4-Amino-phenyl)-4-isopropyl-pyrrolidin-2-on (509 mg, 2 mmol) was dissolved in ethanol (10 mL) and triethylamine (404 mg, 4 mmol), silver sulfate (623 mg, 2 mmol) and iodine (507 mg, 2 mmol) were added. The suspension was stirred at room temperature overnight, diluted with ethanol, filtered, washed with ethanol and the filtrate was concentrated in vacuum. The residue was purified by column chromatography on silica gel using dichloromethane / methanol (9:1) as eluent to yield the title compound as a light red solid (280 mg, 41 %).

### b) 5-(3-Isopropyl-5-oxo-pyrrolidin-3-yl)-1H-indole-2-carboxylic acid

To a solution of 4-(4-amino-3-iodo-phenyl)-4-isopropyl-pyrrolidin-2-on (160 mg) in DMF (1.4 mL) was added pyruvic acid (99 µL), DABCO (165 mg) and palladium (II) acetate (5.2 mg). The reaction was stirred overnight at 100°C under nitrogen. 1M aqueous sodium hydroxide solution was added and the reaction was extracted three times with ethyl acetate. The aqueous layer was set to pH 1 with 1 M aqueous hydrogen chloride solution and extracted three times with ethyl acetate. The combined organic layers were washed with water, dried over sodium sulfate, filtered and concentrated to yield the title compound as a brown solid (127 mg, 95 %).

### c) 5-(3-Isopropyl-5-oxo-pyrrolidin-3-yl)-1H-indole-2-carboxylic acid (3,4-difluoro-phenl)-amide

A solution of 3,4-difluoraniline (37.4 µL), 5-(3-isopropyl-5-oxo-pyrrolidin-3-yl)-1H-indole-2-carboxylic acid (131 mg) and HBTU (215 mg) in NMP (2.5 mL) was stirred at room temperature overnight and then at 80°C for 3 hours. Water was added and the reaction was extracted three times with ethyl acetate. The combined organic layers were washed with brine and dried over sodium sulfate, filtered and concentrated. The residue was purified by column chromatography on silica gel using dichloromethane / methanol / saturated aqueous ammonia solution (9:1:0.1) to yield the title compound as a brown solid (46 mg, 31 %).
MS ISP (m/e): 398.2 (100) [(M+H)⁺].

### Example 19

### 5-(3-Ethyl-2,6-dioxo-piperidin-3-yl)-1H-indole-2-carboxylic acid (3,5-difluoro-phenyl)-amide

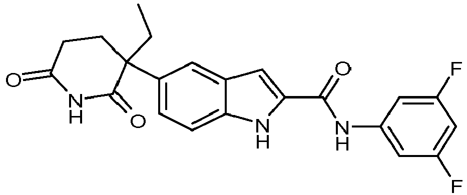

### a) 3-(4-Amino-3-iodo-phenyl)-3-ethyl-piperidine-2,6-dione

To a solution of aminoglutethimide (1.86 g) in methanol (25 mL) and water (25 mL) was added drop wise a solution of potassium iodide (1.55 g) and iodine (2.78 g) in water (45 mL). The reaction was heated to reflux for 45 minutes and concentrated to 1/3 of its volume. Water (100 mL) and diethyl ether (10 mL) were added. A 10 % aqueous solution of sodium thiosulfate was added drop wise until the yellow color disappears and the solution was adjusted to pH 7 with aqueous sodium hydrogen carbonate solution. The reaction was extracted three times with diethylether. The combined organic layers were washed with brine and dried over sodium sulfate, filtered and concentrated. The residue was purified by column chromatography on silica gel using dichloromethane / methanol (19.5:0.5) as eluent to yield the title compound as white crystals (1.09 g, 38 %).

### b) 5-(3-Ethyl-2,6-dioxo-piperidin-3-yl)-1H-indole-2-carboxylic acid

The title compound was prepared in the same manner as described in example 18 step b) from 3-(4-amino-3-iodo-phenyl)-3-ethyl-piperidine-2,6-dione as a brown foam (76 %).
MS ISP (m/e): 380.3 (100) [(M+H)⁺].

### c) 5-(3-Ethyl-2,6-dioxo-piperidin-3-yl)-1H-indole-2-carboxylic acid (3,5-difluoro-phenyl)-amide

Oxalyl chloride (150 µL) was added at room temperature to a brown solution of 5-(3-ethyl-2,6-dioxopiperidin-3-yl)-1H-indole-2-carboxylic acid (130 mg) in tetrahydrofuran (7 mL) and DMF (20 µL). Gas evolution occurred. The reaction was stirred at room temperature for 2 hours. The solvent was removed in vacuo. The residue was evaporated with toluene. The residue was dissolved in tetrahydrofuran (6 mL). 3,5-Difluoroaniline (101 mg) and pyridine (185 µL) in tetrahydrofuran (1.4 mL) was added drop wise at room temperature. The reaction was stirred for 2 hours at room temperature, diluted with ethyl acetate and washed three times with water and brine. The organic layer was dried over sodium sulfate, filtered and concentrated. The residue was purified by column chromatography on silica gel using dichloromethane / methanol (96.5:3.5) as eluent to yield the title compound as light brown crystals (71 mg, 23 %).
MS ISN (m/e): 410.4 (100) [(M+H)⁺].

### Example 20

### 5-(1-Isopropyl-piperidin-4-yl)-1H-indole-2-carboxylic acid (3-fluoro-phenyl)-amide

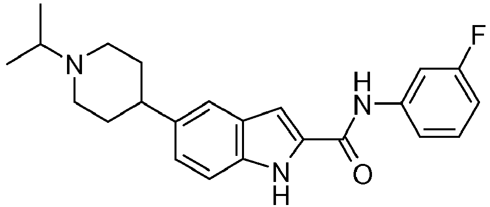

### a) 5-Bromo-1H-indole-2-carboxylic acid ethyl ester

5-Bromoindole-2-carboxylic acid (2.5 g; 10.4 mmol) was dissolved in ethanol (100 mL). Concentrated sulphuric acid (2.5 mL) was added and the reaction mixture heated at 80°C for 12 hours. The mixture was cooled to room temperature and the solution neutralized to pH 7 using aqueous sodium bicarbonate solution (50 mL). The reaction mixture was concentrated *in vacuo* to remove ethanol, and the aqueous phase was extracted using ethyl acetate (2 x 100 mL). The combined organic phases were dried over magnesium sulfate, filtered and concentrated *in vacuo* to afford the title compound (2.63 g, 94 %).
HPLC-MS (purity; retention time) = 100 %; 2.31 min.
MS ISP (m/e): 270.0/268.1 (100/100) [(M+H)⁺].

### b) 5-Pyridin-4-yl-1H-indole-2-carboxylic acid ethyl ester

Potassium acetate (2.57 g; 26.2 mmol) was dried under high vacuum at 50°C in the reaction flask over 2 hours. 5-Bromo-1H-indole-2-carboxylic acid ethyl ester (2.34 g; 8.75 mmol) was dissolved in degassed dioxane (100 mL) and added to the reaction flask, followed by bis(pinacolato)diboron and bis(triphenylphosphine)palladium (II) chloride, (0.30 g; 0.44 mmol). The reaction mixture was heated to reflux under a nitrogen atmosphere and stirred for 17 hours. The reaction mixture was cooled to 50°C. 4-Iodopyridine (3.58 g; 1.75 mmol) was added to the reaction portion wise, followed by bis(triphenylphosphine)palladium (II) chloride (0.30 g; 0.44 mmol) and 2 M aqueous sodium carbonate solution (23 mL). The reaction was returned to reflux and stirred for 24 hours. Following complete consumption of the intermediate, the reaction was cooled to room temperature and the solvent was removed *in vacuo.* The crude reaction mixture was re-dissolved in ethyl acetate (100 mL) and washed with water (50 mL) and brine (50 mL). The organic phase was dried over magnesium sulfate, filtered and concentrated *in vacuo.* Trituration with ethyl acetate / heptane afforded the title compound (1.35 g, 59 %).
¹H NMR (400MHz, DMSO): δ (ppm) = 1.35 (3H, t, *J =* 7.09 Hz), 4.36 (2H, q, *J =* 7.09 MHz), 7.24 (1H, s), 7.58 (1H, d, *J=* 8.56 Hz), 7.70-7.73 (3H, m), 8.14 (1H, s), 8.60 (2H, d, *J=* 5.87 Hz), 12.08 (1H, s).
HPLC-MS (purity); retention time = 89%; 1.28min.
MS ISP (m/e): 267.2 (100) [(M+H)⁺].

### c) 4-(2-Ethoxycarbonyl-1H-indol-5-yl)-1-isopropyl-pyridinium bromide

5-Pyridin-4-yl-1H-indole-2-carboxylic acid ethyl ester (2.0 g; 7.51mmol) was dissolved in dimethylformamide (8 mL) and 2-bromopropane (3.5 mL; 37.5 mmol) was added portion-wise. The reaction mixture was stirred under nitrogen at 115°C for 18 hours. The solvent was removed *in vacuo* to afford the title compound as a brown oil (2.3 g, 98 %). The compound was used directly in the next step without further purification.
¹H NMR (400 MHz, DMSO): δ (ppm) =1.36 (3H, t, *J =* 7.34 Hz), 1.62 (6H, d, *J =* 6.85 Hz), 4.38 (2H, q, *J*=7.09 Hz), 4.96-5.03 (1H, m), 7.32 (1H, s), 7.66 (1H, d, *J=* 8.80 Hz), 8.00 (1H, d, *J =* 6.85 Hz), 8.50-8.54 (3H, m), 9.13 (2H, d, *J =* 6.85 Hz), 12.35 (1H, s).
HPLC-MS (purity; retention time) = 94 %; 1.34 min.
MS EI (m/e): 309.2 (100) [M⁺].

### d) 5-(1-Isopropyl-piperidin-4-yl)-1H-indole-2-carboxylic acid ethyl ester

4-(2-Ethoxycarbonyl-1H-indol-5-yl)-1-isopropyl-pyridinium bromide (2.3 g; 7.43 mmol) was dissolved in methanol (60 mL) and platinum oxide (0.252 g; 1.11 mmol) was added. The reaction flask was flushed with hydrogen and the mixture stirred under a hydrogen atmosphere for 18 hours. Re-treatment of the reaction was required at this stage and as such the mixture was filtered, a further quantity of platinum oxide (0.252 g; 1.11 mmol; 0.15 eq) was added and the reaction was stirred overnight under hydrogen. A further two re-treatments of the reaction solution was required before it was deemed complete. Following final filtration the solvent was removed *in vacuo* to afford the title compound (1.05 g, 44 %).
¹H NMR (400 MHz, DMSO): δ (ppm) =1.14 (6H, d, *J =* 6.68 Hz), 1.19 (3H, t, *J =* 7.14 Hz), 1.76-1.92 (4H, m), 2.74-3.01 (3H, m), 3.26-3.39 (3H, m), 4.18 (2H, q, *J =* 7.04 Hz), 6.98-7.03 (2H, m), 7.27-7.33 (2H, m), 11.69 (1H, s).
HPLC-MS (purity; retention time) = 95 %; 1.34 min
MS ISP (m/e): 315.3 (100) [(M+H)⁺].

### e) 5-(1-Isopropyl-piperidin-4-yl)-1H-indole-2-carboxylic acid (3-fluoro-phenyl)-amide

3-Fluoro-aniline (0.11 g; 1.0 mmol) was dissolved in molecular sieve dried dioxane (1.5 mL) and trimethyl aluminium (0.18 mL; 0.35 mmol) was added portion wise as a tetrahydrofuran solution (2 M). 5-(1-Isopropyl-piperidin-4-yl)-1H-indole-2-carboxylic acid ethyl ester (0.11 g; 0.35 mmol) was dissolved in molecular sieve dried dioxane (1.5 mL) and added to the reaction portion wise. The mixture was heated to 70°C for 18 hours after which time the reaction was cooled to room temperature and quenched with Rochelle's salt solution (1.5 mL; saturated). After stirring for 2 hours the aqueous layer was extracted with chloroform / isopropylalcohol (2 x 3 mL) and the combined organic phases were dried over magnesium sulfate, filtered and concentrated *in vacuo.* Trituration with dichloromethane / heptane afforded the title compound (19 mg, 28 %).
HPLC-MS (purity; retention time) = 99 %; 1.60 min.
MS ISP (m/e): 380.2 (100) [(M+H)⁺].

### Example 21

### 5-(1-Isopropyl-piperidin-3-yl)-1H-indole-2-carboxylic acid (3,5-difluoro-phenyl)-amide

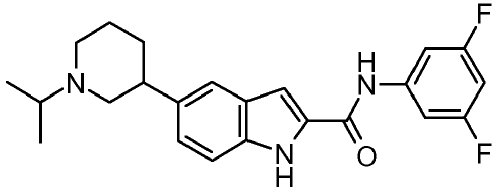

### a) 5-Pyridin-3-yl-1H-indole-2-carboxylic acid ethyl ester

5-Bromo-1H-indole-2-carboxylic acid ethyl ester (5.0 g), 3-tributylstannylpyridine (7.552 g), and bis-(triphenylphosphin)-palladium(II)-dichlorid (0.131 mg) were suspended in dioxane (10 mL) and refluxed at 120°C overnight. Still starting material left, another bis-(triphenylphosphin)-palladium(II)-dichlorid (0.131 mg) was added, reaction was refluxed over the weekend. The reaction mixture was evaporated, the residue was purified by column chromatography on basic silica gel using heptane / ethyl acetate (7:3) and crystallized in diethylether to yield the title compound as a white solid (1.454 g, 29 %).
MS ISP (m/e): 267.1 (100) [(M+H)⁺].

### b) 5-(1-Isopropyl-piperidin-3-yl)-1H-indole-2-carboxylic acid ethyl ester

To a solution of 5-pyridin-3-yl-1H-indole-2-carboxylic acid ethyl ester (1.45 g) in dimethylformamid (20 mL) was added 2-bromopropane (1.005 g), reaction was stirred overnight at 100°C and then evaporated. Methanol (30ml) and platin(IV)-oxid hydrate (0.012 g) were added, the reaction was stirred overnight at room temperature in the presence of hydrogene. Reaction was filtered, solvent was evaporated and the residue was purified by column chromatography on basic silica gel using ethyl acetate and crystallized in diethylether to yield the title compound as an off-white solid (0.411 g, 24 %).
MS ISP (m/e): 315.2 (100) [(M+H)⁺].

### c) 5-(1-Isopropyl-piperidin-3-yl)-1H-indole-2-carboxylic acid (3,5-difluoro-phenyl)-amide

To a solution of 3,5-difluoroaniline (287 mg) in dioxan was added under nitrogen a 2M solution of trimethylaluminium in heptane. The reaction was stirred 1 hour at room temperature and 5-(1-isopropyl-piperidin-3-yl)-1H-indole-2-carboxylic acid ethyl ester in dioxan (1 mL) was added and stirred at room temperature overnight. Water was added and the reaction was extracted with ethyl acetate. The combined organic layers were dried over magnesium sulfate, filtered and concentrated. The residue was purified by column chromatography on silica gel (basic) with ethyl acetate / heptane (1:1) as eluent. The product was stirred with diethyl ether, filtered and dried to yield the title compound as a light brown solid (28 mg, 11 %).
MS ISP (m/e): 398.3 (100) [(M+H)⁺].

The following examples (Table 4) were prepared in analogy to example 20 step e), replacing 3-fluoro-aniline with the corresponding aniline and 5-(1-isopropyl-piperidin-4-yl)-1H-indole-2-carboxylic acid ethyl ester with the corresponding ester.

**Table 4**

| **Ex.** | **Aniline / Ester** | **Name and Structure** | **MS (m/e)** |
|---|---|---|---|
| 22 | 3-trifluoromethylaniline / 5-(1-isopropyl-piperidin-4-yl)-1H-indole-2-carboxylic acid ethyl ester | **5-(1-Isopropyl-piperidin-4-yl)-1H-indole-2-carboxylic acid (3-trifluoromethyl-phenyl)-amide** | ISP 430.1 [(M+H)⁺] |
| | | 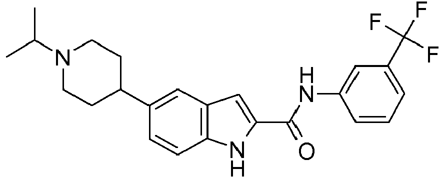 | |
| 23 | 3-chloroaniline / 5-(1-isopropyl-piperidin-4-yl)-1H-indole-2-carboxylic acid ethyl ester | **5-(1-Isopropyl-piperidin-4-yl)-1H-indole-2-carboxylic acid (3-chloro-phenyl)-amide** | ISP 396.1 [(M+H)⁺] |
| | | 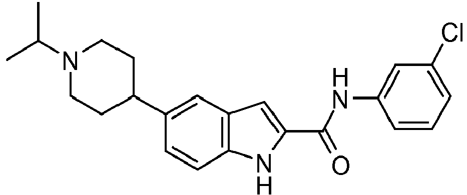 | |
| 24 | 2-fluoroaniline / 5-(1-isopropyl-piperidin-4-yl)-1H-indole-2-carboxylic acid ethyl ester | **5-(1-Isopropyl-piperidin-4-yl)-1H-indole-2-carboxylic acid (2-fluoro-phenyl)-amide** | ISP 380.1 [(M+H)⁺] |
| | | 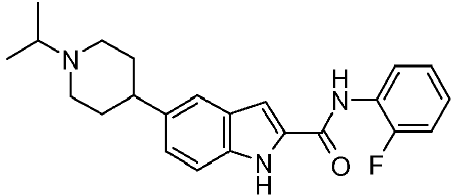 | |
| 25 | 3-methoxyaniline / 5-(1-isopropyl-piperidin-4-yl)-1H-indole-2-carboxylic acid ethyl ester | **5-(1-Isopropyl-piperidin-4-yl)-1H-indole-2-carboxylic acid (3-methoxy-phenyl)-amide** | ISP 392.2 [(M+H)⁺] |
| | | 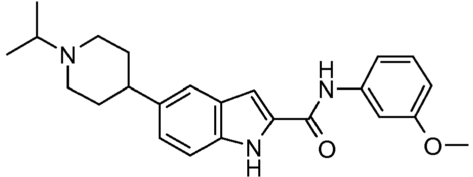 | |
| 26 | 4-methoxaniline / 5-(1-isopropyl-piperidin-4-yl)-1H-indole-2-carboxylic acid ethyl ester | **5-(1-Isopropyl-piperidin-4-yl)-1H-indole-2-carboxylic acid (4-methoxy-phenyl)-amide** | ISP 392.2 [(M+H)⁺] |
| | | 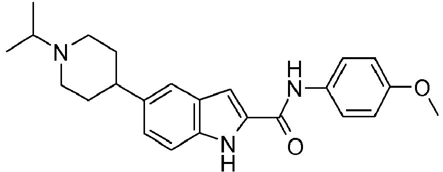 | |
| 27 | 4-*tert*-butyl-aniline / 5-(1- isopropyl-piperidin-4-yl)-1H-indole-2-carboxylic acid ethyl ester | **5-(1-Isopropyl-piperidin-4-yl)-1H-indole-2-carboxylic acid (4-tert-butyl-phenyl)-amide** | ISP 418.2 [(M+H)⁺] |
| | | 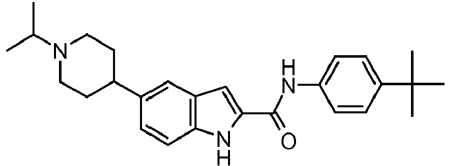 | |
| 28 | 3,4-difluoroaniline / 5-(1-isopropyl-piperidin-4-yl)-1H-indole-2-carboxylic acid ethyl ester | **5-(1-Isopropyl-piperidin-4-yl)-1H-indole-2-carboxylic acid (3,4-difluoro-phenyl)-amide** | ISP 398.2 [(M+H)⁺] |
| | | 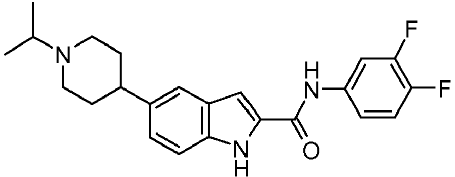 | |
| 29 | 2,4-difluoroaniline / 5-(1-isopropyl-piperidin-4-yl)-1H-indole-2-carboxylic acid ethyl ester | **5-(1-Isopropyl-piperidin-4-yl)-1H-indole-2-carboxylic acid (2,4-difluoro-phenyl)-amide** | ISP 398.2 [(M+H)⁺] |
| | | 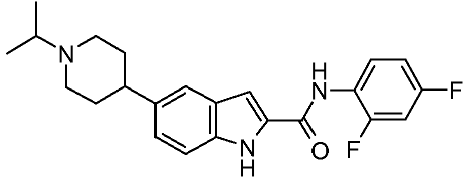 | |
| 30 | 3-chloro-5-fluoroaniline / ethyl 5-(1-isopropylpiperidin-3-yl)-1H-indole-2-carboxylate | **N-(3-Chloro-5-fluorophenyl)-5-(1-isopropylpiperidin-3-yl)-1H-indole-2-carboxamide** | ISP 416.4 [(M+H)⁺] |
| | | 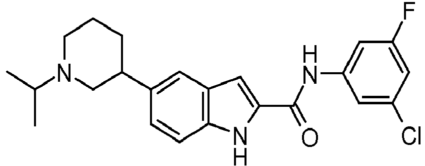 | |
| 31 | 5-amino-3-fluorobenzonitrile / ethyl 5-(1-isopropylpiperidin-3-yl)-1H-indole-2-carboxylate (200°C microwave oven) | **5-(1-Isopropyl-piperidin-3-yl)-1H-indole-2-carboxylic acid (3-cyano-5-fluoro-phenyl)-amide** | ISP 405.4 [(M+H)⁺] |
| | | 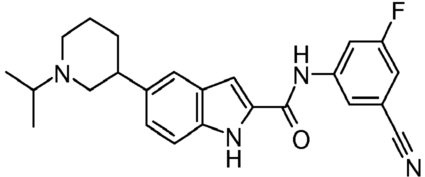 | |
| 32 | 4-amino-2-fluoropyridine / 5-(1-isopropyl-piperidin-4-yl)-1H-indole-2-carboxylic acid ethyl ester | **5-(1-Isopropyl-piperidin-4-yl)-1H-indole-2-carboxylic acid (6-fluoro-pyridin-3-yl)-amide** | ISP 381.2 [(M+H)⁺] |
| | | 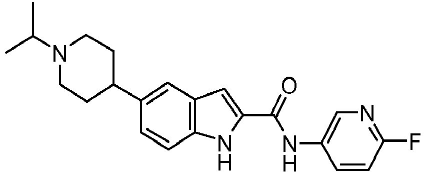 | |
| 33 | m-toluidine / ethyl 5-(1-isopropylpiperidin-3-yl)-1H-indole-2-carboxylate (200°C microwave oven) | **5-(1-Isopropyl-piperidin-3-yl)-1H-indole-2-carboxylic acid m-tolylamide** | ISP 376.4 [(M+H)⁺] |
| | | 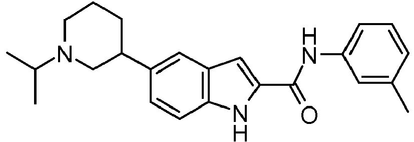 | |
| 34 | 3-fluoro-5-methylaniline / ethyl 5-(1-isopropylpiperidin-3-yl)-1H-indole-2-carboxylate | **N-(3-Fluoro-5-methylphenyl)-5-(1-isopropylpiperidin-3-yl)-1H-indole-2-carboxamide** | ISP 394.2 [(M+H)⁺] |
| | | 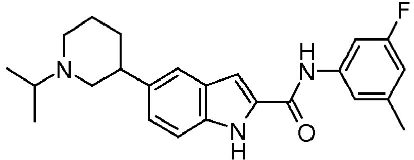 | |

### Example 35

### 5-(1-Benzoyl-piperidin-4-yl)-1H-indole-2-carboxylic acid (4-fluoro-phenyl)-amide

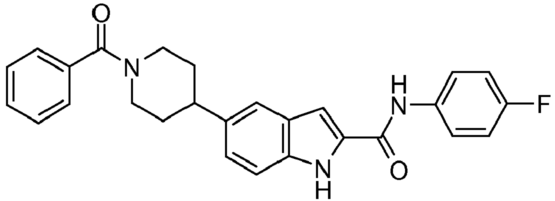

### a) 1-Benzyl-4-(2-ethoxycarbonyl-1H-indol-5-yl)-pyridinium bromide

5-Pyridin-4-yl-1H-indole-2-carboxylic acid ethyl ester (4.58 g; 17.2 mmol) was dissolved in dimethylformamide (8 mL) and benzyl bromide (10.2 mL; 86.0 mmol) was added portion-wise. The reaction mixture was stirred under nitrogen at 115°C for 18 hours. The reaction was cooled to room temperature and ethyl acetate (100 mL) was added. 1-Benzyl-4-(2-ethoxycarbonyl-1H-indol-5-yl)-pyridinium bromide was isolated from the crude reaction mixture by filtration (6.15 g, 82 %). The compound was used directly in the next step without further purification.
HPLC-MS (purity; retention time) = 97 %; 1.50 min.
MS EI (m/e): 357.2 (100) [M⁺].

### b) 5-Piperidin-4-yl-1H-indole-2-carboxylic acid ethyl ester

1-Benzyl-4-(2-ethoxycarbonyl-1H-indol-5-yl)-pyridinium bromide (6.15 g, 14.1 mmol) was dissolved in methanol (200 mL) and platinum oxide (0.48 g; 2.1 mmol) was added. The reaction flask was flushed with hydrogen and the mixture was stirred under a hydrogen atmosphere for 18 hours. Re-treatment of the reaction was required at this stage and as such the mixture was filtered, a further quantity of platinum oxide (0.252 g; 1.11 mmol) was added and the reaction was stirred overnight under hydrogen. Following final filtration the solvent was removed *in vacuo* to afford the title compound (3.86 g, 82 %). The compound was used directly in the next step without further purification.
HPLC-MS (purity; retention time) = 68 %; 1.27 min.
MS ISP (m/e): 273.1 (100) [(M+H)⁺].

### c) 5-(1-tert-Butoxycarbonyl-piperidin-4-yl)-1H-indole-2-carboxylic acid ethyl ester

Piperidin-4-yl-1H-indole-2-carboxylic acid ethyl ester (1 g, 3.67 mmol) was dissolved in dry tetrahydrofuran (10 mL) and boc-anhydride (1.2 g, 5.51 mmol) was added portion-wise followed by 4-dimethylaminopyridine (0.045 g, 0.37 mmol, 0.1 eq). The reaction was stirred at room temperature under a nitrogen atmosphere for 16 hours after which time the solution was acidified to pH6 using 0.5 M aqueous hydrochloric acid solution. The desired product was extracted from the aqueous phase using ethyl acetate (2 x 10 mL). The combined organic layers were dried over magnesium sulfate, filtered and the solvent was removed *in vacuo.* Following trituration with dichloromethane / heptane the title compound was obtained (0.35 g, 26 %).
HPLC-MS (purity; retention time) = 93 %; 2.47 min.
MS EI (m/e): 373.3 (100) [M⁺].

### d) 5-(1-tert-Butoxycarbonyl-piperidin-4-yl)-1H-indole-2-carboxylic acid

5-(1-*tert*-Butoxycarbonyl-piperidin-4-yl)-1H-indole-2-carboxylic acid ethyl ester (0.353 g, 0.95 mmol) was dissolved in tetrahydrofuran (4 mL) and 2M aqueous lithium hydroxide solution (2 mL) was added portion-wise. The reaction was stirred at room temperature for 16 hours, after which time the mixture was acidified with 1M aqueous hydrochloric acid solution to pH 5 and the aqueous layer was extracted with dichloromethane (3 x 10 mL). The combined organic layers were dried over magnesium sulfate, filtered and concentrated *in vacuo* to afford the title compound as an off-white solid (0.316 g, 97 %).
¹H NMR (400MHz, DMSO): δ (ppm) =1.65 (9H, s), 1.68-1.79 (2H, m), 1.99-2.02 (2H, m), 2.90-3.13 (3H, m), 4.29-4.32 (2H, m), 7.24 (1H, s), 7.37 (1H, d, *J=* 10.06 Hz), 7.58 (1H, d, *J=* 8.51 Hz), 7.68 (1H, s), 11.86 (1H,s).
HPLC-MS (purity; retention time) = 96 %; 2.09 min.
MS ISP (m/e): 345.2 (100) [(M+H)⁺].

### e) 4-[2-(4-Fluoro-phenylcarbamoyl)-1H-indol-5-yl]-piperidine-1-carboxylic acid tert-butyl ester

5-(1-*tert*-Butoxycarbonyl-piperidin-4-yl)-1H-indole-2-carboxylic acid (0.316 g, 0.92 mmol) was dissolved in dimethylformamide (3 mL) and NN-diisopropylethylamine (0.61 mL, 3.67 mmol) was added, followed by 2-(1H-7-azabenzotriazol-1-yl)-1,1,3,3-tetramethyl uranium hexafluorophosphate (HATU) (0.418 g, 1.10 mmol). The reaction mixture was stirred at room temperature for 1 hour, after which time 4-fluoroaniline (0.11 mL, 1.10 mmol) was added and the reaction stirred at room temperature overnight. The reaction was washed with water (1 x 5 mL). The combined organic phases was dried over magnesium sulfate and concentrated *in vacuo.* The residue was purified by column chromatograph on silica gel using ethyl acetate / heptane as the eluent to afford the title compound (154 mg, 38 %).
HPLC-MS (purity; retention time) = 100 %; 2.4 0min.
MS ISP (m/e): 460.2 (100) [(M+Na)⁺].

### f) 5-Piperidin-4-yl-1H-indole-2-carboxylic acid (4-fluoro-phenyl)-amide

4-[2-(4-Fluoro-phenylcarbamoyl)-1H-indol-5-yl]-piperidine-1-carboxylic acid *tert*-butyl ester (0.154 g, 0.35 mmol) was dissolved in 4 M aqueous hydrochloric acid solution and dioxane (2 mL) and the reaction was stirred for 2 hours at room temperature, followed by heating at 40°C for a further 1 hour. The solvent was removed and the crude oil re-dissolved in dichloromethane (2 mL). A solution of saturated aqueous sodium hydrogen carbonate was added until the solution was basic and the product was extracted with dichloromethane (3 x 2 mL). The combined organic layers were dried over magnesium sulfate and evaporated under reduced pressure to yield the title compound (108 mg, 92 %).
HPLC-MS (purity; retention time) = 97 %; 1.38 min.
MS ISP (m/e): 338.3 (100) [(M+H)⁺].

### g) 5-(1-Benzoyl-piperidin-4-yl)-1H-indole-2-carboxylic acid (4-fluoro-phenyl)-amide

5-Piperidin-4-yl-1H-indole-2-carboxylic acid (4-fluoro-phenyl)-amide (0.027 mg, 0.08 mmol), benzoyl chloride (10 µL, 0.09 mmol, 1.1 eq) and N,N-diisopropylethylamine (15 µL, 0.09 mmol) were suspended in dichloromethane (1 mL) and stirred at room temperature for 18 hours. The reaction mixture was washed with water (2 x 2 mL) and the aqueous layer was extracted with dichloromethane (2 mL). The organic phases were combined, dried over magnesium sulfate, filtered and concentrated *in vacuo.* The residue was purified by prep-HPLC to afford the title compound (8 mg, 12 %).
HPLC-MS (purity; retention time) = 89 %; 2.29 min.
MS ISP (m/e): 442.1 (100) [(M+H)⁺].

### Example 36

### 5-(1-Methyl-piperidin-4-yl)-1H-indole-2-carboxylic acid (4-fluoro-phenyl)-amide

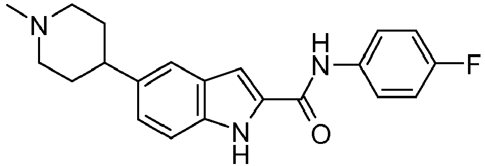

5-Piperidin-4-yl-1H-indole-2-carboxylic acid (4-fluoro-phenyl)-amide (50 mg, 0.15 mmol, 1.0 eq), methyl iodide (15 mg, 0.15 mmol, 1.0 eq), and diisopropylethyl amine (39 mg, 0.45 mmol, 3.0 eq) were suspended in dichloromethane (1.5 mL) and stirred at room temperature overnight. After the usual workup the residue was purified by prep-HPLC affording the title compound.
HPLC-MS (purity; retention time) = 99 %; 1.54 min.
MS ISP (m/e): 352.2 (100) [(M+H)⁺].

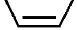

### Example 37

### 5-[1-(2-Methoxy-ethyl)-piperidin-4-yl]-1H-indole-2-carboxylic acid (4-fluoro-phenyl)-amide

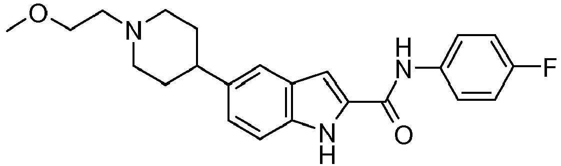

5-Piperidin-4-yl-1H-indole-2-carboxylic acid (4-fluoro-phenyl)-amide (50 mg, 0.15 mmol), methoxy-acetaldehyde (16 mg, 0.22 mmol), and molecular sieves were suspended in dichloromethane (1.5 mL) and stirred at 60°C for 1 hour. On cooling, sodium triacetoxyborohydride (95 mg, 0.45 mmol) was added portion wise to the mixture and the reaction was heated at 60°C for a further 3 hours. The reaction solution was allowed to cool to ambient temperature, washed with water (2 x 1 mL) and the aqueous layer was extracted with dichloromethane (3 x 3 mL). The combined organic layers were dried over magnesium sulfate, filtered and concentrated *in vacuo.* The residue was purified by prep-HPLC affording the title compound (8 mg, 14 %).
¹H NMR (400 MHz, MeOD): δ (ppm) = 1.85-1.92 (4H, m), 2.22-2.30 (2H, br m), 2.59-2.74 (3H, m), 3.13-3.18 (2H, m), 3.36 (3H, s), 3.59 (2H, t, *J=* 5.62 Hz), 7.10 (2H, t, *J=* 8.80 Hz), 7.17 (1H, dd, *J=* 7.09 Hz, 1.47 Hz), 7.23 (1H, s), 7.40 (1H, d, J = 8.56Hz), 7.50 (1H, s), 7.69-7.73 (2H, m).

The following examples (Table 5) were prepared in analogy to example 35 step g) (Method A) or example 36 (Method B) or example 37 (Method C), replacing benzoyl chloride or methyl iodide or methoxy-acetaldehyde with the corresponding acylating, alkylating or reductive amination reagent.

**Table 5**

| **Ex.** | **Reagent/Method** | **Name and Structure** | **MS (m/e)** |
|---|---|---|---|
| 38 | Benzaldehyde / C | **5-(1-Benzyl-piperidin-4-yl)-1H-indole-2-carboxylic acid (4-fluoro-phenyl)-amide** | ISP 428.1 [(M+H)⁺] |
| | | 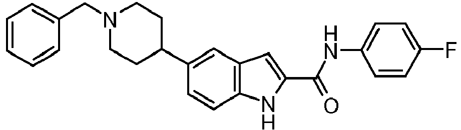 | |
| 39 | Allylbromide / B | **5-[1-Allyl-piperidin-4-yl]-1H-indole-2-carboxylic acid (4-fluoro-phenyl)-amide** | ISP 378.1 [(M+H)⁺] |
| | | 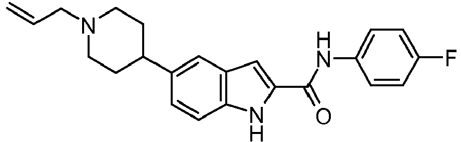 | |
| 40 | Propional / C | **5-[1-Propyl-piperidin-4-yl]-1H-indole-2-carboxylic acid (4-fluoro-phenyl)-amide** | ISP 380.2 [(M+H)⁺] |
| | | 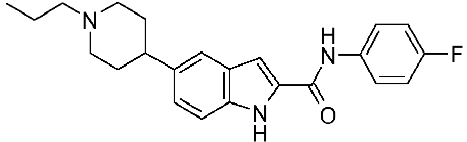 | |
| 41 | Methyl chloroformiate / A | **4-[2-(4-Fluoro-phenylcarbamoyl)-1H-indol-5-yl]-piperidine-1-carboxylic acid methyl ester** | ISP 396.1 [(M+H)⁺] |
| | | 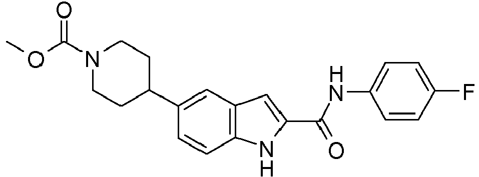 | |
| **Ex.** | **Reagent/Method** | **Name and Structure** | **MS (m/e)** |
| 42 | Acetaldehyde / C | **5-[1-Ethyl-piperidin-4-yl]-1H-indole-2-carboxylic acid (4-fluoro-phenyl)-amide** | ISP 366.2 [(M+H)⁺] |
| | | 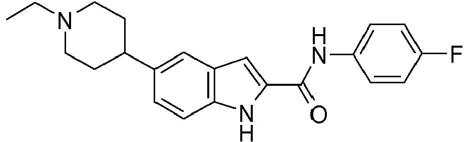 | |
| 43 | Methyl 2-bromo-acetate / B | **{4-[2-(4-Fluoro-phenylcarbamoyl)-1H-indol-5-yl]-piperidin-1-yl}-acetic acid methyl ester** | [(M+H)⁺] |
| | | 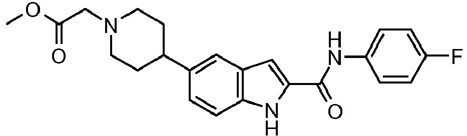 | |
| 44 | Propionyl chloride / A | **5-(1-Propionyl-piperidin-4-yl)-1H-indole-2-carboxylic acid (4-fluoro-phenyl)-amide** | ISP 394.1 [(M+H)⁺] |
| | | 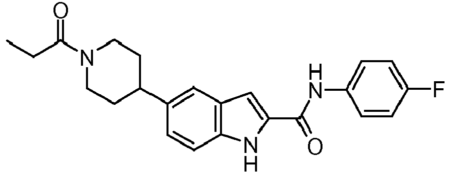 | |
| 45 | Phenylacetaldehyde / C | **5-(1-Phenethyl-piperidin-4-yl)-1H-indole-2-carboxylic acid (4-fluoro-phenyl)-amide** | ISP 442.2 [(M+H)⁺] |
| | | 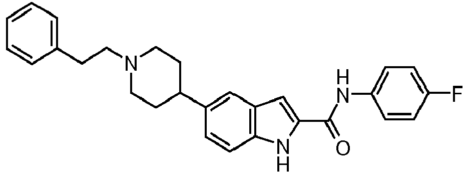 | |

### Example 46

### 5-(1-Isopropyl-piperidin-3-yl)-1H-indole-2-carboxylic acid (3-fluoro-phenyl)-amide

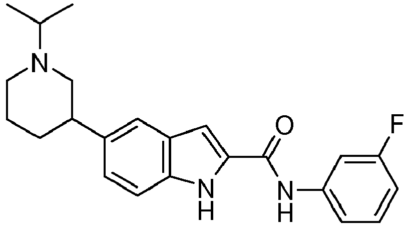

### a) 5-Pyridin-3-yl-1H-indole-2-carboxylic acid (3-fluoro-phenyl)-amide

The title compound was prepared in the same manner as described in example 21 step c) from 5-pyridin-3-yl-1H-indole-2-carboxylic acid ethyl ester (CAS Registry Number: 1012313-08-4) and 3-fluoraniline as an off-white solid (21 %).
MS ISP (m/e): 332.0 (100) [(M+H)⁺].

### b) 5-(1-Isopropyl-piperidin-3-yl)-1H-indole-2-carboxylic acid (3-fluoro-phenyl)-amide

To a solution of 5-pyridin-3-yl-1H-indole-2-carboxylic acid (3-fluoro-phenyl)-amide (36 mg, 0.11 mmol) in dimethyl formamide (2 mL) was added 2-bromopropane (20 mg, 0.16 mmol). The reaction was heated to 100°C overnight. The solvent was removed under reduced pressure after cooling and the residue was dissolved in methanol (3 mL). A spatula tip of platinum(IV) oxide hydrate was added and the reaction was stirred at room temperature overnight under an athmosphere of hydrogen. The reaction was filtered and the solvent was evaporated under reduced pressure. The residue was purified by column chromatography on silica gel (basic) with ethyl acetate as eluent to yield the title compound as a light yellow solid (9 mg, 21 %).
MS ISP (m/e): 380.3 (100) [(M+H)⁺].

### Example 47

### 5-(1-Isopropyl-piperidin-3-yl)-1H-indole-2-carboxylic acid (4-fluoro-phenyl)-amide

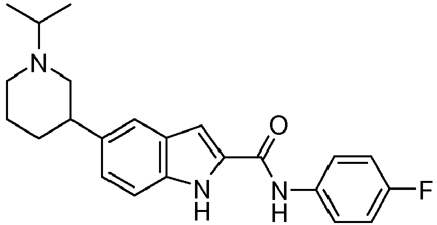

### a) 5-Pyridin-3-yl-1H-indole-2-carboxylic acid (4-fluoro-phenyl)-amide

To a solution of 4-fluoroaniline (0.234g) in dioxan (10 mL) was added trimethylaluminium (1.05 mL), this mixture was stirred for 1hour at room temperature. Then 5-pyridin-3-yl-1H-indole-2-carboxylic acid ethyl ester (0.16 g) in dioxan (1 mL) was added, reaction was stirred overnight at room temperature The reaction was quenched with water and extracted with ethyl acetate. The combined organic layers were dried over magnesium sulfate, filtered and concentrated in vacuo. The residue was purified by column chromatography on silica gel using heptane / ethyl acetate (1:1 v/v) as solvent and crystallized with diethylether to yield the title compound as an off-white solid (33 mg, 17 %).
MS ISN (m/e): 330.1 (100) [(M-H)-].

### b) 5-(1-Isopropyl-piperidin-3-yl)-1H-indole-2-carboxylic acid (4-fluoro-phenyl)-amide

The title compound was prepared in the same manner as described in example 46 step b) from 5-pyridin-3-yl-1H-indole-2-carboxylic acid (4-fluoro-phenyl)-amide and 2-bromopropane and then platin(IV)-oxid hydrate and hydrogene after column chromatography on basic silica gel using heptane / ethyl acetate (1:1 v/v) as an off-white solid (32 %).
MS ISP (m/e): 380.3 (100) [(M+H)⁺].

### Example 48

### 5-(1-Isopropyl-piperidin-4-yl)-1H-indole-2-carboxylic acid (4-fluoro-phenyl)-amide

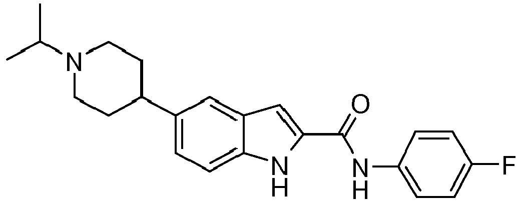

### a) 5-(1-Isopropyl-piperidin-4-yl)-1H-indole-2-carboxylic acid hydrochloride

5-(1-Isopropyl-piperidin-4-yl)-1H-indole-2-carboxylic acid ethyl ester (1.55 g) was dissolved in a 1:1 mixture of tetrahydrofuran (25 mL) and methanol (25 mL). To this solution was added lithium hydroxide monohydrate (207 mg) in water (12 mL). This mixture was refluxed for 1.5 hours and then tetrahydrofuran and methanol were evaporated. To the aqueous residue was added ice water, then pH was adjusted to pH 2 with 4 M aqueous hydrogen chloride solution. The resulting suspension was evaporated to yield the title compound as off-white solid (126 %).
MS ISP (m/e): 287.0 (100) [(M+H)⁺].

### b) 5-(1-Isopropyl-piperidin-4-yl)-1H-indole-2-carboxylic acid (4-fluoro-phenyl)-amide

5-(1-Isopropyl-piperidin-4-yl)-1H-indole-2-carboxylic acid hydrochloride (161 mg) was dissolved in DMF (5 mL), then TBTU (192 mg), 4-fluoroaniline (66 mg) and N-ethyldiisopropylamine (0.52 mL) were added. The reaction was stirred for 65 hours at room temperature. The solvent was evaporated; the residue was extracted with tetrahydrofuran / ethyl acetate, water and 10% aqueous Na₂CO₃ solution. Organic layers were dried over sodium sulfate, filtered and evaporated. The residue was purified by column chromatography on silica gel using dichloromethane / 2 N ammonia in methanol (97:3 to 9:1) to yield the title compound as light brown solid (130 mg, 64%).
MS ISP (m/e): 380.5 (100) [(M+H)⁺].

### Example 49

### 5-(1-Methanesulfonyl-piperidin-3-yl)-1H-indole-2-carboxylic acid (3,5-difluoro-phenyl)-amide

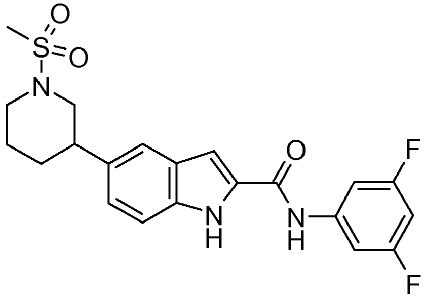

### a) 5-Pyridin-3-yl-1H-indole-2-carboxylic acid ethyl ester

5-Bromo-1H-indole-2-carboxylic acid ethyl ester (1.0 g), diethyl(3-pyridyl)borane (1.097 g), tetrakis-(triphenylphosphine)-palladium (0.431 g) and potassium carbonate (1.804 g) were refluxed at 95°C for 5 hours in a mixture of tetrahydrofuran (30 mL) and water (30 mL). The reaction mixture was allowed to cool to room temperature, the phases were separated, the aqueous phase was extracted three times with dichloromethane, the combined organic layers were washed with brine, dried over magnesium sulfate, filtered and concentrated in vacuo. The residue was crystallized in ethyl acetate, then purified by column chromatography on silica gel using dichloromethane/methanol (98:2 v/v), again recrystallized in ethyl acetate to yield the title compound as a white solid (445 mg, 45 %).
MS ISP (m/e): 267.1 (100) [(M+H)⁺].

### b) 5-Piperidin-3-yl-1H-indole-2-carboxylic acid ethyl ester

Hydrogenation of 5-pyridin-3-yl-1H-indole-2-carboxylic acid ethyl ester (2.7 g) in acetic acid (100 mL) in the presence of PtO₂ and hydrogene for 16 hours at 70°C under 3 bar of pressure. The solvent was evaporated, the residue was crystallized with diethylether to yield the title compound as a white solid (2.45 g, 87%).
MS ISP (m/e): 273.3 (100) [(M+H)⁺].

### c) 5-(1-Methanesulfonyl-piperidin-3-yl)-1H-indole-2-carboxylic acid ethyl ester

To a suspension of 5-piperidin-3-yl-1H-indole-2-carboxylic acid ethyl ester (200 mg) in dichloromethane (5 mL) was added triethylamine (223 mg) and methanesulfonylchloride (84 mg). The reaction was stirred for 30 minutes at room temperature. The reaction mixture was evaporated. The residue was suspended in water, basified with sodium carbonate and extracted with ethyl acetate. The combined organic layers were dried over magnesium sulfate, filtered and concentrated in vacuo. The residue was purified by column chromatography on silica gel using heptane/ethyl acetate (1:1 to 0:1) as eluent to yield the title compound as a light brown solid (33 mg, 13 %).
MS ISP (m/e): 368.1 (100) [(M+NH₄)⁺] / 351.2 (71) [(M+H)⁺].

### d) 5-(1-Methanesulfonyl-piperidin-3-yl)-1H-indole-2-carboxylic acid (3,5-difluoro-phenyl)-amide

The title compound was prepared in the same manner as described in example 21 step c) from 5-(1-methanesulfonyl-piperidin-3-yl)-1H-indole-2-carboxylic acid ethyl ester and 3,5-difluoraniline as a white solid (18 %).
MS ISN (m/e): 432.1 (100) [(M-H)-].

The following examples (Table 6) were prepared in analogy to example 49 step c) and d), replacing methane sulfonylchloride with the acylating agent.

**Table 6**

| **Ex.** | **Acylating reagent** | **Name and Structure** | **MS (m/e)** |
|---|---|---|---|
| 50 | isopropylsulfonyl chloride | **5-[1-(Propane-2-sulfonyl)-piperidin-3-yl]-1H-indole-2-carboxylic acid (3,5-difluoro-phenyl)-amide** | ISP 479.1 (100) [(M+NH₄)⁺], 462.3 (44) [(M+H)⁺]. |
| | | 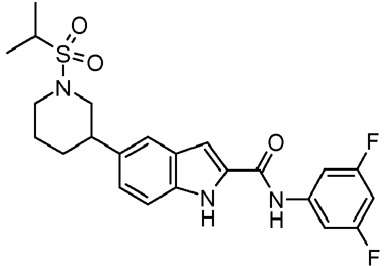 | |
| 51 | isobutyryl chloride | **5-(1-Isobutyryl-piperidin-3-yl)-1H-indole-2-carboxylic acid (3,5-difluorophenyl)-amide** | ISP 426.2 (100) [(M+H)⁺]. |
| | | 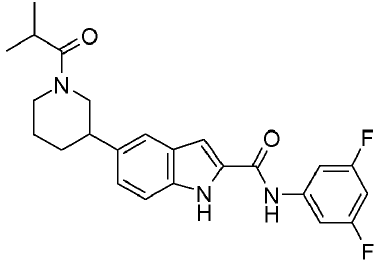 | |
| 52 | acetylchloride | **5-(1-Acetyl-piperidin-3-yl)-1H-indole-2-carboxylic acid (3,5-difluoro-phenyl)-amide** | ISP 398.3 (100) [(M+H)⁺]. |
| | | 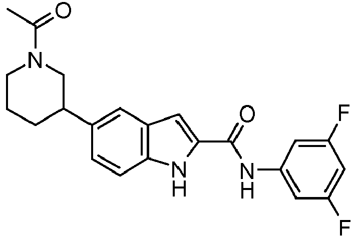 | |
| 53 | benzenesulfonyl chloride | **5-(1-Benzenesulfonyl-piperidin-3-yl)-1H-indole-2-carboxylic acid (3,5-difluorophenyl)-amide** | ISP 513.1 (100) [(M+NH₄)⁺], 496.1 (88) [(M+H)⁺]. |
| | | 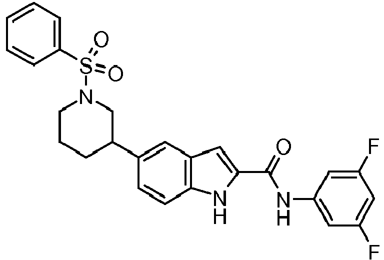 | |
| 54 | cyclopropanecarbonyl chloride | **5-(1-Cyclopropanecarbonyl-piperidin-3-yl)-1H-indole-2-carboxylic acid (3,5-difluoro-phenyl)-amide** | ISP 424.2 (100) [(M+H)⁺]. |
| | | 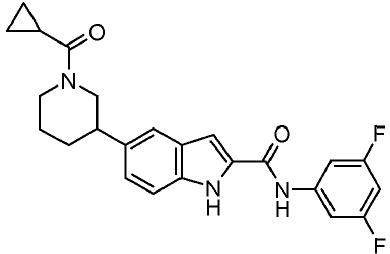 | |
| 55 | benzoylchloride | **5-(1-Benzoyl-piperidin-3-yl)-1H-indole-2-carboxylic acid (3,5-difluoro-phenyl)-amide** | ISP 460.3 (100) [(M+H)⁺]. |
| | | 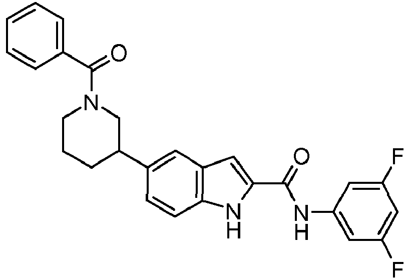 | |
| 56 | cyclopropanesulfonyl chloride | **5-(1-Cyclopropanesulfonyl-piperidin-3-yl)-1H-indole-2-carboxylic acid (3,5-difluoro-phenyl)-amide** | ISP 460.2 (100) [(M+NH₄)⁺], 477.1 (65) [(M+H)⁺]. |
| | | 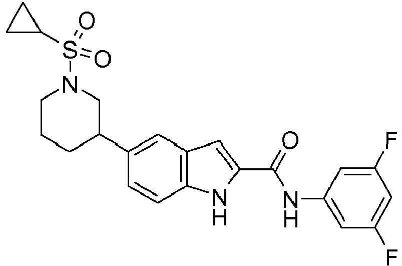 | |
| 57 | methoxyacetyl chloride | **5-[1-(2-Methoxy-acetyl)-piperidin-3-yl]-1H-indole-2-carboxylic acid (3,5-difluoro-phenyl)-amide** | ISP 428.3 (100) [(M+H)⁺]. |
| | | 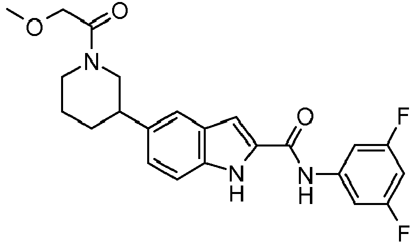 | |
| 58 | 1-methylimidazole-4-sulphonyl chloride | **5-[1-(1-Methyl-1H-imidazole-4-sulfonyl)-piperidin-3-yl]-1H-indole-2-carboxylic acid (3,5-difluoro-phenyl)-amide** | ISP 500.1 (100) [(M+H)⁺]. |
| | | 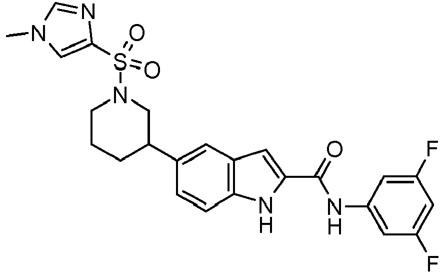 | |

### Example 59

### 5-(1-Methyl-piperidin-3-yl)-1H-indole-2-carboxylic acid (3,5-difluoro-phenyl)-amide

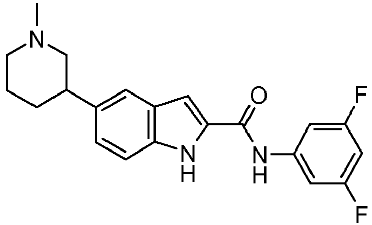

### a) 5-(1-Methyl-piperidin-3-yl)-1H-indole-2-carboxylic acid ethyl ester

The title compound was prepared in the same manner as described in example 21 step b) from 5-pyridin-3-yl-1H-indole-2-carboxylic acid ethyl ester and iodomethane as a white solid (89%).
MS ISP (m/e): 287.1 (100) [(M+H)⁺].

### b) 5-(1-Methyl-piperidin-3-yl)-1H-indole-2-carboxylic acid (3,5-difluoro-phenyl)-amide

The title compound was prepared in the same manner as described in example 20 step e) from 5-(1-methyl-piperidin-3-yl)-1H-indole-2-carboxylic acid ethyl ester and 3,5-difluoraniline after crystallization with ethyl acetate as a light yellow solid (8 %).
MS ISP (m/e): 370.2 (100) [(M+H)⁺].

### Example 60

### 5-[1-(2-Methoxy-ethyl)-piperidin-3-yl]-1H-indole-2-carboxylic acid (3,5-difluoro-phenyl)-amide

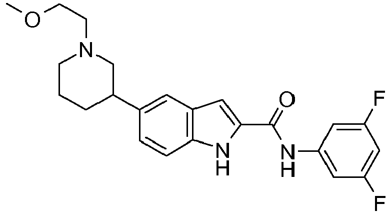

### a) 5-[1-(2-Methoxy-ethyl)-piperidin-3-yl]-1H-indole-2-carboxylic acid ethyl ester

The title compound was prepared in the same manner as described in example 21 step b) from 5-pyridin-3-yl-1H-indole-2-carboxylic acid ethyl ester and 2-bromoethyl methyl ether and then platin(IV)-oxid hydrate and hydrogen as a white oil (53 %).
MS ISP (m/e): 331.2 (100) [(M+H)⁺].

### b) 5-[1-(2-Methoxy-ethyl)-piperidin-3-yl]-1H-indole-2-carboxylic acid (3,5-difluoro-phenyl)-amide

The title compound was prepared in the same manner as described in example 20 step e) but at 110°C from 5-[1-(2-methoxy-ethyl)-piperidin-3-yl]-1H-indole-2-carboxylic acid ethyl ester and 3,5-difluoranilin after column chromatography on silica gel using dichloromethane / methanol (95:5 to 8:2) as a brown solid (10 %).
MS ISP (m/e): 414.4 (100) [(M+H)⁺].

### Example 61 and 62

### {3-[2-(3,5-Difluoro-phenylcarbamoyl)-1H-indol-5-yl]-piperidin-1-yl}-acetic acid methyl ester and 5-Piperidin-3-yl-1H-indole-2-carboxylic acid (3,5-difluoro-phenyl)-amide

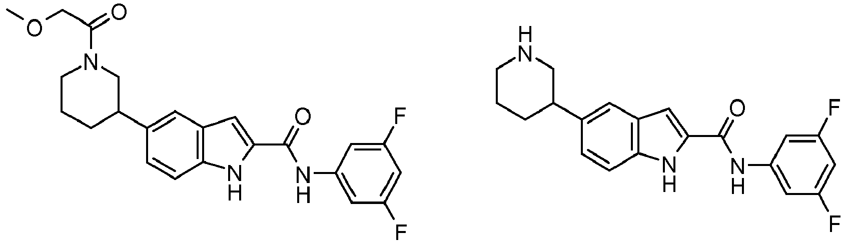

### b) 5-Pyridine-3-yl-1H-indole-2-carboxylic acid (3,5-difluoro-phenyl)-amide

The title compound was prepared in the same manner as described in example 20 step e) but at 120°C from 5-pyridin-3-yl-1H-indole-2-carboxylic acid ethyl ester and 3,5-difluoraniline as a brown- yellow solid (16 %).

### c) {3-[2-(3,5-Difluoro-phenylcarbamoyl)-1H-indol-5-yl]-piperidin-1-yl}-acetic acid methyl ester and 5-Piperidin-3-yl-1H-indole-2-carboxylic acid (3,5-difluoro-phenyl)-amide

To a solution of 5-pyridine-3-yl-1H-indole-2-carboxylic acid (3,5-difluoro-phenyl)-amide (40 mg) in ethyl acetate (2 mL) was added methyl-bromoacetate (230 mg), reaction was stirred overnight at room temperature and then evaporated. Methanol (2 mL) and platin(IV)-oxid hydrate (30 mg) were added, the reaction was stirred overnight at room temperature in the presence of hydrogen. Reaction was filtered, solvent was evaporated and the residue was purified by column chromatography on basic silica gel using dichloromethane / methanol (98:2 to 95:5) and crystallization in diethylether to yield the title compound {3-[2-(3,5-difluorophenylcarbamoyl)-1H-indol-5-yl]-piperidin-1-yl}-acetic acid methyl ester as yellow sticky solid (5 mg, 8 %), MS ISP (m/e): 428.3 (100) [(M+H)⁺]; and 5-piperidin-3-yl-1H-indole-2-carboxylic acid (3,5-difluoro-phenyl)-amide as a byproduct of the reaction as a light yellow solid (7 mg, 13 %), MS ISP (m/e): 356.1 (100) [(M+H)⁺].

### Example 63

### 5-(4-Isopropyl-piperazin-1-yl)-1H-indole-2-carboxylic acid (3,4-difluoro-phenyl)-amide

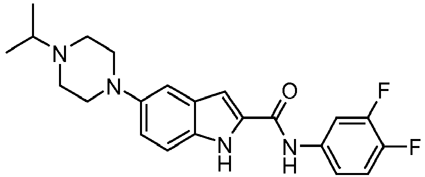

### a) 1-Isopropyl-4-(3-methyl-4-nitro-phenyl)-piperazine

A suspension of 4-fluoro-2-nitrotoluene (21.0 g, 12.9 mmol), 1-(2-propyl)piperazine (2.48 g, 19.3 mmol) and potassium carbonate (2.67 g, 19.3 mmol) in DMSO (30 mL) was heated to 80°C for 3 hours. Water (100 mL) was added and the reaction was extracted with diethyl ether. The combined organic layers were dried over sodium sulfate, filtered and the solvent was removed under vacuum. The crystals were stirred with pentane overnight, filtered and dried under reduced pressure to yield the title compound as light red crystals (2.93 g, 60 %)
MS ISP (m/e): 264.1 (100) [(M+H)⁺].

### b) 5-(4-Isopropyl-piperazin-1-yl)-1H-indole-2-carboxylic acid ethyl ester

1-Isopropyl-4-(3-methyl-4-nitro-phenyl)-piperazine (0.5 g, 1.9 mmol) dissolved in tetrahydrofuran (20 mL) was added drop wise to a solution of potassium ethanolat (294 mg, 3.3 mmol) and oxalic acid diethyl ester (486 mg, 3.3 mmol) within 20 minutes. The reaction was stirred at room temperature overnight and the solvent was removed under reduced pressure. The residue was dissolved in ethanol (100 mL) and cyclohexen (0.5 mL), acetic acid (0.5 mL) and 10% palladium on charcoal (0.05 g) were added. The reaction was heated to reflux under argon for 18 hours. The reaction was filtered and the solvent was evaporated under reduced pressure. The residue was stirred with diethyl ether. The precipitate was filtered off, washed with diethyl ether and dried under vacuum to yield the title compound as a yellow solid (185 mg, 31 %).
MS ISP (m/e): 316.3 (100) [(M+H)⁺].

### c) 5-(4-Isopropyl-piperazin-1-yl)-1H-indole-2-carboxylic acid (3,4-difluoro-phenyl)-amide

The title compound was prepared in the same manner as described in example 21 step c) from 5-(4-isopropyl-piperazin-1-yl)-1H-indole-2-carboxylic acid ethyl ester and 3,4-difluoranilin as a light yellow solid (38 %).
MS ISP (m/e): 399.2 (100) [(M+H)⁺].

### Example 64

### 5-(4-Isopropyl-piperazin-1-yl)-1H-indole-2-carboxylic acid (3,4-difluoro-phenyl)-amide

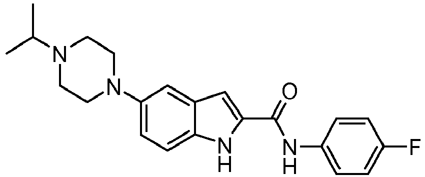

The title compound was prepared in the same manner as described in example 21 step c) from 5-(4-isopropyl-piperazin-1-yl)-1H-indole-2-carboxylic acid ethyl ester and 4-fluoranilin as a light yellow solid (39 %).
MS ISP (m/e): 381.2 (100) [(M+H)⁺].

### Example 65

### 5-(1-Isopropyl-piperidin-4-ylamino)-1H-indole-2-carboxylic acid (3,4-difluoro-phenyl)-amide

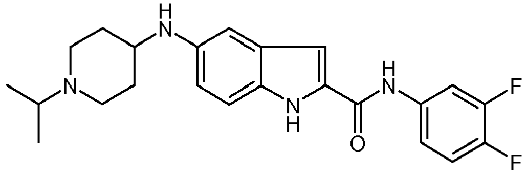

The title compound was prepared in the same manner as described in example 47 step c) from 5-(1-isopropyl-piperidin-4-ylamino)-1H-indole-2-carboxylic acid ethyl ester (CAS Registry Number: 900166-44-1) and 3,4-difluoraniline as a yellow solid (13 %).
MS ISP (m/e): 413.29 (100) [(M+H)⁺].

### Example 66

### N-(3-Fluorophenyl)-5-(1-(2-methoxyethyl)piperidin-3-yl)-1H-indole-2-carboxamide

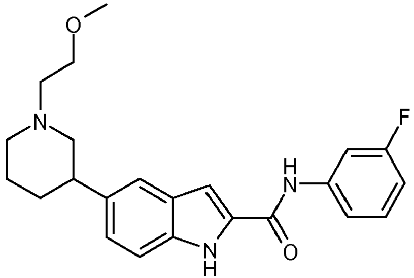

### a) N-(3-Fluorophenyl)-5-(pyridin-3-yl)-1H-indole-2-carboxamide

The title compound was prepared in the same manner as described in example 21 step c) from 5-(pyridin-3-yl)-1H-indole-2-carboxylic acid ethyl ester and 3-fluoraniline as an off-white solid (90 %).
MS ISP (m/e): 332.2 (100) [(M+H)⁺].

### b) N-(3-fluorophenyl)-5-(1-(2-methoxyethyl)piperidin-3-yl)-1H-indole-2-carboxamide

A suspension of N-(3-fluorophenyl)-5-(pyridin-3-yl)-1H-indole-2-carboxamide (249 mg, 750 µmol) in DMF (2.8 mL) was treated with 2-bromoethyl methyl ether (156 mg, 106 µL, 1.13 mmol) and then stirred for 17 hours at 100 °C (became a light brown solution). Then the light yellow suspension was evaporated and the residue was taken in ethanol (15 mL) and sodium borohydride (113 mg, 3.00 mmol) was added in portions. The reaction was stirred for 1.25 hours at 30 °C and for 20.5 hours at room temperature. It was concentrated to 1/3 volume, water (45 mL) was added and the mixture was extracted with chloroform (125 mL) twice. The organic layers were combined, dried over magnesium sulfate, filtered and evaporated. The residue was taken in ethanol (15 mL), palladium hydroxide (26.3 mg, 37.5 µmol) and ammonium formate (236 mg, 3.75 mmol) were added and the reaction was stirred 20 hours at 90 °C. The reaction was filtered and the filtrate was evaporated. Preparative HPLC gave the title compound as yellow semisolid (169.3 mg, 57 %).
MS ISP (m/e): 396.3 (100) [(M+H)⁺].

The following examples (Table 7) were prepared in analogy to example 66 step b), replacing 2-bromoethyl methyl ether with the alkylating agent.

**Table 7**

| **Ex.** | **Alkylating ragent** | **Name and Structure** | **MS (m/e)** |
|---|---|---|---|
| 67 | 2-(2-bromoethoxy)-tetrahydro-2H-pyran | **N-(3-Fluorophenyl)-5-(1-(2-hydroxyethyl)piperidin-3-yl)-1H-indole-2-carboxamide** | ISP 382.4 (100) [(M+H)⁺]. |
| | | 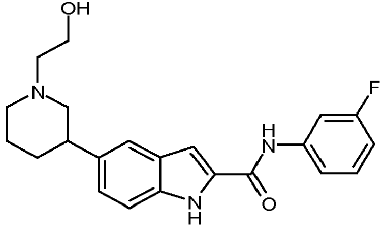 | |
| 68 | tetrahydrofurfuryl bromide | **N-(3-Fluorophenyl)-5-(1-((tetrahydrofuran-2-yl)methyl)piperidin-3-yl)-1H-indole-2-carboxamide** | ISP 422.3 (100) [(M+H)⁺]. |
| | | 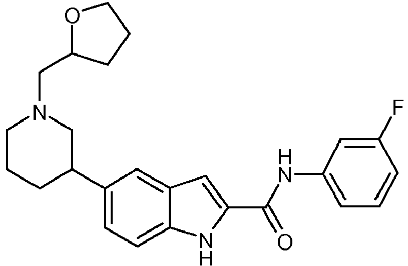 | |
| 69 | benzyl bromide | **N-(3-Fluorophenyl)-5-(piperidin-3-yl)-1H-indole-2-carboxamide** | ISP 338.4 (100) [(M+H)⁺]. |
| | | 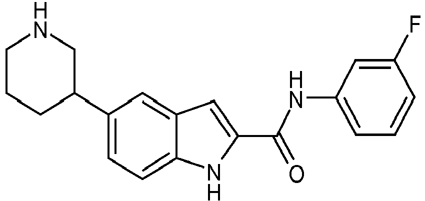 | |
| 70 | iodoethane | **5-(1-Ethylpiperidin-3-yl)-N-(3-fluorophenyl)-1H-indole-2-carboxamide** | ISP 366.2 (100) [(M+H)⁺]. |
| | | 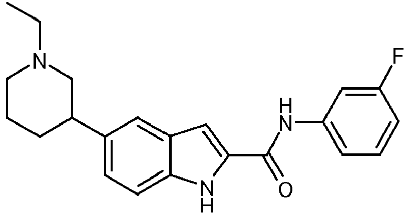 | |
| 71 | cyclopropylmethyl bromide | **5-(1-(Cyclopropylmethyl)piperidin-3-yl)-N-(3-fluorophenyl)-1H-indole-2-carboxamide** | ISP 392.3 (100) [(M+H)⁺]. |
| | | 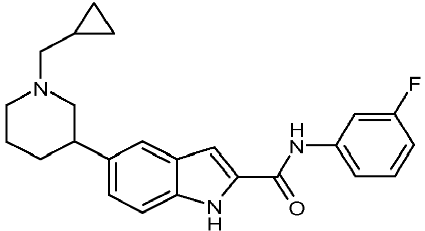 | |

### Example 72

### 5-(1-Cyclopropylpiperidin-3-yl)-N-(3-fluorophenyl)-1H-indole-2-carboxamide

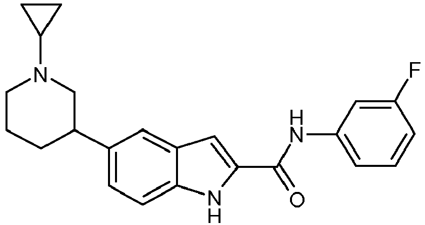

A mixture of N-(3-fluorophenyl)-5-(piperidin-3-yl)-1H-indole-2-carboxamide (100 mg, 296 µmol), (1-ethoxycyclopropoxy)trimethylsilane (51.7 mg, 59.6 µL, 296 µmol), acetic acid (17.8 mg, 17.0 µL, 296 µmol) and sodium triacetoxyborohydride (75.4 mg, 356 µmol) in dichloromethane (1 mL) was stirred for 40 hours at room temperature. The reaction was poured onto saturated aqueous NaHCO₃ solution (25 mL) and extracted with dichloromethane (40 mL) three times. The organic layers were combined, dried over sodium sulfate, filtered and evaporated. The crude product was purified by preparative HPLC (X-0584) to yield the title compound as a light yellow solid (53.6 mg, 48 %).
MS ISP (m/e): 378.4 (100) [(M+H)+].

### Example 73

### N-(3-Fluorophenyl)-5-(1-(2,2,2-trifluoroethyl)piperidin-3-yl)-1H-indole-2-carboxamide

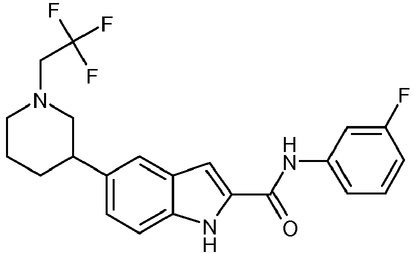

A mixture of N-(3-fluorophenyl)-5-(piperidin-3-yl)-1H-indole-2-carboxamide (100 mg, 296 µmol), 2,2,2-trifluoroethyl trifluoromethanesulfonate (117 mg, 72.6 µL, 504 µmol) and N,N-diisopropylethyl amine (218 mg, 289 µL, 1.69 mmol) in tetrahydrofuran (1.5 mL) was stirred for 20 hours at room temperature. The reaction was diluted with ethyl acetate (25 mL) and washed with 1M aqueous Na₂CO₃ solution (20 mL), water (20 mL) and brine (20 mL). The organic layer was dried over sodium sulfate, filtered off and evaporated. The residue was purified by preparative HPLC to yield the title compound as white solid (16.2 mg, 13 %).
MS ISP (m/e): 420.2 (100) [(M+H)+].

### Example 74

### 5-(1-Benzyl-pyrrolidin-3-yl)-1H-indole-2-carboxylic acid (5-fluoro-pyridin-3-yl)-amide

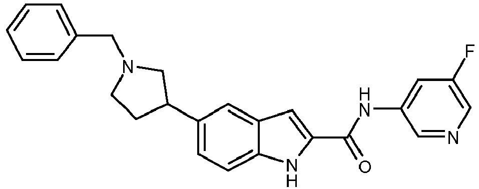

### a) 5-(1-Benzyl-pyrrolidin-3-yl)-1H-indole-2-carboxylic acid

The title compound was prepared in the same manner as described in example 4 step a) from 5-(1-benzyl-pyrrolidin-3-yl)-1H-indole as an off-white solid (71 %).

### b) 5-(1-Benzyl-pyrrolidin-3-yl)-1H-indole-2-carboxylic acid (5-fluoro-pyridin-3-yl)-amide

The title compound was prepared in the same manner as described in example 18 step c) from 5-(1-benzyl-pyrrolidin-3-yl)-1H-indole-2-carboxylic acid and 3-amino-5-fluoropyridine as a light brown oil (10 %).
MS ISP (m/e): 415.2 (100) [(M+H)⁺].

### Example 75

### N-(5-Fluoropyridin-3-yl)-5-(pyrrolidin-3-yl)-1H-indole-2-carboxamide

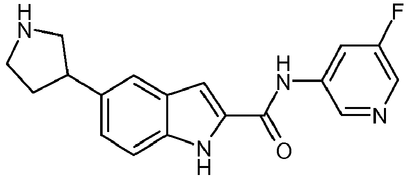

To a suspension of 5-(1-benzylpyrrolidin-3-yl)-N-(5-fluoropyridin-3-yl)-1H-indole-2-carboxamide (256 mg, 618 µmol) in methanol (5 mL) was added under an atmosphere of argon ammonium formate (195 mg, 3.09 mmol) and palladium on carbon 10% (26 mg, 24.4 µmol). The reaction was stirred warmed to 90°C and stirred for 1 hour under an atmosphere of argon at 90°C. The reaction was filtered over Hyflo and the filter was washed well with methanol. The concentrated filtrate was suspended in dichloromethane (3 mL) and filtered off to yield the title compound as a white solid (195 mg, 97 %).
MS ISP (m/e): 325.2 (100) [(M+H)⁺].

### Example 76 and 77

### N-(5-Fluoropyridin-3-yl)-5-(1-isopropylpyrrolidin-3-yl)-1H-indole-2-carboxamide and isopropyl 3-(2-(5-fluoropyridin-3-ylcarbamoyl)-1H-indol-5-yl)pyrrolidine-1-carboxylate

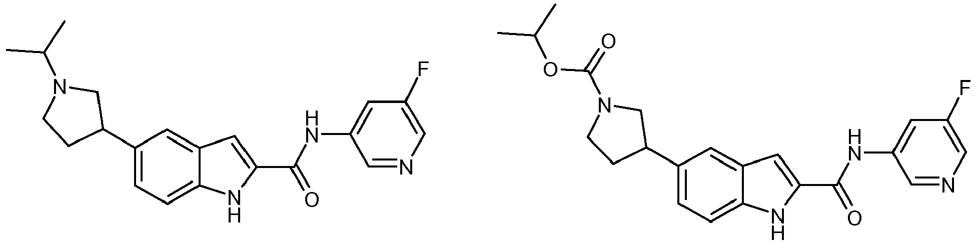

To a suspension of N-(5-fluoropyridin-3-yl)-5-(pyrrolidin-3-yl)-1H-indole-2-carboxamide (180 mg, 555 µmol) and potassium carbonate (383 mg, 2.77 mmol) in DMF (2 mL) was added at room temperature 2-bromopropane (341 mg, 261 µL, 2.77 mmol). The reaction was stirred for 24 hours in a closed flask. After the addition of 2-bromopropane (341 mg, 261 µL, 2.77 mmol) the reaction was stirred over weekend at room temperature. The reaction was diluted with ethyl acetate (15 mL) and washed with a 1 M aqueous solution of Na₂CO₃ (15 mL), water (15 mL) and brine (15 mL). The aqueous layers were extracted with ethyl acetate (15 mL). The organic layers were dried over sodium sulfate, filtered, concentrated and purified by column chromatography using heptane / (5 % triethylamine in ethyl acetate) / methanol (50:50:0 to 0:90:10) as eluent to yield isopropyl 3-(2-(5-fluoropyridin-3-ylcarbamoyl)-1H-indol-5-yl)pyrrolidine-1-carboxylate (108 mg, 53 %), MS ISP (m/e): 367.2 (100) [(M+H)⁺] and N-(5-fluoropyridin-3-yl)-5-(1-isopropylpyrrolidin-3-yl)-1H-indole-2-carboxamide (9 mg, 4 %; probably a side product in the starting material), MS ISP (m/e): 411.18 (100) [(M+H)⁺].

### Example 78 and 79

### 5-(1-(But-3-enyl)pyrrolidin-3-yl)-N-(5-fluoropyridin-3-yl)-1H-indole-2-carboxamide and 5-(1-(Cyclopropylmethyl)pyrrolidin-3-yl)-N-(5-fluoropyridin-3-yl)-1H-indole-2-carboxamide

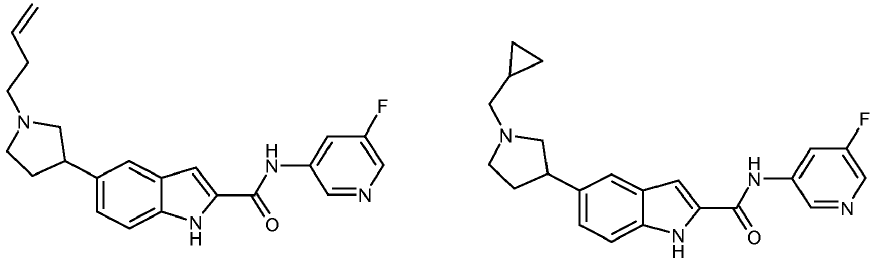

To a solution of N-(5-fluoropyridin-3-yl)-5-(pyrrolidin-3-yl)-1H-indole-2-carboxamide (160 mg, 493 µmol) in DMF (2 mL) was added potassium carbonate (341 mg, 2.47 mmol) and bromocyclobutane (333 mg, 233 µL, 2.47 mmol). The suspension was stirred for 18 hours at room temperature. After the addition of bromocyclobutane (333 mg, 233 µL, 2.47 mmol) the reaction was stirred for 20 hours at room temperature. The reaction was diluted with ethyl acetate (15 mL) and washed with a 1 M aqueous solution of Na₂CO₃ (15 mL), water (15 mL) and brine (15 mL). The aqueous layers were extracted with ethyl acetate (15 mL). The organic layers were dried over sodium sulfate, filtered, concentrated and purified by column chromatography using dichloromethane / 2N ammonia in methanol (97:3 to 90:10) as eluent to yield 5-(1-(but-3-enyl)pyrrolidin-3-yl)-N-(5-fluoropyridin-3-yl)-1H-indole-2-carboxamide as an off-white solid (25 mg, 13 %), MS ISP (m/e): 379.2 (100) [(M+H)⁺] and 5-(1-(cyclopropylmethyl)pyrrolidin-3-yl)-N-(5-fluoropyridin-3-yl)-1H-indole-2-carboxamide as an off-white solid (56 mg, 30 %), MS ISP (m/e): 379.2 (100) [(M+H)⁺].

### Example 80 and 81

### 5-(1-Cyanomethyl-pyrrolidin-3-yl)-1H-indole-2-carboxylic acid (5-fluoro-pyridin-3-yl)-amide and 1-Cyanomethyl-5-(1-cyanomethyl-pyrrolidin-3-yl)-1H-indole-2-carboxylic acid (5-fluoro-pyridin-3-yl)-amide

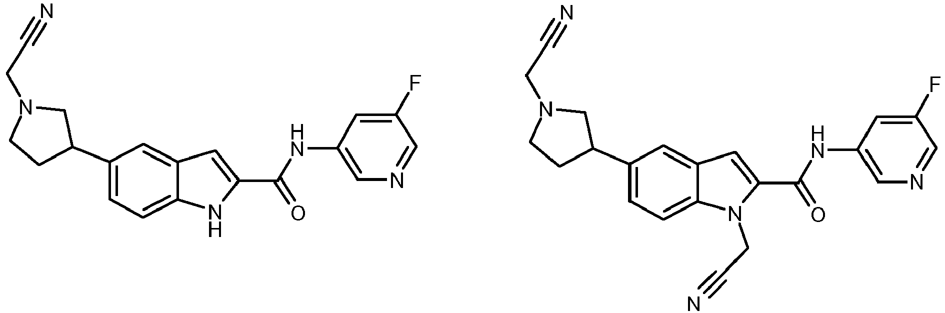

To a solution of N-(3-fluorophenyl)-5-(pyrrolidin-3-yl)-1H-indole-2-carboxamide (122 mg, 377 µmol) in DMF (1.5 mL) was added at 0°C potassium carbonate (261 mg, 1.89 mmol) and bromoacetonitrile (226 mg, 131 µl, 1.89 mmol). The reaction was stirred for 4 hours at room temperature. The reaction was diluted with ethyl acetate (15 mL) and washed with a 1 M aqueous solution of Na₂CO₃ (15 mL), water (15 mL) and brine (15 mL). The aqueous layers were extracted with ethyl acetate (15 mL). The organic layers were dried over sodium sulfate, filtered, concentrated and purified by column chromatography using heptane / (5 % triethylamine in ethyl acetate) / methanol (50:50:0 to 0:90:10) as eluent to yield 5-(1-cyanomethyl-pyrrolidin-3-yl)-1H-indole-2-carboxylic acid (5-fluoro-pyridin-3-yl)-amide as a light brown solid (41 mg, 30 %), MS ISP (m/e): 364.2 (100) [(M+H)⁺] and 1-cyanomethyl-5-(1-cyanomethyl-pyrrolidin-3-yl)-1H-indole-2-carboxylic acid (5-fluoro-pyridin-3-yl)-amide as a light brown solid (11 mg, 7 %), MS ISP (m/e): 403.2 (100) [(M+H)⁺].

### Example 82

### N-(5-Fluoropyridin-3-yl)-5-(1-(2-methoxyacetyl)pyrrolidin-3-yl)-1H-indole-2-carboxamide

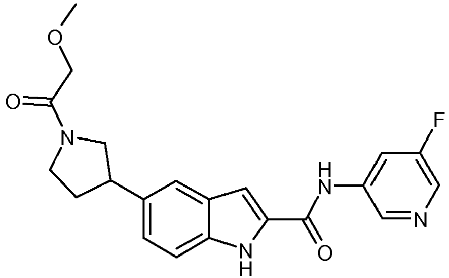

To a solution of N-(5-fluoropyridin-3-yl)-5-(pyrrolidin-3-yl)-1H-indole-2-carboxamide (172 mg, 530 µmol) in DMF (1.5 mL) was added N,N-diisopropyethlyamine (137 mg, 185 µL, 1.06 mmol) and methoxyacetyl chloride (86.3 mg, 72.5 µL, 795 µmol). The reaction was exothermic. The red solution was stirred for 1 hour at room temperature. The reaction was diluted with ethyl acetate (15 mL) and washed with a 1 M aqueous solution of Na₂CO₃ (15 mL), water (15 mL) and brine (15 mL). The aqueous layers were extracted with ethyl acetate (15 mL). The organic layers were dried over sodium sulfate, filtered, concentrated and suspended in dichloromethane / TBME (1:1) (10 mL) and filtered off to yield the title compound as a light brown solid (117 mg, 56 %).
MS ISP (m/e): 397.2 (100) [(M+H)⁺].

### Example 83

### 5-(1-(6-Cyanopyridazin-3-yl)pyrrolidin-3-yl)-N-(5-fluoropyridin-3-yl)-1H-indole-2-carboxamide

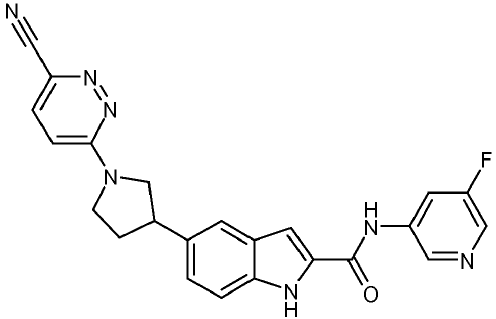

To a solution of N-(5-fluoropyridin-3-yl)-5-(pyrrolidin-3-yl)-1H-indole-2-carboxamide (99 mg, 305 µmol, Eq: 1.00) in NMP (1 mL) was added N,N-diisopropyethlyamine (118 mg, 160 µL, 916 µmol) and 6-chloropyridazine-3-carbonitrile (51.1 mg, 366 µmol). The reaction was exothermic. The red solution was stirred for 1 hour at room temperature. The reaction was diluted with ethyl acetate (15 mL) and washed with a 1 M aqueous solution of Na₂CO₃ (15 mL), water (15 mL) and brine (15 mL). The aqueous layers were extracted with ethyl acetate (15 mL). The organic layers were dried over sodium sulfate, filtered, concentrated and suspended in boiling ethanol (3 mL). After cooling to 0°C the suspension was filtered off and washed with ethanol (1 mL) to yield the title compound as an off-white solid (97 mg, 74 %).
MS ISP (m/e): 428.3 (100) [(M+H)⁺].

### Example 84

### 5-(1-(4-Fluorophenylsulfonyl)pyrrolidin-3-yl)-N-(5-fluoropyridin-3-yl)-1H-indole-2-carboxamide

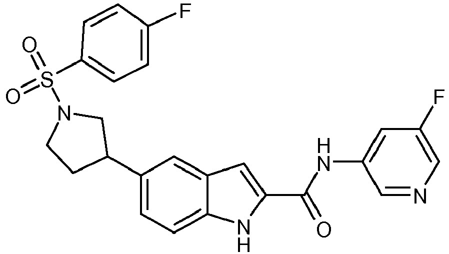

To a turbid solution of N-(5-fluoropyridin-3-yl)-5-(pyrrolidin-3-yl)-1H-indole-2-carboxamide (70 mg, 216 µmol) in NMP (1 mL) was added N,N-diisopropyethlyamine (41.8 mg, 56.5 µL, 324 µmol) and 4-fluorobenzene-1-sulfonyl chloride (44.1 mg, 227 µmo). The reaction was stirred for 3 hours at room temperature. The reaction was diluted with ethyl acetate (15 mL) and washed with a 1 M aqueous solution of Na₂CO₃ (15 mL), water (15 mL) and brine (15 mL). The aqueous layers were extracted with ethyl acetate (15 mL). The organic layers were dried over sodium sulfate, filtered, concentrated and purified by column chromatography using heptane / ethyl acetate (66:34 to 30:70) as eluent to yield the title compound as a white solid (60 mg, 58 %).
MS ISP (m/e): 483.3 (100) [(M+H)⁺].

### Example 85

### 5-(5,6-Dihydro-4H-pyran-3-yl)-1H-indole-2-carboxylic acid (3,4-difluoro-phenyl)-amide

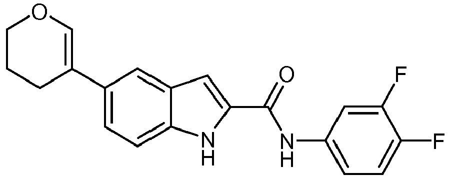

### a) 5-Bromo-1H-indole-2-carboxylic acid (3,4-difluoro-phenyl)-amid

A mixture of 5-bromoindole-2-carboxylic acid (2 g), 3,4-difluoroaniline (1.63 g), EDC*HCl (2.44 g), 1-hydroxybenzotriazole (1.49 g) and N,N-diisopropylethylamine (3.64 mL) in tetrahydrofuran (60 mL) was stirred at room temperature for 20 hours. The reaction was diluted with ethyl acetate, washed water and brine. The combined organic layers were dried over magnesium sulfate. The concentrated filtered solution was purified by flash chromatography using heptane / ethyl acetate (95:5 to 0:100) as eluent to yield the title compound as a light yellow solid (1.8 g, 62 %).

### b) 5-(4,4,5,5-Tetramethyl-[1,3,2]dioxaborolan-2-yl)-1H-indole-2-carboxylic acid (3,4-difluoro-phenyl-amid

The title compound was prepared in the same manner as described in example 17 step b) from 5-bromo-1H-indole-2-carboxylic acid (3,4-difluoro-phenyl)-amid as a light yellow solid (85 %). MS ISP (m/e): 397.5 (100) [(M+H)⁺].

### c) 5-(1-Benzyl-2,5-dioxopyrrolidin-3-yl)-N-(3-fluorophenyl)-1H-indole-2-carboxamide

The title compound was prepared in the same manner as described in example 1 step a) from 1-benzyl-1H-pyrrole-2,5-dione and N-(3-fluorophenyl)-5-(4,4,5,5-tetramethyl-1,3,2-dioxaborolan-2-yl)-1H-indole-2-carboxamide as a light brown solid (14 %).
MS ISN (m/e): 440.3 (100) [(M-H)-].

### d) 5-(5,6-Dihydro-4H-pyran-3-yl)-1H-indole-2-carboxylic acid (3,4-difluoro-phenyl)-amide

A solution of 5-(4,4,5,5-tetramethyl-[1,3,2]dioxaborolan-2-yl)-1H-indole-2-carboxylic acid (3,4-difluoro-phenyl)-amide (270 mg) and 5-bromo-3,4-dihydro-2H-pyran (122 mg, CAS: 26274-19-1) in toluene (14 mL) and ethanol (6 mL) was purged with argon for 5 minutes. 1,1-Bis(diphenylphosphino)ferrocen palladium(II) chloride (27.7 mg), was added and the mixture was heated to 80°C. Sodium carbonate (1.01 g) in water (7 mL) was added and the reaction was stirred at 80°C for 18 hours. The reaction was diluted with ethyl acetate, washed with water and brine, dried over magnesium sulfate, filtered and concentrated. The residue was purified by column chromatography using heptane / ethyl acetate (95:5 to 70:30) as eluent to yield the title compound as a light yellow solid (93 mg, 39 %).
MS ISN (m/e): 353.4 (100) [(M-H)-].

### Example 86

### 5-(Tetrahydro-pyran-3-yl)-1H-indole-2-carboxylic acid (3,4-difluoro-phenyl)-amide

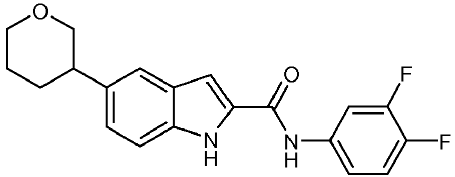

A mixture of 5-(5,6-Ddhydro-4H-pyran-3-yl)-1H-indole-2-carboxylic acid (3,4-difluoro-phenyl)-amide (86 mg) and palladium 10% on carbon (100 mg) in methanol (30 mL) and tetrahydrofuran (20 mL) was stirred for 2.5 days at room temperature under an atmosphere of hydrogen. The mixture was filtered and concentrated to yield the tile compound as a white solid (87 mg, 95 %).
MS ISN (m/e): 355.4 (100) [(M-H)⁻].

### Example 87

### 5-(1-Isopropyl-1,2,3,6-tetrahydro-pyridin-4-yl)-1H-indole-2-carboxylic acid (3,5-difluorophenyl)-amide

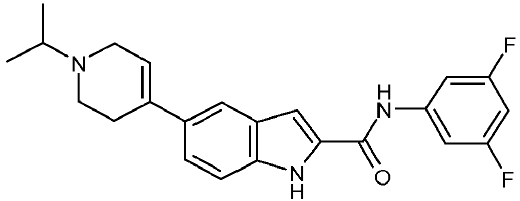

### a) Trifluoro-methanesulfonic acid 1-isopropyl-1,2,3,6-tetrahydro-pyridin-4-yl ester

To a solution ofN,N-diisopropylamine (2.1 mL) in THF (15 mL) was added n-butyl lithium (1.6 M in hexanes, 9.7 mL) drop wise at -78 °C under argon atmosphere. The mixture was stirred for 90 minutes at -78 °C. A solution of 1-(1'-methylethyl)-4-piperidone (2.0 g) in THF (10 mL) was added drop wise at -70 °C, after 20 minutes a solution of phenyltrifluoromethanesulfonimide (5.5 g) in THF (15 mL) was added drop wise at the same temperature. The mixture was allowed to warm to 0 °C and was stirred for additional 4 hours. The solvent was evaporated and the residue was dissolved in diethyl ether, washed with 1N aqueous sodium hydroxide solution, water and brine, dried over magnesium sulfate, filtered and evaporated to yield the title compound as an orange liquid (3.87 g, 98 %).
MS ISP (m/e): 274.0 (100) [(M+H)⁺].

### b) 5-Bromo-1-(4-methoxy-benzyl)-1H-indole-2-carboxylic acid ethyl ester

To a solution of 5-bromoindole-2-carboxylic acid ethyl ester (500 mg) in DMF (10 mL) were added potassium carbonate (521 mg) and 4-methoxybenzyl bromide (563 mg) at room temperature. The mixture was stirred for 18 hours at room temperature, heated to 50 °C and stirred for additional 20 hours. The reaction was diluted with ethyl acetate, washed with 1N aqueous hydrogen chloride solution, brine, dried over magnesium sulfate, filtered and evaporated. The residue was purified by column chromatography using heptane / ethyl acetate (95:5 to 70:30) as eluent to yield the title compound as a white solid (396 mg, 55 %).
MS ISP (m/e): 387 (100) [(M+H)⁺].

### c) 5-Bromo-1-(4-methoxy-benzyl)-1H-indole-2-carboxylic acid (3,5-difluoro-phenyl)-amide

The title compound was prepared in the same manner as described in example 20 step e) from 5-bromo-1-(4-methoxy-benzyl)-1H-indole-2-carboxylic acid ethyl ester and 3,5-difluoroaniline as a light yellow solid (58 %).
MS ISN (m/e): 471.4/469.4 (100) [(M-H)⁻].

### d) 1-(4-Methoxy-benzyl)-5-(4,4,5,5-tetramethyl-[1,3,2]dioxaborolan-2-yl)-1H-indole-2-carboxylic acid (3,5-difluoro-phenyl)-amide

The title compound was prepared in the same manner as described in example 17 step b) from 5-bromo-1-(4-methoxy-benzyl)-1H-indole-2-carboxylic acid (3,5-difluoro-phenyl)-amide as a light yellow viscous oil (69 %).

### e) 5-(1-Isopropyl-1,2,3,6-tetrahydro-pyridin-4-yl)-1-(4-methoxy-benzyl)-1#H!-indole-2-carboxylic acid (3,4-difluoro-phenyl)-amide

1-(4-Methoxy-benzyl)-5-(4,4,5,5-tetramethyl-[1,3,2]dioxaborolan-2-yl)-1H-indole-2-carboxylic acid (3,5-difluoro-phenyl)-amide (210 mg) and trifluoro-methanesulfonic acid 1-isopropyl-1,2,3,6-tetrahydro-pyridin-4-yl ester (277 mg) were dissolved in dioxane (2.4 mL) and 1M aqueous sodium carbonate solution (0.93 mL). Argon was bubbled through the solution and tetrakis(triphenylphospnine) palladium (23.6 mg) was added. The mixture was heated for 5 hours to 90 °. The reaction was diluted with ethyl acetate, washed with water and brine, dried over magnesium sulfate, filtered and evaporated. The residue was purified by column chromatography using dichloromethane / methanol (100:0 to 90:10) as eluent to yield the title compound as an orange viscous oil (209 mg, 42 %).

### f) 5-(1-Isopropyl-1,2,3,6-tetrahydro-pyridin-4-yl)-1H-indole-2-carboxylic acid (3,5-difluoro-phenyl)-amide

A suspension of 5-(1-isopropyl-1,2,3,6-tetrahydro-pyridin-4-yl)-1-(4-methoxy-benzyl)-1H-indole-2-carboxylic acid (3,4-difluoro-phenyl)-amide (80 mg), TFA (365 µL) and anisole (16.9 µL) was stirred for 5 hours at 60 °C and was left at room temperature over the weekend.

The suspension was dissolved in ethyl acetate and made basic with saturated aqueous sodium hydrogen carbonate solution. The organic layer was washed with water and brine, dried over magnesium sulfate, filtered and evaporated. The residue was purified by column chromatography using dichloromethane / methanol (100:0 to 90:10) as eluent to yield the title compound as a yellow solid (9 mg, 15 %).
MS ISN (m/e): 394.4 (100) [(M-H)⁻].

### Example 88

### 5-[1-(2-Oxo-pyrrolidin-1-yl)-ethyl]-1H-indole-2-carboxylic acid (3,5-difluoro-phenyl)-amide

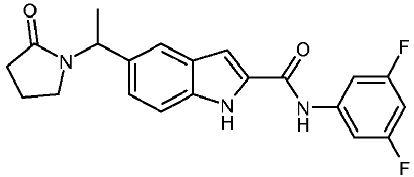

### a) 5-[1-(2-Oxo-pyrrolidin-1-yl)-vinyl]-1H-indole-2-carboxylic acid ethyl ester

Palladium-(II)-acetat (17 mg) and tri-(o-tolyl)phospine (68 mg) were dissolved in DMF (10 mL), then 5-bromo-1H-indole-2-carboxylic acid ethyl ester (400 mg), sodium hydrogen carbonate (501 mg), N,N-diisopropyl ethyl amine (1.02 mL) and 1-vinyl-2-pyrrolidon (207 mg) were added. The reaction was stirred at 125°C for 1.5 hours, followed by extraction with ethyl acetate and water. Column chromatography on silica gel using heptane / ethyl acetate (1:1) yielded the title compound as the minor component of the reaction as a light grey solid (161 mg, 36 %).
MS ISP (m/e): 299.2 (100) [(M+H)+].

### b) 5-[1-(2-Oxo-pyrrolidin-1-yl)-ethyl]-1H-indole-2-carboxylic acid ethyl ester

Hydrogenation of 5-[1-(2-oxo-pyrrolidin-1-yl)-vinyl]-1H-indole-2-carboxylic acid ethyl ester in methanol in the presence of PtO2 and hydrogene overnight at room temperature. Solvent was evaporated, the residue was dried under high vacuum to yield the title compound as a light grey viscous oil (73%).
MS ISP (m/e): 301.3 (23) [(M+H)+].

### c) 5-[1-(2-Oxo-pyrrolidin-1-yl)-ethyl]-1H-indole-2-carboxylic acid (3,5-difluoro-phenyl)-amide

The title compound was prepared in the same manner as described in example 21 step e) but at 110°C from 5-[1-(2-oxo-pyrrolidin-1-yl)-ethyl]-1H-indole-2-carboxylic acid ethyl ester and 3,5-difluoranilin after crystallization in diethylether as a light grey solid (27 %).
MS ISP (m/e): 384.2 (100) [(M+H)⁺].

### Example 89

### 5-[1-(2-Oxo-piperidin-1-yl)-ethyl]-1H-indole-2-carboxylic acid (3,5-difluoro-phenyl)-amide

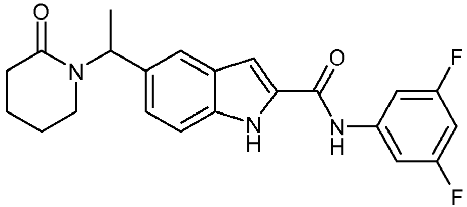

### a) 1-Vinyl-piperidin-2-one

Delta-valerolactam (2 g) was dissolved in butylvinylether (26 ml). Bis-trifluoroaceto-(4,7-diphenyl-1,10-phenanthroline)palladium(II) (0.134 g) was added, mixture was stirred for 5 hours at 75°C to yield the title compound after column chromatography on silica gel using heptane/EtOAc (7:3) and crystallization in pentane for 1 hour at -20°C and filtration as a colourless solid (0.645 g, 26 %).

### b) 5-[1-(2-Oxo-piperidin-1-yl)-vinyl]-1H-indole-2-carboxylic acid ethyl ester

The title compound was prepared in the same manner as example 88 step a) from ethyl-5-bromoindole-2-carboxylate and 1-vinyl-piperidin-2-one after column chromatography on silica gel using heptane / ethyl acetate (70:30 to 0:100) and crystallization in pentane as a byproduct of the reaction as a yellow solid (34 %).

### c) 5-[1-(2-Oxo-piperidin-1-yl)-ethyl]-1H-indole-2-carboxylic acid ethyl ester

The title compound was prepared in the same manner as example 88 step b) from 5-[1-(2-oxo-piperidin-1-yl)-vinyl]-1H-indole-2-carboxylic acid ethyl ester as a yellow solid (98 %).
MS ISP (m/e): 299.5 (100) [(M+H)⁺].

### d) 5-[1-(2-Oxo-piperidin-1-yl)-ethyl]-1H-indole-2-carboxylic acid (3,5-difluoro-phenyl)-amide

The title compound was prepared in the same manner as described in example 21 step e) but at 110°C from 5-[1-(2-oxo-piperidin-1-yl)-ethyl]-1H-indole-2-carboxylic acid ethyl ester and 3,5-difluoranilin after column chromatography on silica gel using heptane / ethyl acetate (7:3) and crystallization in pentane as an off-white solid (71 %).
MS ISNP (m/e): 396.4 (100) [(M-H)⁻].

### Example 90

### 5-(1-Pyrrolidin-1-yl-ethyl)-1H-indole-2-carboxylic acid (3,5-difluoro-phenyl)-amide

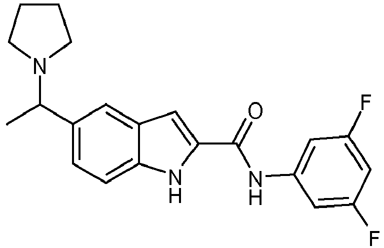

### a) 5-(1-Pyrrolidin-1-yl-ethyl)-1H-indole-2-carboxylic acid ethyl ester

A suspension of 5-acetyl-1H-indole-2-carboxylic acid ethyl ester (200 mg, 0.865 mmol; CAS: 31380-56-0), pyrrolidine (79 µL, 0.95 mmol), sodium triacetoxyborohydride (275 mg, 1.30 mmol) and acetic acid (50 µL, 0.86 mmol) in THF (2 mL) was stirred for 18 hours at room temperature. The suspension was stirred for 3 hours at 60°C. After cooling to room temperature pyrrolidine (160 µL, 1.90 mmol) and sodium triacetoxyborohydride (275 mg, 1.30 mmol) were added and the reaction was stirred for 18 hours at 60°C. After the addition of magnesium sulfate the reaction was irradiated for 30 minutes at 150°C. The reaction was diluted with ethyl acetate (15 mL) and washed with a 1 M aqueous solution of Na₂CO₃ (15 mL) and brine (15 mL). The aq. Layers were extracted with ethyl acetate (15 mL). The organic layers were dried over sodium sulfate, filtered and concentrated. The residue was purified by column chromatography on silica gel using heptane /ethyl acetate / (5 % NEt₃ in ethyl acetate) (50:50:0 to 50:0:50) to yield the title compound as a colorless oil (163, 66 %).
MS ISP (m/e): 287.1 (100) [(M+H)⁺].

### b) 5-(1-Pyrrolidin-1-yl-ethyl)-1H-indole-2-carboxylic acid

To a solution of 5-(1-pyrrolidin-1-yl-ethyl)-1H-indole-2-carboxylic acid ethyl ester (160 mg, 0.559 mmol) in THF (4 mL) was added a 1 M aqueous solution of sodium hydroxide (2.2 mL, 2.2 mmol). The raction was stirred for 18 hours at 100°C. After cooling to room temperature the reaction was set to pH 5 by addition of a 1 M aqueous solution of hydrochloric acid. The reaction was concentrated in vacuo. After the addition of THF (20 mL) the reaction was concentrated again. The residue was suspended in ethanol (10 mL), dried over sodium sulfate, filtered off and washed well with ethanol to yield the title compound as a white solid (156 mg, quant.).
MS ISP (m/e): 259.1 (100) [(M+H)⁺].

### c) 5-(1-Pyrrolidin-1-yl-ethyl)-1H-indole-2-carboxylic acid (3,5-difluoro-phenyl)-amide

To a suspension of 5-(1-pyrrolidin-1-yl-ethyl)-1H-indole-2-carboxylic acid (153 mg, 0.592 mmol) in NMP (2 mL) was added 3,5-difluoroaniline (92 mg, 0.711 mmol) and N-methylmorpholine (195 µL, 1.78 mmol). After stirring for 15 minutes [2-(1H-benzotriazol-1-yl)-1,1,3,3-tetramethyluronium hexafluorophosphate] (337 mg, 0.888 mmol) was added and the reaction was stirred at room temperature for 3 days. The solution was diluted with ethyl acetate (15 mL) and washed with a 1 M aqueous solution of Na₂CO₃ (15 mL), twice with water (15 mL) and brine (15 mL). The organic layers were dried over sodium sulfate, filtered and concentrated. The residue was purified by column chromatography on silica gel using heptane / ethyl acetate / (5 % NEt₃ in ethyl acetate) (50:50:0 to 10:0:90) to yield the title compound as an off-white foam (37 mg, 17 %).
MS ISP (m/e): 370.2 (100) [(M+H)⁺].

### Example 91

### 5-(1-(1-Benzylpyrrolidin-3-yl)vinyl)-N-(3,5-difluorophenyl)-1H-indole-2-carboxamide

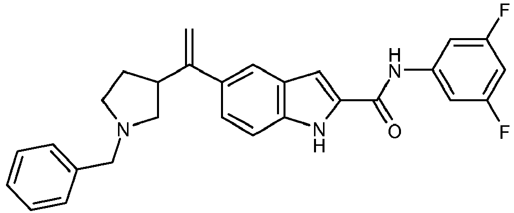

### a) Ethyl 5-(1-benzylpyrrolidine-3-carbonyl)-1H-indole-2-carboxylate

To a solution of 1-benzylpyrrolidine-3-carbonyl chloride (3.65 g, 16.3 mmol) in 1,2-dichloroethane (23 mL) was added under an atmosphere of nitrogen at 0°C aluminum chloride (3.26 g, 24.5 mmol). After stirring for 30 minutes at 0°C ethyl 1H-indole-2-carboxylate (3.09 g, 16.3 mmol) was added and stirred for 18 hours at room temperature. After the addition of aluminum chloride (3.26 g, 24.5 mmol) the reaction was stirred for 3 hours at 70°C. After cooling to 0°C the reaction was diluted with ethyl acetate (100 mL) and basified by drop wise addition of a 1 M aqueous solution of sodium carbonate (200 mL). After stirring for 30 minutes the suspension was filtered over Hyflo and was washed well with ethyl acetate. The aqueous layer was extracted with ethyl acetate (150 mL). The organic layers were washed with a 1 M aqueous solution of sodium carbonate (100 mL). The combined organic layers were dried over sodium sulfate, filtered and purified by flash chromatography on silica gel using heptane / (ethyl acetate: NEt₃ 95:5) (50.50 to 30:70) to yield the title compound as a light brown foam (5.38 g, 88 %).
MS ISP (m/e): 377.4 (100) [(M+H)⁺].

### b) Ethyl 5-(1-(1-benzylpyrrolidin-3-yl)vinyl)-1H-indole-2-carboxylate

To a solution of potassium *tert*-butoxide (3.20 g, 28.5 mmol) in THF (70 mL) was added under an atmosphere of nitrogen at 0°C methyltriphenylphosphonium bromide (11.2 g, 31.4 mmol).

After stirring for 30 minutes at 0°C a solution of ethyl 5-(1-benzylpyrrolidine-3-carbonyl)-1H-indole-2-carboxylate (5.37 g, 14.3 mmol) in THF (10 mL) was added during 5 minutes. The reaction was stirred for 18 hours in a thawing ice bath. To a solution of potassium *tert*-butoxide (3.20 g, 28.5 mmol) in THF (70 mL) was added under an atmosphere of nitrogen at 0°C methyltriphenylphosphonium bromide (11.2 g, 31.4 mmol). After stirring for 30 minutes the yellow suspension was added to the upper reaction at 0°C and was stirred for 2 hours at 0°C and 2 hours at room temperature. After cooling to 0°C a 1 M aqueous solution of sodium carbonate (100 mL) was added carefully. The reaction was diluted with ethyl acetate (50 mL). The aqueous layer was extracted with ethyl acetate (50 mL). The organic layers were washed with water (100 mL) and brine (50 mL). The combined organic layers were dried over sodium sulfate. The concentrated filtered solution was purified by flash chromatography on silica gel using heptane /ethyl acetate / (ethyl acetate: NEt₃ 95:5) (90:10:0 to 0:50:50) to yield the title compound as a light yellow oil (4.31 g, 81 %).
MS ISP (m/e): 375.3 (100) [(M+H)⁺].

### c) 5-(1-(1-Benzylpyrrolidin-3-yl)vinyl)-N-(3,5-difluorophenyl)-1H-indole-2-carboxamide

The title compound was prepared in the same manner as described in example 20 step e) but at 100°C from ethyl 5-(1-(1-benzylpyrrolidin-3-yl)vinyl)-1H-indole-2-carboxylate and 3,5-difluoranilin as an off-white foam (55 %).
MS ISP (m/e): 458.3 (100) [(M+H)⁺].

### Example 92

### N-(3,5-Difluorophenyl)-5-(1-(pyrrolidin-3-yl)ethyl)-1H-indole-2-carboxamide

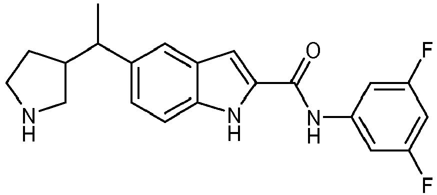

To a suspension of 5-(1-(1-benzylpyrrolidin-3-yl)vinyl)-N-(3,5-difluorophenyl)-1H-indole-2-carboxamide (2.90 g, 6.34 mmol) in methanol (21 mL) and THF (4.2 mL) was added under an atmosphere of argon palladium on carbon (675 mg, 6.34 mmol) and ammonium formate (4.00 g, 63.4 mmol). The reaction was stirred for 4 hours at room temperature, filtered over Hyflo and was washed well with methanol. The concentrated filtrate was purified by column chromatography on silica gel (basic) using heptane / ethyl acetate / dichloromethane / methanol (20:80:0:0 to 0:85:10:5) to yield the title compound as a white foam (1.8 g, 77 %).
MS ISP (m/e): 370.2 (100) [(M+H)⁺].

### Example 93

### N-(3,5-Difluorophenyl)-5-(1-(1-isopropylpyrrolidin-3-yl)ethyl)-1H-indole-2-carboxamide

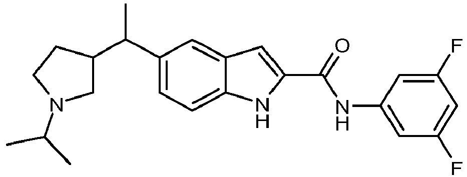

The title compound was prepared in the same manner as described in example 76 from N-(3,5-difluorophenyl)-5-(1-(pyrrolidin-3-yl)ethyl)-1H-indole-2-carboxamide and isopropyl bromide using NMP as solvent as a white solid (82 %).
MS ISP (m/e): 412.4 (100) [(M+H)⁺].

### Example 94

### 5-(1-(1-(Cyanomethyl)pyrrolidin-3-yl)ethyl)-N-(3,5-difluorophenyl)-1H-indole-2-carboxamide

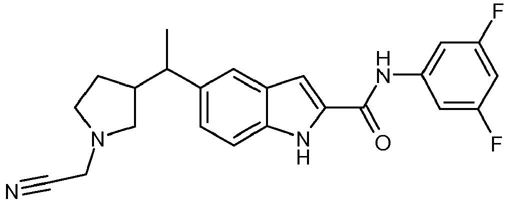

The title compound was prepared in the same manner as described in example 76 from N-(3,5-difluorophenyl)-5-(1-(pyrrolidin-3-yl)ethyl)-1H-indole-2-carboxamide and bromo acetonitril using NMP as solvent as a white solid (50 %).
MS ISP (m/e): 409.2 (100) [(M+H)⁺].

### Example 95

### 5-(1-(1-Acetylpyrrolidin-3-yl)ethyl)-N-(3,5-difluorophenyl)-1H-indole-2-carboxamide

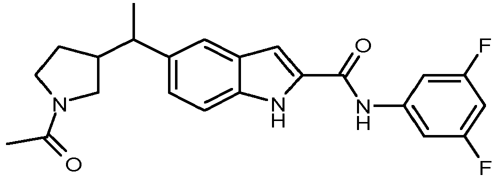

The title compound was prepared in the same manner as described in example 10 from N-(3,5-difluorophenyl)-5-(1-(pyrrolidin-3-yl)ethyl)-1H-indole-2-carboxamide and acetyl chloride as a white solid (15 %).
MS EI (m/e): 411.17 (100) [(M)⁺].

### Example 96

### N-(3,5-Difluorophenyl)-5-(1-(1-(2,2,2-trifluoroethyl)pyrrolidin-3-yl)ethyl)-1H-indole-2-carboxamide

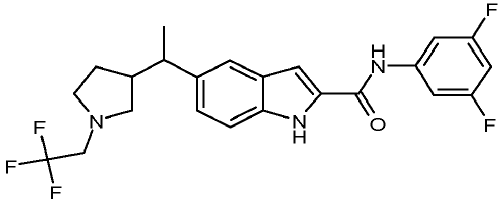

The title compound was prepared in the same manner as described in example 6 from N-(3,5-difluorophenyl)-5-(1-(pyrrolidin-3-yl)ethyl)-1H-indole-2-carboxamide and 2,2,2-trifluoroethyl trifluoromethanesulfonate as a white solid (69 %).
MS ISP (m/e): 452.2 (100) [(M+H)⁺].

### Example 97

### N-(3,5-Difluorophenyl)-5-(4-(piperidin-1-yl)but-1-en-2-yl)-1H-indole-2-carboxamide

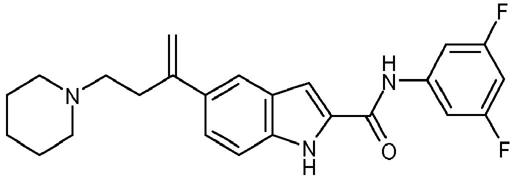

### a) Ethyl 5-(3-(piperidin-1-yl)propanoyl)-1H-indole-2-carboxylate

To a solution of 3-(piperidin-1-yl)propanoic acid (1.86 g, 11.8 mmol) in dichloromethane (20 mL) was added at 0°C drop wise during 5 minutes thionyl chloride (2.81 g, 1.73 mL, 23.6 mmol). The reaction was stirred for 18 hours at room temperature and the solvent was removed. The crude acid chloride was used directly in the next step.

The title compound was prepared in the same manner as described in example 91 step a) from ethyl 5-(3-(piperidin-1-yl)propanoyl)-1H-indole-2-carboxylate as a light brown solid (29 %). MS ISP (m/e): 329.4 (100) [(M+H)⁺].

### b) Ethyl 5-(4-(piperidin-1-yl)but-1-en-2-yl)-1H-indole-2-carboxylate

The title compound was prepared in the same manner as described in example 91 step b) from ethyl 5-(3-(piperidin-1-yl)propanoyl)-1H-indole-2-carboxylate as an off-white solid (44 %).
MS ISP (m/e): 375.3 (100) [(M+H)⁺].

### c) N-(3,5-Difluorophenyl)-5-(4-(piperidin-1-yl)but-1-en-2-yl)-1H-indole-2-carboxamide

The title compound was prepared in the same manner as described in example 20 step e) but at 100°C from ethyl 5-(4-(piperidin-1-yl)but-1-en-2-yl)-1H-indole-2-carboxylate and 3,5-difluoranilin as a light yellow foam (84 %).
MS ISP (m/e): 410.3 (100) [(M+H)⁺].

### Example 98

### N-(3,5-Difluorophenyl)-5-(4-(piperidin-1-yl)butan-2-yl)-1H-indole-2-carboxamide

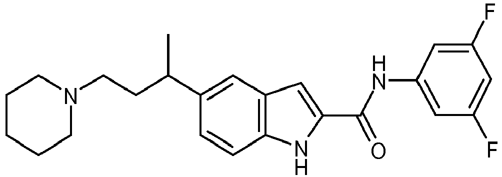

The title compound was prepared in the same manner as described in example 92 from N-(3,5-difluorophenyl)-5-(4-(piperidin-1-yl)but-1-en-2-yl)-1H-indole-2-carboxamide as a white foam (39 %).
MS ISP (m/e): 412.2 (100) [(M+H)⁺].

### Example 99

### N-(3-Fluorophenyl)-5-(1-(pyrrolidin-1-yl)propan-2-yl)-1H-indole-2-carboxamide

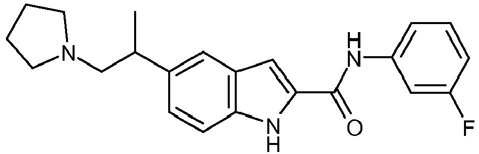

### a) Ethyl 5-(2-(pyrrolidin-1-yl)acetyl)-1H-indole-2-carboxylate

To a solution of ethyl 5-(2-chloroacetyl)-1H-indole-2-carboxylate (700 mg, 2.63 mmol, CAS: 90395-35-0) in THF (7 mL) and N-methyl-2-pyrrolidinone (1 mL) was added under an atmosphere of nitrogen potassium carbonate (546 mg, 3.95 mmol) and during 2 minutes pyrrolidine (375 mg, 436 µL, 5.27 mmol). The suspension was stirred for several hours at room temperature. The reaction was diluted with ethyl acetate (15 mL) and washed with a 1 M aqueous solution of Na₂CO₃ (10 mL), water (10 mL) and brine (10 mL). The aqueous layers were extracted with ethyl acetate (15 mL). The combined organic layers were dried over sodium sulfate. The concentrated solid was suspended in ethyl acetate (3 mL) filtered off and was washed with ethyl acetate (3 mL) to yield the title compound as a light brown solid (622 mg, 79 %).
MS ISN (m/e): 299.3 (100) [(M-H)⁻].

### b) Ethyl 5-(3-(pyrrolidin-1-yl)prop-1-en-2-yl)-1H-indole-2-carboxylate

The title compound was prepared in the same manner as described in example 91 step b) from ethyl 5-(2-(pyrrolidin-1-yl)acetyl)-1H-indole-2-carboxylate as a white solid (95 %).
MS ISP (m/e): 299.2 (100) [(M+H)⁺].

### c) Ethyl 5-(1-(pyrrolidin-1-yl)propan-2-yl)-1H-indole-2-carboxylate

The title compound was prepared in the same manner as described in example 91 step c) from ethyl 5-(3-(pyrrolidin-1-yl)prop-1-en-2-yl)-1H-indole-2-carboxylate as a white solid (31 %).

### d) N-(3-Fluorophenyl)-5-(1-(pyrrolidin-1-yl)propan-2-yl)-1H-indole-2-carboxamide

The title compound was prepared in the same manner as described in example 20 step e) but at 110°C from ethyl 5-(1-(pyrrolidin-1-yl)propan-2-yl)-1H-indole-2-carboxylate and 3-fluoranilin as a white solid (33 %).
MS ISN (m/e): 364.3 (100) [(M-H)⁻].

### Example 100

### 5-(1-Hydroxy-2-piperidin-1-yl-ethyl)-1H-indole-2-carboxylic acid (3,4-difluoro-phenyl)-amide

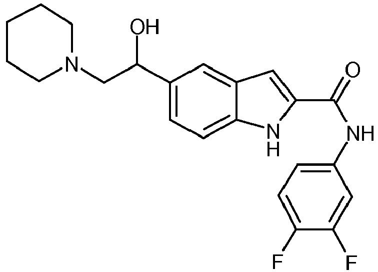

### a) 5-(2-Piperidin-1-yl-acetyl)-1H-indole-2-carboxylic acid ethyl ester

The title compound was prepared in the same manner as described in example 99 step a) from piperidine and ethyl 5-(2-chloroacetyl)-1H-indole-2-carboxylate as light brown crystals solid (88 %).

### b) 5-(1-Hydroxy-2-piperidin-1-yl-ethyl)-1H-indole-2-carboxylic acid ethyl ester

Sodium boronhydride (48 mg) was added to a solution of 5-(2-piperidin-1-yl-acetyl)-1H-indole-2-carboxylic acid ethyl ester (200 mg) in methanol (5 mL). The reaction was stirred at room temperature for 90 minutes and concentrated. The residue was partitioned between ethyl acetate and water. The organic layer was dried over magnesium sulfate, filtered and concentrated. The residue was crystallized from diethyl ether to yield the title compound as white crystals (110 mg, 55 %).
MS ISN (m/e): 315.2 (100) [(M-H)⁻].

### c) 5-(1-Hydroxy-2-piperidin-1-yl-ethyl)-1H-indole-2-carboxylic acid (3,4-difluoro-phenyl)-amide

The title compound was prepared in the same manner as described in example 47 step c) from 5-(1-hydroxy-2-piperidin-1-yl-ethyl)-1H-indole-2-carboxylic acid ethyl ester and 3,4-difluoranilin as a white solid (33 %).
MS ISN (m/e): 398.4 (100) [(M-H)⁻].

### Example 101

### 5-[(E)-2-((1S,4R)-3-Oxo-2-aza-bicyclo[2.2.1]hept-2-yl)-vinyl]-1H-indole-2-carboxylic acid (3,5-difluoro-phenyl)-amide

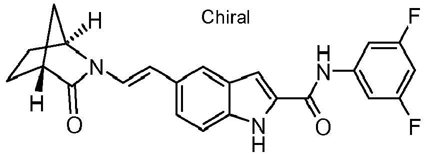

### a) (1S,4R)-2-Vinyl-2-aza-bicyclo[2.2.1]heptan-3-one

(1S,4R)-2-Azabicyclo[2.2.1]heptan-3-one (1.425 g) and butylvinyl ether (12.8 g) were heated to 75°C for 5 hours under argon in the presence of bis-trifluoroaceto-(4,7-diphenyl-1,10-phenanthroline)palladium(II) (300 mg) (in analogy to Org. Lett., Vol 6, No. 11, 2004). The concentrated reaction was purified by column chromatography on silica gel using heptane /ethyl acetate (70:30) to yield the title compound as a yellow oil (761 mg, 43 %).

### b) 5-[(E)-2-((1S,4R)-3-Oxo-2-aza-bicyclo[2.2.1]hept-2-yl)-vinyl]-1H-indole-2-carboxylic acid (3,5-difluoro-phenyl)-amide

To a solution of palladium acetate (16 mg), tri-(o-tolyl)phosphine (72 mg) in DMF (10 mL) was added under argon ethyl 5-bromoindole-2-carboxylate (391 mg), sodium bicarbonate (490 mg), N,N-disiopropylethylamine (754 mg) and (1S,4R)-2-vinyl-2-aza-bicyclo[2.2.1]heptan-3-one (250 mg). The reactio was heated to 120°C for 2 hours. Water was added and the reaction was extracted with ethyl acetate. The combined organic layers were dried over magnesium sulfate, filtered and concentrated. The residue was purified by column chromatography on silica gel using heptane /ethyl acetate (70:30 to 0:100) to yield the title compound as an off-white solid (59 mg, 10 %).

### c) 5-[(E)-2-((1S,4R)-3-Oxo-2-aza-bicyclo[2.2.1]hept-2-yl)-vinyl]-1H-indole-2-carboxylic acid (3,5-difluoro-phenyl)-amide

The title compound was prepared in the same manner as described in example 20 step e) from 5-[(E)-2-((1S,4R)-3-oxo-2-aza-bicyclo[2.2.1]hept-2-yl)-vinyl]-1H-indole-2-carboxylic acid ethyl ester and 3,5-difluoroanilin as a yellow solid (28 %).
MS ISN (m/e): 406.5 (100) [(M-H)⁻].

### Example 102 and 103

### 5-((S)-1-Methyl-pyrrolidin-3-yl)-1H-indole-2-carboxylic acid (3,5-difluoro-phenyl)-amide and 5-((R)-1-Methyl-pyrrolidin-3-yl)-1H-indole-2-carboxylic acid (3,5-difluoro-phenyl)-amide

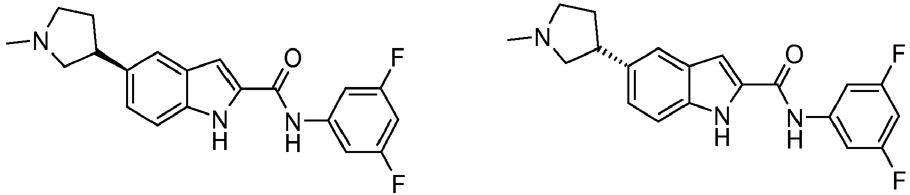

5-(1-Methyl-pyrrolidin-3-yl)-1H-indole-2-carboxylic acid (3,5-difluoro-phenyl)-amide (45 mg) was separated in its enantiomers via chiral HPLC (Chiralpak AD) using 25 % ethanol in heptane as the eluent providing the title compounds as light brown solids as the (+)-enantiomer (59 min.; 12.8 mg, 29 %) and the (-)-enantiomer (82 min.; 14.7 mg, 33 %).
MS ISP (m/e): 356.1 (100) [(M+H)⁺].

The following examples (Table 8) were prepared in analogy to example 100 and 101, by separation via chiral HPLC.

**Table 8**

| **Ex.** | **Racemat** | **Name and Structure** | **MS (m/e)** |
|---|---|---|---|
| 104 | Example 10 | **5-[(S)-1-(2,2,2-Trifluoro-ethyl)-pyrrolidin-3-yl]-1H-indole-2-carboxylic acid (3,5-difluoro-phenyl)-amide** | ISP 424.2 [(M+H)⁺] |
| | | 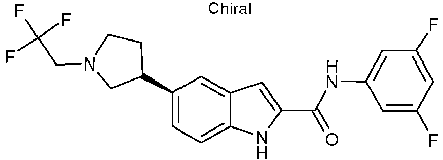 | |
| | | Second eluent (70 min) | |
| 105 | Example 13 | **5-((R)-1-Isobutyryl-pyrrolidin-3-yl)-1H-indole-2-carboxylic acid (3,5-difluorophenyl)-amide** | ISP 412.2 [(M+H)⁺] |
| | | 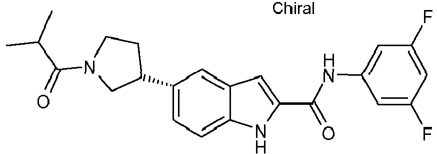 | |
| | | First eluent (67 min) | |
| 106 | Example 13 | **5-((S)-1-Isobutyryl-pyrrolidin-3-yl)-1H-indole-2-carboxylic acid (3,5-difluorophenyl)-amide** | ISP 412.2 [(M+H)⁺] |
| | | 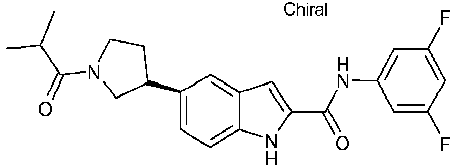 | |
| | | Second eluent (70 min) | |
| 107 | Example 12 | **5-((S)-1-Isobutyryl-pyrrolidin-3-yl)-1H-indole-2-carboxylic acid (3,5-difluorophenyl)-amide** | ISP 448.1 [(M+H)⁺] |
| | | 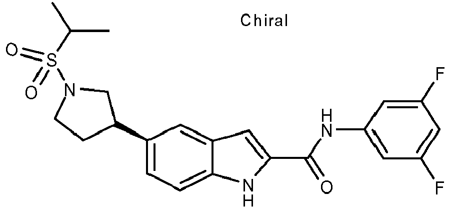 | |
| | | First eluent on LUX2 Amylose with 40% isopropanol in heptane as eluent (20 min) | |
| 108 | Example 16 | **(R)-5-(1-(Cyanomethyl)pyrrolidin-3-yl)-N-(3-fluorophenyl)-1H-indole-2-carboxamide** | ISN 361.3 [(M-H)⁻] |
| | | 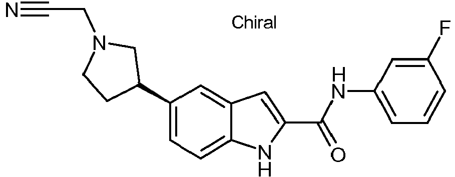 | |
| | | First eluent | |
| 109 | Example 16 | **(S)-5-(1-(Cyanomethyl)pyrrolidin-3-yl)-N-(3-fluorophenyl)-1H-indole-2-carboxamide** | ISP 361.3 [(M-H)⁻] |
| | | 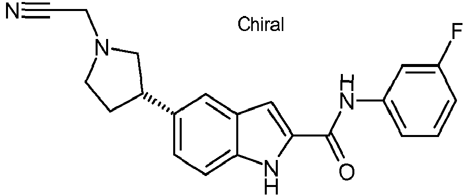 | |
| | | Second eluent | |
| 110 | Example 46 | **5-((R)-1-Isopropyl-piperidin-3-yl)-1H-indole-2-carboxylic acid (3-fluoro-phenyl)-amide** | |
| | | 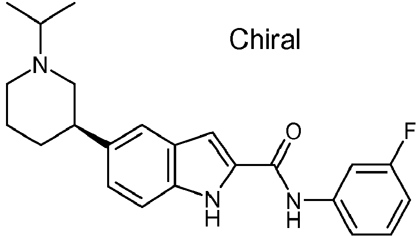 | |
| | | First eluent, optical rotation 24.8 (conc. 0.19 [MeOH]; WL 589, T=20°C) | |
| 111 | Example 46 | **5-((S)-1-Isopropyl-piperidin-3-yl)-1H-indole-2-carboxylic acid (3-fluoro-phenyl)-amide** | |
| | | 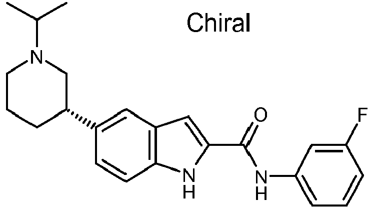 | |
| | | Second eluent, optical rotation -16.2 (conc. 0.26 [MeOH]; WL 589, T=20°C) | |
| 112 | Example 51 | **5-((R)-1-Acetyl-piperidin-3-yl)-1H-indole-2-carboxylic acid (3,5-difluoro-phenyl)-amide** | |
| | | 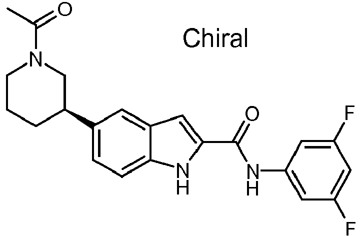 | |
| | | Second eluent, optical rotation 69.5 (conc. 0.46 [CHCl₃]; WL 589, T=20°C) | |
| 113 | Example 51 | **5-((S)-1-Acetyl-piperidin-3-yl)-1H-indole-2-carboxylic acid (3,5-difluoro-phenyl)-amide** | |
| | | 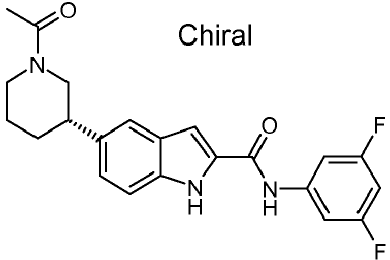 | |
| | | Second eluent, optical rotation -83.8 (conc. 0.49 [MeOH]; WL 589, T=20°C) | |
| 114 | Example 66 | **(R)-N-(3-Fluorophenyl)-5-(1-(2-hydroxyethyl)piperidin-3-yl)-1H-indole-2-carboxamide** | ISP 382.4 [(M+H)⁺] |
| | | 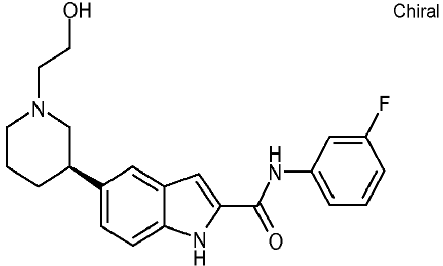 | |
| | | First eluent, (+)-isomer | |
| 115 | Example 66 | **(S)-N-(3-Fluorophenyl)-5-(1-(2-hydroxyethyl)piperidin-3-yl)-1H-indole-2-carboxamide** | ISP 382.4 [(M+H)⁺] |
| | | 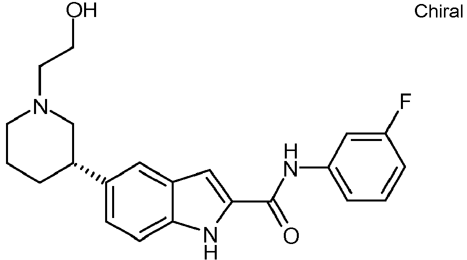 | |
| | | Second eluent, (-)-isomer | |
| 116 | Example 71 | **(R)-5-(1-Cyclopropylpiperidin-3-yl)-N-(3-fluorophenyl)-1H-indole-2-carboxamide** | ISP 378.4 [(M+H)⁺] |
| | | 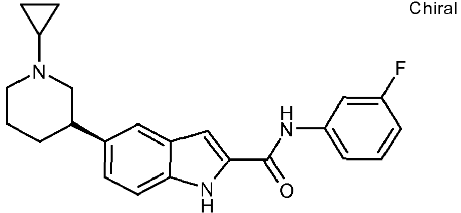 | |
| | | First eluent, (+)-isomer | |
| 117 | Example 71 | **(S)-5-(1-Cyclopropylpiperidin-3-yl)-N-(3-fluorophenyl)-1H-indole-2-carboxamide** | ISP 378.4 [(M+H)⁺] |
| | | 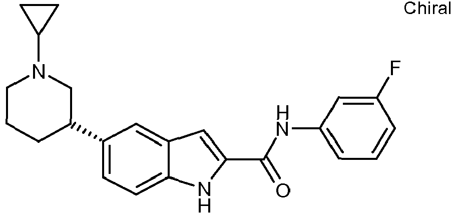 | |
| | | Second eluent, (-)-isomer | |
| 118 | Example 99 | **(S)-N-(3-Fluorophenyl)-5-(1-(pyrrolidin-1-yl)propan-2-yl)-1H-indole-2-carboxamide** | ISP 365.2 [(M+H)⁺] |
| | | 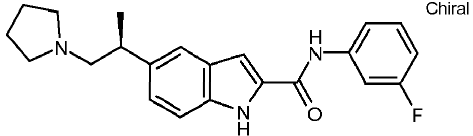 | |
| | | First eluent, 76 min | |
| 119 | Example 99 | **(R)-N-(3-Fluorophenyl)-5-(1-(pyrrolidin-1-yl)propan-2-yl)-1H-indole-2-carboxamide** | ISP 365.2 [(M+H)⁺] |
| | | 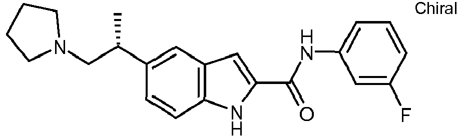 | |
| | | Second eluent, 90 min | |
| 120 | Example 96 | **N-(3,5-Difluorophenyl)-5-((R)-1-((S)-1-(2,2,2-trifluoroethyl)pyrrolidin-3-yl)ethyl)-1H-indole-2-carboxamide** | ISN 450.2 [(M-H)⁻] |
| | | 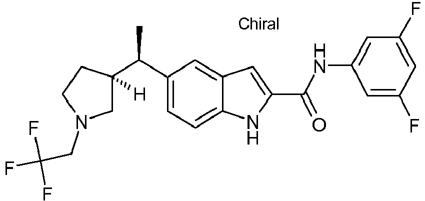 | |
| | | First eluent, 76 min | |
| 121 | Example 96 | **N-(3,5-Difluorophenyl)-5-((R)-1-((R)-1-(2,2,2-trifluoroethyl)pyrrolidin-3-yl)ethyl)-1H-indole-2-carboxamide** | ISN 450.2 [(M-H)⁻] |
| | | 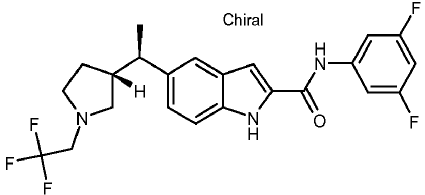 | |
| | | Second eluent, 90 min | |
| 122 | Example 95 | **5-((S)-1-((S)-1-Acetylpyrrolidin-3-yl)ethyl)-N-(3,5-difluorophenyl)-1H-indole-2-carboxamide** | ISP 412.2 [(M+H)⁺] |
| | | 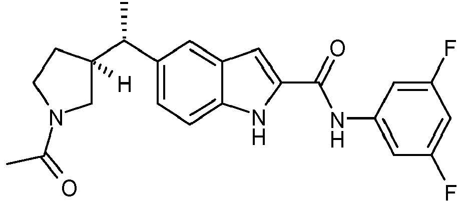 | |
| | | Second eluent | |

### Cellular γ-secretase assay

The compounds were investigated in accordance with the test given hereinafter.

Human neuroglioma H4 cells overexpressing human APP were plated at 30,000 cells/well/200 µL in 96-well plates in IMDM media containing 10% FCS, 0.2 mg/L Hygromycin B and incubated for 2 hours at 37 °C, 5% CO₂ prior to adding test compounds.

Compounds for testing were dissolved in 100% Me₂SO yielding in a 10 mM stock solution. Typically 12 µL of these solutions were further diluted in 1000 µL of IMDM media (w/o FCS). Subsequent 1:1 dilutions gave a ten point dose response curve. 100 µL of each dilution was added to the cells in 96-well plates. Appropriate controls using vehicle only and reference compound were applied to this assay. The final concentration of Me2SO was 0.4%.

After incubation for 22 hours at 37 °C, 5% CO₂, 50 µL supernatant was transferred into round-bottom 96-well polypropylene plates for detection of Aβ42. 50 µL assay buffer (50 mM Tris/Cl, pH 7.4, 60 mM NaCl, 0.5% BSA, 1% TWEEN 20) was added to the wells followed by the addition of 100 µL of detection antibody (ruthenylated BAP15 0.0625 µg/mL in assay buffer). 50 µL of a premix of capture antibody (biotinylated 6E10 antibody, 1 µg/mL) and Streptavidin-coated magnetic beads (Dynal M-280, 0.125 mg/mL) were preincubated for 1 hour at room temperature before adding the assay plates. Assay plates were incubated on a shaker for 3 hours at room temperature and finally read in the Bioveris M8 Analyser according to the manufacturer's instructions (Bioveris).

Toxicity of compounds was monitored by a cell viability test of the compound-treated cells using a colorimetric assay (CellTiter 96TM AQ assay, Promega) according to the manufacturer's instructions. Briefly, after removal of 50 µL cell culture supernatant for detection of Aβ42, 20 µL of 1x MTS/PES solution was added to the cells and incubated for 30 minutes at 37 °C, 5% CO₂. Optical density was then recorded at 490 nm.

IC50 values for inhibition of Aβ42 secretion were calculated by nonlinear regression fit analysis using XLfit 4.0 software (IDBS).

Particular compounds of present invention show an EC50 Aβ42 < 1000nM. Most particular compounds of present invention show an EC50 Aβ42 < 200nM. In In the list below (table 9) are described the data for all compounds to the inhibition of Aβ42 secretion:

**Table 9**

| **Example** | **EC₅₀ Aβ42 (nM)** | | **Example** | **EC₅₀ Aβ42 (nM)** | | **Example** | **EC₅₀ Aβ42 (nM)** |
|---|---|---|---|---|---|---|---|
| 1 | 795 | | 11 | 321 | | 21 | 177 |
| 2 | 178 | | 12 | 1711 | | 22 | 2266 |
| 3 | 1235 | | 13 | 713 | | 23 | 897 |
| 4 | 402 | | 14 | 519 | | 24 | 19357 |
| 5 | 631 | | 15 | 1245 | | 25 | 2677 |
| 6 | 1421 | | 16 | 373 | | 26 | 10857 |
| 7 | 253 | | 17 | 8457 | | 27 | 8357 |
| 8 | 627 | | 18 | 2319 | | 28 | 852 |
| 9 | 1270 | | 19 | 2628 | | 29 | 12597 |
| 10 | 3831 | | 20 | 832 | | 30 | 742 |
| | | | | | | | |
| 31 | 2380 | | 41 | 1908 | | 51 | 263 |
| 32 | 8149 | | 42 | 1908 | | 52 | 273 |
| 33 | 1583 | | 43 | 2473 | | 53 | 392 |
| 34 | 926 | | 44 | 4382 | | 54 | 403 |
| 35 | 1389 | | 45 | 12928 | | 55 | 449 |
| 36 | 1090 | | 46 | 588 | | 56 | 466 |
| 37 | 1432 | | 47 | 1152 | | 57 | 668 |
| 38 | 1289 | | 48 | 2506 | | 58 | 744 |
| 39 | 1325 | | 49 | 148 | | 59 | 183 |
| 40 | 1696 | | 50 | 202 | | 60 | 1379 |
| | | | | | | | |
| 61 | 3443 | | 71 | 977 | | 81 | 4249 |
| 62 | 354 | | 72 | 1112 | | 82 | 1932 |
| 63 | 614 | | 73 | 9287 | | 83 | 3339 |
| 64 | 2471 | | 74 | 1861 | | 84 | 4859 |
| 65 | 2946 | | 75 | 1312 | | 85 | 445 |
| 66 | 727 | | 76 | 796 | | 86 | 990 |
| 67 | 657 | | 77 | 3761 | | 87 | 1295 |
| 68 | 777 | | 78 | 927 | | 88 | 321 |
| 69 | 662 | | 79 | 981 | | 89 | 4405 |
| 70 | 810 | | 80 | 942 | | 90 | 877 |
| | | | | | | | |
| 91 | 1492 | | 101 | 3828 | | 111 | 876 |
| 92 | 1180 | | 102 | 300 | | 112 | 226 |
| 93 | 1188 | | 103 | 374 | | 113 | 1794 |
| 94 | 1340 | | 104 | 528 | | 114 | 273 |
| 95 | 2485 | | 105 | 1208 | | 115 | 1501 |
| 96 | 7717 | | 106 | 889 | | 116 | 545 |
| 97 | 1259 | | 107 | 628 | | 117 | 4362 |
| 98 | 1039 | | 108 | 646 | | 118 | 753 |
| 99 | 1245 | | 109 | 472 | | 119 | 1733 |
| 100 | 2614 | | 110 | 116 | | 120 | 7423 |
| | | | | | | | |
| 121 | 7479 | | 122 | 2072 | | | |

## Claims

1. Compounds of formula (I) 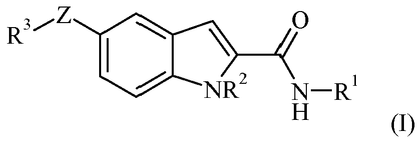 wherein
Z is a bond, alkylene, or alkenylene, wherein alkylene and alkenylene are optionally substituted with one, two or three substituents selected independently from the list of halo, cyano, hydroxy, and alkoxy;
R¹ is aryl or heteroaryl, wherein each is optionally substituted with one, two or three substituents selected independently from the list of halo, cyano, alkyl, haloalkyl, hydroxyalkyl, alkoxy and haloalkoxy;
R² is hydrogen, alkyl, haloalkyl, cyanoalkyl, or hydroxyalkyl;
R³ is saturated or partially unsaturated heterocycloalkyl which is substituted with one, two, three or four R⁴;
R⁴ is hydrogen, alkyl, alkenyl, alkynyl, cycloalkyl, heterocycloalkyl, aryl, heteroaryl, hydroxy, alkoxy, oxo, -C(O)-R⁵, or -S(O)₂-R⁶; wherein alkyl, alkenyl, alkynyl, cycloalkyl, heterocycloalkyl, aryl, heteroaryl, and alkoxy are optionally substituted by one, two or three R⁷;
R⁵ is hydrogen, alkyl, haloalkyl, cyanoalkyl, hydroxyalkyl, alkoxyalkyl, cycloalkyl, aryl, hydroxy, or alkoxy;
R⁶ is hydrogen, alkyl, haloalkyl, cyanoalkyl, hydroxyalkyl, alkoxyalkyl, cycloalkyl, aryl, haloaryl, heteroaryl or alkylheteroaryl;
R⁷ is halo, haloalkyl, cyano, hydroxy, alkoxy, cycloalkyl, heterocycloalkyl, aryl or -C(O)O-alkyl;
and pharmaceutically acceptable salts and esters thereof.

2. Compounds of formula (I) according to claim 1, wherein Z is a bond, -CH(CH₃)-, or-C(=CH₂)-.

3. Compounds of formula (I) according to any of claims 1 to 2, wherein Z is a bond.

4. Compounds of formula (I) according to any of claims 1 to 3, wherein R¹ is aryl or heteroaryl, wherein each is substituted with one or two substituents selected independently from the list of halo, cyano, alkyl, haloalkyl, and alkoxy.

5. Compounds of formula (I) according to any of claims 1 to 4, wherein R¹ is phenyl or pyridinyl, wherein each is substituted with one or two substituents selected independently from the list of halo, cyano, alkyl, haloalkyl, and alkoxy.

6. Compounds of formula (I) according to any of claims 1 to 5, wherein R¹ is 3-fluorophenyl or 3,5-difluorophenyl.

7. Compounds of formula (I) according to any of claims 1 to 6, wherein R² is hydrogen.

8. Compounds of formula (I) according to any of claims 1 to 7, wherein R³ is a saturated or partly unsaturated mono- or bicyclic ring system of 5 to 7 ring atoms, comprising 1 or 2 ring heteroatoms selected from N and O the remaining ring atoms being carbon, which is substituted by one, two, three or four R⁴.

9. Compounds of formula (I) according to any of claims 1 to 8, wherein R³ is a saturated monocyclic ring system of 5 or 6 ring atoms, comprising one nitrogen ring heteroatom the remaining ring atoms being carbon, which is substituted by one R⁴.

10. Compounds of formula (I) according to any of claims 1 to 8, wherein R³ is pyrrolidinyl, piperidinyl, tetrahydropyridinyl, tetrahydropyranyl, or dihydropyranyl, wherein each is substituted by one, two, three or four R⁴.

11. Compounds of formula (I) according to any of claims 1 to 10, wherein R³ is pyrrolidinyl substituted by one R⁴ or piperidinyl substituted by one R⁴.

12. Compounds of formula (I) according to any of claims 1 to 11, wherein R⁴ is hydrogen, alkyl, alkenyl, cycloalkyl, heteroaryl, oxo, -C(O)-R⁵, or -S(O)₂-R⁶; wherein alkyl, alkenyl, cycloalkyl, and heteroaryl are optionally substituted by one, two or three R⁷.

13. Compounds of formula (I) according to any of claims 1 to 12, wherein R⁴ is hydrogen, alkyl, -C(O)-R⁵, or -S(O)₂-R⁶; wherein alkyl is optionally substituted by one R⁷.

14. Compounds of formula (I) according to any of claims 1 to 13, wherein R⁴ is hydrogen, methyl, cyanomethyl, ethyl, hydroxyethyl, isopropyl, -C(O)-methyl, -C(O)-isopropyl, -C(O)-cyclopropyl, -C(O)-phenyl, -S(O)₂-methyl, -S(O)₂-isopropyl, -S(O)₂-cyclopropyl, or -S(O)₂-phenyl.

15. Compounds of formula (I) according to any of claims 1 to 14, wherein R⁵ is alkyl, alkoxyalkyl, cycloalkyl, aryl, or alkoxy.

16. Compounds of formula (I) according to any of claims 1 to 15, wherein R⁵ is methyl, ethyl, isopropyl, methoxymethyl, cyclopropyl, phenyl, methoxy, isopropoxy.

17. Compounds of formula (I) according to any of claims 1 to 16, wherein R⁶ is alkyl, cycloalkyl, aryl, haloaryl, heteroaryl, or alkylheteroaryl.

18. Compounds of formula (I) according to any of claims 1 to 17, wherein R⁶ is methyl, isopropyl, cyclopropyl, phenyl, fluoro-phenyl, or methyl-imidazolyl.

19. Compounds of formula (I) according to any of claims 1 to 18, wherein R⁷ is haloalkyl, cyano, hydroxy, alkoxy, cycloalkyl, heterocycloalkyl, aryl or -C(O)O-alkyl.

20. Compounds of formula (I) according to any of claims 1 to 19, wherein R⁷ is trifluoromethyl, cyano, hydroxy, methoxy, cyclopropyl, tetrahydrofuranyl, phenyl or -C(O)O-methyl.

21. Compounds of formula (I) according to any of claims 1 - 20, selected from the group consisting of:
5-((1-Benzylpyrrolidin-3-yl)-N-(3,5-difluorophenyl)-1H-indole-2-carboxamide;
5-((1-Methyl-pyrrolidin-3-yl)-1H-indole-2-carboxylic acid (3,5-difluoro-phenyl)-amide;
5-((1-Benzylpyrrolidin-3-yl)-N-(3-fluorophenyl)-1H-indole-2-carboxamide;
N-(3-Fluorophenyl)-5-(1-methylpyrrolidin-3-yl)-1H-indole-2-carboxamide;
5-((1-Benzylpyrrolidin-3-yl)-N-(3-cyano-5-fluorophenyl)-1H-indole-2-carboxamide;
N-(3-Cyano-5-fluorophenyl)-5-(1-methylpyrrolidin-3-yl)-1H-indole-2-carboxamide;
5-Pyrrolidin-3-yl-1H-indole-2-carboxylic acid (3,5-difluoro-phenyl)-amide;
N-(3-Fluorophenyl)-5-(pyrrolidin-3-yl)-1H-indole-2-carboxamide;
N-(3-cyano-5-fluorophenyl)-5-(pyrrolidin-3-yl)-1H-indole-2-carboxamide;
5-[1-(2,2,2-Trifluoro-ethyl)-pyrrolidin-3-yl]-1H-indole-2-carboxylic acid (3,5-difluorophenyl)-amide;
5-((1-Isopropyl-pyrrolidin-3-yl)-1H-indole-2-carboxylic acid (3,5-difluoro-phenyl)-amide;
5-[1-(Propane-2-sulfonyl)-pyrrolidin-3-yl]-1H-indole-2-carboxylic acid (3,5-difluorophenyl)-amide;
5-((1-Isobutyryl-pyrrolidin-3-yl)-1H-indole-2-carboxylic acid (3,5-difluoro-phenyl)-amide;
N-(3-Fluorophenyl)-5-(1-isopropylpyrrolidin-3-yl)-1H-indole-2-carboxamide;
N-(3-Fluorophenyl)-5-(1-(2,2,2-trifluoroethyl)pyrrolidin-3-yl)-1H-indole-2-carboxamide;
5-((1-(Cyanomethyl)pyrrolidin-3-yl)-N-(3-fluorophenyl)-1H-indole-2-carboxamide;
5-((1-Benzyl-2,5-dioxopyrrolidin-3-yl)-N-(3-fluorophenyl)-1H-indole-2-carboxamide;
5-((3-Isopropyl-5-oxo-pyrrolidin-3-yl)-1H-indole-2-carboxylic acid (3,4-difluoro-phenyl)-amide;
5-(3-Ethyl-2,6-dioxo-piperidin-3-yl)-1H-indole-2-carboxylic acid (3,5-difluoro-phenyl)-amide;
5-((1-Isopropyl-piperidin-4-yl)-1H-indole-2-carboxylic acid (3-fluoro-phenyl)-amide;
5-((1-Isopropyl-piperidin-3-yl)-1H-indole-2-carboxylic acid (3,5-difluoro-phenyl)-amide;
5-((1-Isopropyl-piperidin-4-yl)-1H-indole-2-carboxylic acid (3-trifluoromethyl-phenyl)-amide;
5-((1-Isopropyl-piperidin-4-yl)-1H-indole-2-carboxylic acid (3-chloro-phenyl)-amide;
5-((1-Isopropyl-piperidin-4-yl)-1H-indole-2-carboxylic acid (2-fluoro-phenyl)-amide;
5-((1-Isopropyl-piperidin-4-yl)-1H-indole-2-carboxylic acid (3-methoxy-phenyl)-amide;
5-((1-Isopropyl-piperidin-4-yl)-1H-indole-2-carboxylic acid (4-methoxy-phenyl)-amide;
5-((1-Isopropyl-piperidin-4-yl)-1H-indole-2-carboxylic acid (4-tert-butyl-phenyl)-amide;
5-((1-Isopropyl-piperidin-4-yl)-1H-indole-2-carboxylic acid (3,4-difluoro-phenyl)-amide;
5-((1-Isopropyl-piperidin-4-yl)-1H-indole-2-carboxylic acid (2,4-difluoro-phenyl)-amide;
N-(3-Chloro-5-fluorophenyl)-5-(1-isopropylpiperidin-3-yl)-1H-indole-2-carboxamide;
5-((1-Isopropyl-piperidin-3-yl)-1H-indole-2-carboxylic acid (3-cyano-5-fluoro-phenyl)-amide;
5-((1-Isopropyl-piperidin-4-yl)-1H-indole-2-carboxylic acid (6-fluoro-pyridin-3-yl)-amide;
5-((1-Isopropyl-piperidin-3-yl)-1H-indole-2-carboxylic acid m-tolylamide;
N-(3-Fluoro-5-methylphenyl)-5-(1-isopropylpiperidin-3-yl)-1H-indole-2-carboxamide;
5-((1-Benzoyl-piperidin-4-yl)-1H-indole-2-carboxylic acid (4-fluoro-phenyl)-amide;
5-((1-Methyl-piperidin-4-yl)-1H-indole-2-carboxylic acid (4-fluoro-phenyl)-amide;
5-[1-(2-Methoxy-ethyl)-piperidin-4-yl]-1H-indole-2-carboxylic acid (4-fluoro-phenyl)-amide;
5-((1-Benzyl-piperidin-4-yl)-1H-indole-2-carboxylic acid (4-fluoro-phenyl)-amide;
5-[1-Allyl-piperidin-4-yl]-1H-indole-2-carboxylic acid (4-fluoro-phenyl)-amide;
5-[1-Propyl-piperidin-4-yl]-1H-indole-2-carboxylic acid (4-fluoro-phenyl)-amide;
4-[2-(4-Fluoro-phenylcarbamoyl)-1H-indol-5-yl]-piperidine-1-carboxylic acid methyl ester;
5-[1-Ethyl-piperidin-4-yl]-1H-indole-2-carboxylic acid (4-fluoro-phenyl)-amide;
{4-[2-(4-Fluoro-phenylcarbamoyl)-1H-indol-5-yl]-piperidin-1-yl}-acetic acid methyl ester;
5-((1-Propionyl-piperidin-4-yl)-1H-indole-2-carboxylic acid (4-fluoro-phenyl)-amide;
5-((1-Phenethyl-piperidin-4-yl)-1H-indole-2-carboxylic acid (4-fluoro-phenyl)-amide;
5-((1-Isopropyl-piperidin-3-yl)-1H-indole-2-carboxylic acid (3-fluoro-phenyl)-amide;
5-((1-Isopropyl-piperidin-3-yl)-1H-indole-2-carboxylic acid (4-fluoro-phenyl)-amide;
5-((1-Isopropyl-piperidin-4-yl)-1H-indole-2-carboxylic acid (4-fluoro-phenyl)-amide;
5-((1-Methanesulfonyl-piperidin-3-yl)-1H-indole-2-carboxylic acid (3,5-difluoro-phenyl)-amide;
5-[1-(Propane-2-sulfonyl)-piperidin-3-yl]-1H-indole-2-carboxylic acid (3,5-difluorophenyl)-amide;
5-(1-Isobutyryl-piperidin-3-yl)-1H-indole-2-carboxylic acid (3,5-difluoro-phenyl)-amide;
5-((1-Acetyl-piperidin-3-yl)-1H-indole-2-carboxylic acid (3,5-difluoro-phenyl)-amide;
5-((1-Benzenesulfonyl-piperidin-3-yl)-1H-indole-2-carboxylic acid (3,5-difluoro-phenyl)-amide;
5-((1-Cyclopropanecarbonyl-piperidin-3-yl)-1H-indole-2-carboxylic acid (3,5-difluorophenyl)-amide;
5-((1-Benzoyl-piperidin-3-yl)-1H-indole-2-carboxylic acid (3,5-difluoro-phenyl)-amide;
5-((1-Cyclopropanesulfonyl-piperidin-3-yl)-1H-indole-2-carboxylic acid (3,5-difluorophenyl)-amide;
5-[1-(2-Methoxy-acetyl)-piperidin-3-yl]-1H-indole-2-carboxylic acid (3,5-difluorophenyl)-amide;
5-[1-(1-Methyl-1H-imidazole-4-sulfonyl)-piperidin-3-yl]-1H-indole-2-carboxylic acid (3,5-difluoro-phenyl)-amide;
5-((1-Methyl-piperidin-3-yl)-1H-indole-2-carboxylic acid (3,5-difluoro-phenyl)-amide;
5-[1-(2-Methoxy-ethyl)-piperidin-3-yl]-1H-indole-2-carboxylic acid (3,5-difluoro-phenyl)-amide;
{3-[2-(3,5-Difluoro-phenylcarbamoyl)-1H-indol-5-yl]-piperidin-1-yl}-acetic acid methyl ester;
5-Piperidin-3-yl-1H-indole-2-carboxylic acid (3,5-difluoro-phenyl)-amide;
5-((1-Isopropyl-piperidin-4-ylamino)-1H-indole-2-carboxylic acid (3,4-difluoro-phenyl)-amide;
N-(3-Fluorophenyl)-5-(1-(2-methoxyethyl)piperidin-3-yl)-1H-indole-2-carboxamide;
N-(3-Fluorophenyl)-5-(1-(2-hydroxyethyl)piperidin-3-yl)-1H-indole-2-carboxamide;
N-(3-Fluorophenyl)-5-(1-((tetrahydrofuran-2-yl)methyl)piperidin-3-yl)-10H-indole-2-carboxamide;
N-(3-Fluorophenyl)-5-(piperidin-3-yl)-1H-indole-2-carboxamide;
5-((1-Ethylpiperidin-3-yl)-N-(3-fluorophenyl)-1H-indole-2-carboxamide;
5-((1-(Cyclopropylmethyl)piperidin-3-yl)-N-(3-fluorophenyl)-1H-indole-2-carboxamide;
5-((1-Cyclopropylpiperidin-3-yl)-N-(3-fluorophenyl)-1H-indole-2-carboxamide;
N-(3-Fluorophenyl)-5-(1-(2,2,2-trifluoroethyl)piperidin-3-yl)-1H-indole-2-carboxamide;
5-((1-Benzyl-pyrrolidin-3-yl)-1H-indole-2-carboxylic acid (5-fluoro-pyridin-3-yl)-amide;
N-(5-Fluoropyridin-3-yl)-5-(pyrrolidin-3-yl)-1H-indole-2-carboxamide;
N-(5-Fluoropyridin-3-yl)-5-(1-isopropylpyrrolidin-3-yl)-1H-indole-2-carboxamide;
Isopropyl 3-(2-(5-fluoropyridin-3-ylcarbamoyl)-1H-indol-5-yl)pyrrolidine-1-carboxylate;
5-((1-(But-3-enyl)pyrrolidin-3-yl)-N-(5-fluoropyridin-3-yl)-1H-indole-2-carboxamide;
5-((1-(Cyclopropylmethyl)pyrrolidin-3-yl)-N-(5-fluoropyridin-3-yl)-1H-indole-2-carboxamide;
5-(1-Cyanomethyl-pyrrolidin-3-yl)-1H-indole-2-carboxylic acid (5-fluoro-pyridin-3-yl)-amide;
1-Cyanomethyl-5-(1-cyanomethyl-pyrrolidin-3-yl)-1H-indole-2-carboxylic acid (5-fluoro-pyridin-3-yl)-amide;
N-(5-Fluoropyridin-3-yl)-5-(1-(2-methoxyacetyl)pyrrolidin-3-yl)-1H-indole-2-carboxamide;
5-((1-(6-Cyanopyridazin-3-yl)pyrrolidin-3-yl)-N-(5-fluoropyridin-3-yl)-1H-indole-2-carboxamide;
5-((1-(4-Fluorophenylsulfonyl)pyrrolidin-3-yl)-N-(5-fluoropyridin-3-yl)-1H-indole-2-carboxamide;
5-((5,6-Dihydro-4H-pyran-3-yl)-1H-indole-2-carboxylic acid (3,4-difluoro-phenyl)-amide;
5-((Tetrahydro-pyran-3-yl)-1H-indole-2-carboxylic acid (3,4-difluoro-phenyl)-amide;
5-((1-Isopropyl-1,2,3,6-tetrahydro-pyridin-4-yl)-1H-indole-2-carboxylic acid (3,5-difluorophenyl)-amide;
5-((1-(1-Benzylpyrrolidin-3-yl)vinyl)-N-(3,5-difluorophenyl)-1H-indole-2-carboxamide;
N-(3,5-Difluorophenyl)-5-(1-(pyrrolidin-3-yl)ethyl)-1H-indole-2-carboxamide;
N-(3,5-Difluorophenyl)-5-(1-(1-isopropylpyrrolidin-3-yl)ethyl)-1H-indole-2-carboxamide;
5-((1-(1-(Cyanomethyl)pyrrolidin-3-yl)ethyl)-N-(3,5-difluorophenyl)-1H-indole-2-carboxamide;
5-((1-(1-Acetylpyrrolidin-3-yl)ethyl)-N-(3,5-difluorophenyl)-1H-indole-2-carboxamide;
N-(3,5-Difluorophenyl)-5-(1-(1-(2,2,2-trifluoroethyl)pyrrolidin-3-yl)ethyl)-1H-indole-2-carboxamide;
5-(((S)-1-Methyl-pyrrolidin-3-yl)-1H-indole-2-carboxylic acid (3,5-difluoro-phenyl)-amide;
5-(((R)-1-Methyl-pyrrolidin-3-yl)-1H-indole-2-carboxylic acid (3,5-difluoro-phenyl)-amide;
5-[((S)-1-(2,2,2-Trifluoro-ethyl)-pyrrolidin-3-yl]-1H-indole-2-carboxylic acid (3,5-difluorophenyl)-amide;
5-(((R)-1-Isobutyryl-pyrrolidin-3-yl)-1H-indole-2-carboxylic acid (3,5-difluoro-phenyl)-amide;
5-(((S)-1-Isobutyryl-pyrrolidin-3-yl)-1H-indole-2-carboxylic acid (3,5-difluoro-phenyl)-amide;
5-(((S)-1-Isobutyryl-pyrrolidin-3-yl)-1H-indole-2-carboxylic acid (3,5-difluoro-phenyl)-amide;
(R)-5-(1-(Cyanomethyl)pyrrolidin-3-yl)-N-(3-fluorophenyl)-1H-indole-2-carboxamide;
(S)-5-(1-(Cyanomethyl)pyrrolidin-3-yl)-N-(3-fluorophenyl)-1H-indole-2-carboxamide;
5-(((R)-1-Isopropyl-piperidin-3-yl)-1H-indole-2-carboxylic acid (3-fluoro-phenyl)-amide;
5-(((S)-1-Isopropyl-piperidin-3-yl)-1H-indole-2-carboxylic acid (3-fluoro-phenyl)-amide;
5-(((R)-1-Acetyl-piperidin-3-yl)-1H-indole-2-carboxylic acid (3,5-difluoro-phenyl)-amide;
5-(((S)-1-Acetyl-piperidin-3-yl)-1H-indole-2-carboxylic acid (3,5-difluoro-phenyl)-amide;
(R)-N-(3-Fluorophenyl)-5-(1-(2-hydroxyethyl)piperidin-3-yl)-1H-indole-2-carboxamide;
(S)-N-(3-Fluorophenyl)-5-(1-(2-hydroxyethyl)piperidin-3-yl)-1H-indole-2-carboxamide;
(R)-5-(1-Cyclopropylpiperidin-3-yl)-N-(3-fluorophenyl)-1H-indole-2-carboxamide;
(S)-5-(1-Cyclopropylpiperidin-3-yl)-N-(3-fluorophenyl)-1H-indole-2-carboxamide;
N-(3,5-Difluorophenyl)-5-((R)-1-((S)-1-(2,2,2-trifluoroethyl)pyrrolidin-3-yl)ethyl)-1H-indole-2-carboxamide;
N-(3,5-Difluorophenyl)-5-((R)-1-((R)-1-(2,2,2-trifluoroethyl)pyrrolidin-3-yl)ethyl)-1H-indole-2-carboxamide;
5-(((S)-1-((S)-1-Acetylpyrrolidin-3-yl)ethyl)-N-(3,5-difluorophenyl)-1H-indole-2-carboxamide;
and pharmaceutically acceptable salts and esters thereof.

22. Compounds of formula (I) according to any of claims 1 - 21, selected from the group consisting of:
5-((1-Methyl-pyrrolidin-3-yl)-1H-indole-2-carboxylic acid (3,5-difluoro-phenyl)-amide;
N-(3-Fluorophenyl)-5-(1-methylpyrrolidin-3-yl)-1H-indole-2-carboxamide;
5-Pyrrolidin-3-yl-1H-indole-2-carboxylic acid (3,5-difluoro-phenyl)-amide;
5-((1-Isopropyl-pyrrolidin-3-yl)-1H-indole-2-carboxylic acid (3,5-difluoro-phenyl)-amide;
5-((1-(Cyanomethyl)pyrrolidin-3-yl)-N-(3-fluorophenyl)-1H-indole-2-carboxamide;
5-((1-Isopropyl-piperidin-3-yl)-1H-indole-2-carboxylic acid (3,5-difluoro-phenyl)-amide;
5-((1-Methanesulfonyl-piperidin-3-yl)-1H-indole-2-carboxylic acid (3,5-difluoro-phenyl)-amide;
5-[1-(Propane-2-sulfonyl)-piperidin-3-yl]-1H-indole-2-carboxylic acid (3,5-difluorophenyl)-amide;
5-((1-Isobutyryl-piperidin-3-yl)-1H-indole-2-carboxylic acid (3,5-difluoro-phenyl)-amide;
5-((1-Acetyl-piperidin-3-yl)-1H-indole-2-carboxylic acid (3,5-difluoro-phenyl)-amide;
5-((1-Benzenesulfonyl-piperidin-3-yl)-1H-indole-2-carboxylic acid (3,5-difluoro-phenyl)-amide;
5-((1-Cyclopropanecarbonyl-piperidin-3-yl)-1H-indole-2-carboxylic acid (3,5-difluorophenyl)-amide;
5-((1-Benzoyl-piperidin-3-yl)-1H-indole-2-carboxylic acid (3,5-difluoro-phenyl)-amide;
5-((1-Cyclopropanesulfonyl-piperidin-3-yl)-1H-indole-2-carboxylic acid (3,5-difluorophenyl)-amide;
5-((1-Methyl-piperidin-3-yl)-1H-indole-2-carboxylic acid (3,5-difluoro-phenyl)-amide;
5-Piperidin-3-yl-1H-indole-2-carboxylic acid (3,5-difluoro-phenyl)-amide;
5-(((S)-1-Methyl-pyrrolidin-3-yl)-1H-indole-2-carboxylic acid (3,5-difluoro-phenyl)-amide;
5-(((R)-1-Methyl-pyrrolidin-3-yl)-1H-indole-2-carboxylic acid (3,5-difluoro-phenyl)-amide;
(S)-5-(1-(Cyanomethyl)pyrrolidin-3-yl)-N-(3-fluorophenyl)-1H-indole-2-carboxamide;
5-((R)-1-Isopropyl-piperidin-3-yl)-1H-indole-2-carboxylic acid (3-fluoro-phenyl)-amide;
5-(((R)-1-Acetyl-piperidin-3-yl)-1H-indole-2-carboxylic acid (3,5-difluoro-phenyl)-amide;
(R)-N-(3-Fluorophenyl)-5-(1-(2-hydroxyethyl)piperidin-3-yl)-1H-indole-2-carboxamide;
and pharmaceutically acceptable salts and esters thereof.

23. A process for the preparation of compounds of formula (I) according to any of claims 1 - 22, comprising:
a) the reaction of a compound of formula (II) 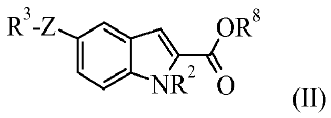 with a compound of formula R¹NH₂; or
b) the reaction of a compound of formula (III) 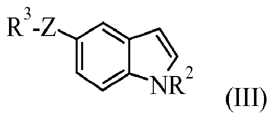 with a compound of formula R¹NCO; or
c) the reaction of a compound of formula (IV) 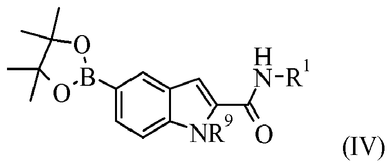 with 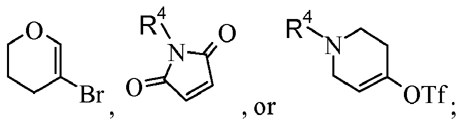 wherein Z, R¹, R², R³ and R⁴ are as defined in any of claims 1 to 22, R⁸ is hydrogen or alkyl, R⁹ is either R² as defined above or a protecting group selected from 4-methoxybenzyl bromide and Tf is trifluoromethanesulfonyl.

24. Compounds of formula (I) according to any of claims 1 - 22, obtainable by a process of claim 23.

25. Pharmaceutical compositions comprising compounds of formula (I) according to any of claims 1 - 22 or their pharmaceutically acceptable salts and esters and one or more pharmaceutically acceptable excipients.

26. Compounds of formula (I) according to any of claims 1 - 22 or their pharmaceutically acceptable salts and esters above for use as therapeutically active substances.

27. Compounds of formula (I) according to any of claims 1 - 22 or their pharmaceutically acceptable salts and esters for the treatment or prevention of Alzheimer's disease, cerebral amyloid angiopathy, hereditary cerebral hemorrhage with amyloidosis Dutch-type (HCHWA-D), multi-infarct dementia, dementia pugilistica or Down syndrome.

28. A method for the treatment or prevention of Alzheimer's disease, cerebral amyloid angiopathy, hereditary cerebral hemorrhage with amyloidosis Dutch-type (HCHWA-D), multi-infarct dementia, dementia pugilistica or Down syndrome, which method comprises administering compounds of formula (I) according to any of claims 1 - 22 or their pharmaceutically acceptable salts and esters as defined above to a human being or animal.

29. The use of compounds of formula (I) according to any of claims 1 - 22 or their pharmaceutically acceptable salts and esters for the treatment or prevention of Alzheimer's disease, cerebral amyloid angiopathy, hereditary cerebral hemorrhage with amyloidosis Dutch-type (HCHWA-D), multi-infarct dementia, dementia pugilistica or Down syndrome.

30. The use of compounds of formula (I) according to any of claims 1 - 22 or their pharmaceutically acceptable salts and esters for the preparation of medicaments for the treatment or prevention of Alzheimer's disease, cerebral amyloid angiopathy, hereditary cerebral hemorrhage with amyloidosis Dutch-type (HCHWA-D), multi-infarct dementia, dementia pugilistica or Down syndrome.

31. The invention as hereinbefore described.
